(19) Europäisches Patentamt
European Patent Office
Office européen des brevets

(11) Veröffentlichungsnummer: **0 384 349 B1**

(12) **EUROPÄISCHE PATENTSCHRIFT**

(45) Veröffentlichungstag der Patentschrift: **27.04.94**

(51) Int. Cl.[5]: **C07D 403/04**, C07D 487/04, C07D 403/14, A61K 31/40

(21) Anmeldenummer: **90103142.7**

(22) Anmeldetag: **19.02.90**

(54) **Substituierte Pyrrole.**

(30) Priorität: **23.02.89 GB 8904161**
**13.12.89 GB 8928210**

(43) Veröffentlichungstag der Anmeldung:
**29.08.90 Patentblatt 90/35**

(45) Bekanntmachung des Hinweises auf die Patenterteilung:
**27.04.94 Patentblatt 94/17**

(84) Benannte Vertragsstaaten:
**AT BE CH DE DK ES FR GB GR IT LI LU NL SE**

(56) Entgegenhaltungen:
**EP-A- 0 328 026**

**CHEMICAL ABSTRACTS, Band 109, Nr. 9, 29. August 1988, Columbus, Ohio, USA BERGMAN J.; PELCMAN, B. "Coupling of indoleacetic acid trianion or methyl indoleacetic acid dianion. Abiomimetic approach to indolocarbazole alkaloids.Seite 722, Spalte 2**

(73) Patentinhaber: **F. HOFFMANN-LA ROCHE AG**
**Postfach 3255**
**CH-4002 Basel(CH)**

(72) Erfinder: **Davis, Peter David,**
**16 Olden Mead,**
**Letchworth, Herts.,(GB)**
Erfinder: **Hill, Christopher Huw,**
**6 Woodstock,**
**Knebworth, Herts.,(GB)**
Erfinder: **Lawton, Geoffrey,**
**108 Whitehill Road,**
**Hitchin, Herts.,(GB)**

(74) Vertreter: **Lederer, Franz, Dr. et al**
**Lederer, Keller & Riederer**
**Patentanwälte**
**Prinzregentenstrasse 16**
**D-80538 München (DE)**

**EP 0 384 349 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 109, Nr. 15, 10. Oktober 1988, Columbus, Ohio, USA AGUSTIN, M.; JESCHKE, P. "Reaction behavior of 2-ethoxycarbonyl-3-hydroxy-N-phenylmaleimide" Seite 665, Spalte 2

CHEMICAL ABSTRACTS, Band 104, Nr. 23, 9. Juni 1986, Columbus, Ohio, USA KANEKO, T. et al. "Two synthetic approaches to rebeccamycin." Seite 789, Spalte 1

CHEMICAL ABSTRACTS, Band 101, Nr. 7, 13. August 1984, Columbus, Ohio, USA WEINREB, STEVEN M. et al. "Natural product synthesis via cycloadditions with N-sulfinyl dienophiles." Seite 654, Spalte 2;

CHEMICAL ABSTRACTS, Band 102, Nr. 1. 7. Jänner 1985, Columbus, Ohio, USA AUGUSTIN, M. et al. "Reactions of 2-cyano-3-methoxy-maleimide with active methylene compounds and enamines." Seite 567, Spalte 1

CHEMICAL ABSTRACTS, Band 98, Nr. 25, 20. Juni 1983, Columbus Ohio, USA SARSTEDT, BURKHARD et al. "Reactions with indole derivatives. XLVIII. A simple synthesis of the staurosporine aglycon." Seite 585, Spalte 1

CHEMICAL ABSTRACTS, Band 93, Nr. 7, 18. August 1980, Columbus, Ohio, USA STEGLICH, WOLFGANG et al. "Fungus pigments. 36. Indole pigments from the fruit bodies of the slime fungus Arcyria denudata." Seite 503 Spalte 1

## Beschreibung

Die vorliegende Erfindung betrifft substituierte Pyrrole, insbesondere solche der allgemeinen Formel

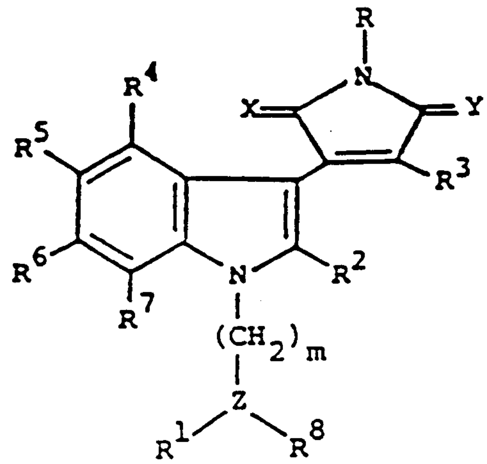

I

worin R Wasserstoff oder Hydroxy, $R^1$ und $R^2$ zusammen eine Gruppe der Formel $-(CH_2)_n-$ und $R^7$ Wasserstoff, oder $R^1$ und $R^7$ zusammen eine Gruppe der Formel $-(CH_2)_n-$ und $R^2$ Wasserstoff; $R^3$ Aryl oder Heteroaryl; $R^4$, $R^5$ und $R^6$ Wasserstoff, Halogen, Alkyl, Hydroxy, Alkoxy, Haloalkyl, Nitro, Amino, Acylamino, Alkylthio, Alkylsulfinyl oder Alkylsulfonyl; $R^8$ eine Gruppe der Formel $-(CH_2)_p-R^9$ oder $-(CH_2)_q-R^{10}$; $R^9$ Wasserstoff, Alkylcarbonyl, Aminoalkylcarbonyl, Cyano, Amidino, Alkoxycarbonyl, Aryloxycarbonyl, Alkylsulfonyl, Aminocarbonyl oder Aminothiocarbonyl; $R^{10}$ Hydroxy, Alkoxy, Halogen, Amino, Monoalkylamino, Dialkylamino, Trialkylamino, Azido, Acylamino, Alkylsulfonylamino, Arylsulfonylamino, Alkylthio, Alkoxycarbonylamino, Aminoacylamino, Aminocarbonylamino, Isothiocyanato, Alkylcarbonyloxy, Alkylsulfonyloxy oder Arylsulfonyloxy, eine 5- oder 6-gliedrige gesättigte stickstoffhaltige heterocyclische Gruppe, die über das N-Atom gebunden ist oder eine Gruppe der Formel -U-C(V)-W; U ein S-Atom oder NH; V die Gruppen NH, $NNO_2$, NCN oder $CHNO_2$; W Amino, Mono- oder Dialkylamino; eins von X und Y ein O-Atom und das andere O oder (H,H); Z die Gruppe CH oder ein N-Atom; m, p und q eine Zahl von 0 bis 5 sind und n eine Zahl von 1 bis 5 ist, mit der Bedingung, dass m und q eine Zahl von 2 bis 5 sind, wenn Z ein N-Atom ist;
sowie pharmazeutisch verwendbare Salze von sauren Verbindungen der Formel I mit Basen und von basischen Verbindungen der Formel I mit Säuren.

Die Erfindung betrifft die weiter oben definierten Verbindungen als solche sowie als therapeutisch aktive Substanzen, ein Verfahren zu ihrer Herstellung sowie neue Zwischenprodukte; Medikamente die diese Verbindungen enthalten und die Herstellung dieser Medikamente; sowie die Verwendung der besagten Verbindungen bei der Behandlung oder der Prophylaxe von Krankheiten, speziell von inflammatorischen, immunologischen, onkologischen, bronchopulmonären und kardiovaskulären Krankheiten, sowie bei der Behandlung von Asthma oder AIDS, oder bei der Herstellung von solchen Medikamenten.

Im Rahmen der Erfindung bezeichnet "Alkyl" allein oder in Kombination eine geradkettige oder verzweigte Alkylgruppe mit bis zu 7, vorzugsweise bis zu 4 C-Atome, wie Methyl, Aethyl, Propyl, Isopropyl, Butyl, s-Butyl, t-Butyl und Pentyl. Beispiele von Alkoxygruppen sind Methoxy, Aethoxy, Propoxy, Isopropoxy, Butoxy und t-Butoxy. Ein Haloalkyl kann eine oder mehrere Halogenatome enthalten. Ein Beispiel für eine solche Gruppe ist Chlormethyl und Trifluormethyl. "Acyl" bezeichnet eine Gruppe, die von einer Alkancarbonsäure mit bis zu 7, vorzugsweise bis zu 4 C-Atomen, z.B. Formyl, Acetyl, Propionyl oder Butyryl, oder von einer aromatischen Carbonsäure, z.B. Benzoyl, abgeleitet ist. "Aryl" allein oder in Kombination bezeichnet eine monocyclische oder polycyclische, vorzugsweise eine monocyclische oder bicyclische Gruppe, d.h. Phenyl oder Naphthyl, die durch einen oder mehreren Substituenten, vorzugsweise 1-3 Substituenten, substituiert sein können aus der Gruppe der folgenden: Halogen, Alkyl, Hydroxy, Alkoxy, Haloalkyl, Nitro, Amino, Acylamino, Alkylthio, Alkylsulfinyl oder Alkylsulfonyl. Beispiele von Arylgruppen sind Phenyl, 2-, 3- oder 4-Chlorphenyl, 3-Bromphenyl, 2- oder 3-Methylphenyl, 2,5-Dimethylphenyl, 4-Methoxyphenyl, 2-Trifluormethylphenyl, 3-Trifluormethylphenyl, 2-, 3- oder 4-Nitrophenyl, 3-Aminophenyl, 4-Aminophenyl, 4-Methylthiophenyl, 4-Methylsulfinylphenyl, 4-Methylsulfonylphenyl und 1- oder 2-Naphthyl. "Heteroaryl" bezeichnet eine 5- oder 6-gliedrige heterocyclische aromatische Gruppe, die gewünschtenfalls einen ankondensierten Benzolring enthält und die durch eine oder mehrere, vorzugsweise 1-3 Substituenten aus

der Gruppe der folgenden: Halogen, Alkyl, Hydroxy, Alkoxy, Haloalkyl, Nitro, Amino, Acylamino, Alkylthio, Alkylsulfinyl und Alkylsulfonyl substituiert sein kann. Beispiele von solchen Heteroarylgruppen sind 2- oder 3-Thienyl, 3-Benzothienyl, 3-Benzofuranyl, 2-Pyrrolyl und 3-Indolyl, die wie weiter oben beschrieben, substituiert sein können. Die 5- oder 6-gliedrige gesättigte stickstoffhaltige heterocyclische Gruppe, die über ein N-Atom gebunden ist, kann ein zusätzliches Stickstoff-, Sauerstoff- oder Schwefelatom enthalten. Beispiele von solchen Heterocyclen sind Pyrrolidino, Piperidino, Piperazino, Morpholino und Thiamorpholino. "Halogen" bezeichnet Fluor, Chlor, Brom oder Jod.

Die Verbindungen der Formel I, worin Z die Gruppe CH und $R^8$ eine Gruppe $-(CH_2)_p-R^9$, in der p eine Zahl zwischen 1 und 5 oder $-(CH_2)_q-R^{10}$ sind, enthalten ein asymmetrisches C-Atom und können daher in racemischer oder optich aktiver Form vorliegen. Die Erfindung umfasst sowohl die racemischen Verbindungen, wie die optisch aktiven Isomeren.

In den bevorzugten Verbindungen der Formel I sind $R^1$ und $R^2$ zusammen $-CH_2-$ und $R^7$ Wasserstoff, m die Zahl 1 oder 2 und Z CH; oder $R^1$ und $R^2$ zusammen $-(CH_2)_2-$ und $R^7$ Wasserstoff, m die Zahl 1 und Z die Gruppe CH; oder $R^1$ und $R^2$ zusammen $-CH_2-$ und $R^7$ Wasserstoff, m die Zahl 2 und Z ein N-Atom; oder $R^1$ und $R^7$ zusammen $-CH_2-$ und $R^2$ Wasserstoff, m die Zahl 1 und Z die Gruppe CH; oder $R^1$ und $R^7$ zusammen $-(CH_2)_2-$ und $R^2$ Wasserstoff, m O und Z die Gruppe CH. $R^3$ ist vorzugsweise Phenyl, Naphthyl, 3-Benzothienyl, 3-Benzofuranyl oder 3-Indolyl, die gewünschtenfalls wie weiter oben beschrieben substituiert sein können, insbesondere 1-Methyl-3-indolyl. $R^4$, $R^5$ und $R^6$ sind vorzugsweise Wasserstoff. $R^8$ ist vorzugsweise $-(CH_2)_q-R^{10}$. q ist vorzugsweise 1 oder 2. $R^{10}$ ist vorzugsweise Hydroxy, Amino, Mono-, Di- oder Trialkylamino, Azido, Acylamino, Alkylcarbonyloxy oder Alkylsulfonyloxy oder eine Gruppe -U-C(V)-W. U ist vorzugsweise S; V ist vorzugsweise NH und W ist vorzugsweise Amino.

Besonders bevorzugte Verbindungen sind:

3-[8-(Aminomethyl)-6,7,8,9-tetrahydropyrido[1,2-a]indol-10-yl]-4-(1-methyl-3-indolyl)-1H-pyrrol-2,5-dion,

3-[7-(Amidinothiomethyl)-6,7,8,9-tetrahydropyrido[1,2-a]indol-10-yl]-4-(1-methyl-3-indolyl)-1H-pyrrol-2,5-dion und

3-[6,7,8,9-Tetrahydro-8-[(dimethylamino)methyl]pyrido[1,2-a]indol-10-yl]-4-(1-methyl-3-indolyl)-1H-pyrrol-2,5-dion

und pharmazeutisch verwendbare Säureadditionssalze davon.

Die wie weiter oben definierten Verbindungen der Formel I und Salze davon können dadurch hergestellt werden, dass man

a) zur Herstellung einer Verbindung der Formel I, in der X und Y Sauerstoff sind, eine Verbindung der allgemeinen Formel

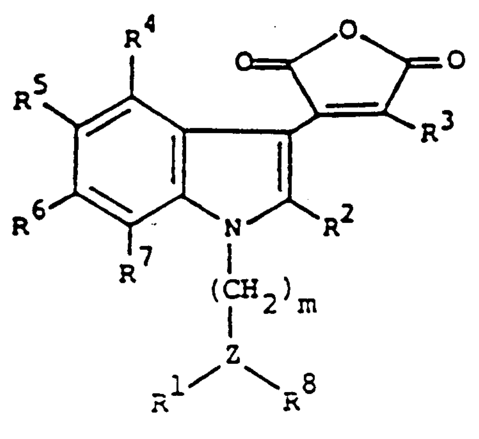

II

worin $R^1$, $R^2$, $R^3$, $R^4$, $R^5$, $R^6$, $R^7$, $R^8$, Z

und m die in Anspruch 1 angegebene Bedeutung haben,

mit Ammoniak unter Druck oder mit Hexamethyldisilazan und Methanol zu einer Verbindung der Formel I umsetzt, in der R Wasserstoff ist, oder mit Hydroxylamin zu einer Verbindung der Formel, in der R Hydroxy ist, umsetzt, oder

b) zur Herstellung einer Verbindung der Formel I, in der eins von X und Y Sauerstoff und das andere (H,H) ist, eine Verbindung der Formel I, in der X und X Sauerstoff sind, mit Lithiumaluminiumhydrid reduziert, und

c) gewünschtenfalls eine in einer erhaltenen Verbindung der Formel I enthaltene reaktionsfähige Gruppe funktionell abwandelt, und

d) gewünschtenfalls eine saure Verbindung der Formel I in ein pharmazeutisch verwendbares Salz mit einer Base oder eine basische Verbindung der Formel I in ein pharmazeutisch verwendbares Salz mit einer Säure umwandelt.

Die Reaktion einer Verbindung der Formel II mit Ammoniak unter Druck nach Variante a) kann zweckmässig mittels wässrigem Ammoniak, vorzugsweise 33% wässriges Ammoniak, und in Gegenwart von einem mit Wasser mischbaren inerten organischen Lösungsmittel, wie Dimethylformamid (DMF), durchgeführt werden. Die Reaktion wird vorzugsweise bei erhöhter Temperatur, z.B. bei einer Temperatur zwischen etwa 100 und 150 °C durchgeführt.

Die Reaktion einer Verbindung der Formel II mit Hexalmethyldisilazan und Methanol kann zweckmässig in einem inerten organischen Lösungsmittel, wie einem halogenierten Kohlenwasserstoff, z.B. Chloroform, Tetrachlorkohlenstoff oder Chlorbenzol, oder einem aromatischen Kohlenwasserstoff, z.B. Benzol, Toluol oder Xylol, bei erhöhter Temperatur, z.B. zwischen etwa 40 und 110 °C durchgeführt werden.

Die Reaktion einer Verbindung der Formel II mit Hydroxylamin kann zweckmässig in einem inerten organischen Lösungsmittel, wie DMF und bei Raumtemperatur oder bei erhöhter Temperatur, vorzugsweise bei etwa 100 °C durchgeführt werden. Vorzugsweise wird das Hydroxylamin in Form eines Salzes, wie dem Hydrochlorid, verwendet und die Reaktion wird in Gegenwart einer Base, wie einem Alkalimetallcarbonat, z.B. dem Natrium- oder Kaliumcarbonat, durchgeführt.

Die Reaktion eine Verbindung der Formel I, in der X und Y O sind mit Lithiumaluminiumhydrid ($LiAlH_4$) nach Variante b) kann zweckmässig in einem inerten organischen Lösungsmittel, wie einem aliphatischen oder cyclischen Aether, z.B. Diäthyläther oder Tetrahydrofuran (THF), bei einer Temperatur zwischen etwa 0 °C und Rückflusstemperatur des Reaktionsgemisches durchgeführt werden.

Nach Variante c) kann eine in einer Verbindung der Formel I enthaltene reaktionsfähige Gruppe in an sich bekannter Weise abgewandelt werden. Wenn $R^8$ eine Gruppe $-(CH_2)_p-R^9$ ist, in der $R^9$ Alkoxycarbonyl und p O ist, in eine entsprechende Gruppe umgewandelt werden, in der $R^9$ Wasserstoff ist, durch Behandlung mit einer Säure. Eine Gruppe $-(CH_2)_q-R^{10}$, in der $R^{10}$ Alkylcarbonyloxy ist, kann in eine entsprechende Gruppe umgewandelt werden, in der $R^{10}$ Hydroxy ist, durch Behandlung mit einer Base. Eine Gruppe $-(CH_2)_q-R^{10}$, in der $R^{10}$ Hydroxy ist, kann in eine entsprechende Gruppe umgewandelt werden, in der $R^{10}$ Amino, Mono-, Di- oder Trialkylamino oder eine 5- oder 6-gliedrige gesättigte N-haltige heterocyclische Gruppe, die über ein N-Atom gebunden ist, übergeführt werden, durch Behandlung zuerst mit Trifluormethanesulfonsäureanhydrid und dann mit Ammoniak, einem Mono-, Di- oder Trialkylamin bzw. einem geeigneten Heterocyclen. Eine Gruppe der Formel $-(CH_2)_q-R^{10}$, in der $R^{10}$ Hydroxy ist, kann ferner mittels eines Alkanesulfonsäureanhydrids in die entsprechende Gruppe, in der $R^{10}$ Alkylsulfonyloxy ist umgewandelt werden. Eine Gruppe der Formel $-(CH_2)_q-R^{10}$, in der $R^{10}$ Alkylsulfonyloxy ist, kann man durch Reaktion mit Ammoniak in DMF in die entsprechende Gruppe, in der $R^{10}$ Formamido ist, oder durch Behandlung mit einem Alkalimetallazid in die entsprechende Gruppe, in der $R^{10}$ Azido ist, oder durch Reaktion mit Thiourea in die entsprechende Gruppe, in der $R^{10}$ eine Gruppe -U-C(V)-W, U ein S-Atom, V eine NH-Gruppe und W Amino ist, umwandeln. Ferner kann man eine Gruppe $-(CH_2)_q-R^{10}$, in der $R^{10}$ Azido ist, durch katalytische Hydrierung in eine entsprechende Gruppe, in der $R^{10}$ Amino ist, umwandeln. Eine Gruppe $-(CH_2)_q-R^{10}$, in der $R^{10}$ Alkoxycarbonylamino ist, kann man in die entsprechende Gruppe, in der $R^{10}$ Amino ist, durch Behandlung mit einer Säure umwandeln. Eine Gruppe $-(CH_2)_q-R^{10}$, in der $R^{10}$Amino ist, kann man zur entsprechenden Gruppe, in der $R^{10}$ Acylamino ist, acylieren oder mit 3,5-Dimethyl-$N^2$-nitro-1-pyrazol-1-carboxamid zur entsprechenden Gruppe, in der $R^{10}$ eine Gruppe -U-C(V)-W, U und V die Gruppe NH und W die Gruppe $NNO_2$ ist, umwandeln. Ferner kann man eine Gruppe $-(CH_2)_q-R^{10}$, in der $R^{10}$ Amino ist, in die entsprechende Gruppe, in der $R^{10}$ Isothiocyanato ist, durch Behandlung mit 1,1-Thiocarbonyldiimidazol, umwandeln. Eine Gruppe $-(CH_2)_p-R^9$, in der $R^9$ Cyan ist, kann man durch Behandlung mit Chlorwasserstoff und dann mit Ammoniak in die entsprechende Gruppe, in der $R^9$ Amidino ist, umwandeln.

Eine Verbindung der Formel I, in der Z ein N-Atom, $R^8$ eine Gruppe $-(CH_2)_p-R^9$, p O und $R^9$ Wasserstoff ist, kann in die entsprechende Verbindung umgewandelt werden, in der $R^9$ Alkanoyl, Alkoxycarbonyl oder Aralkoxycarbonyl ist, durch geeignete Acylierung; in die entsprechende Verbindung, in der $R^9$ Alkylsulfonyl durch Reaktion mit einem Alkansulfonylchlorid; in die entsprechende Verbindung, in der $R^9$ Aminoalkylcarbonyl ist, durch Behandlung mit Trifluoracetamidoalkanoylchlorid und dann mit Ammoniak; in eine entsprechende Verbindung, in der $R^9$ Aminocarbonyl ist, durch Behandlung mit 1,1-Carbonyldiimidazol und dann mit Ammoniak; oder in eine entsprechende Verbindung, in der $R^9$ Aminothiocarbonyl ist, durch Behandlung mit 1,1-Thiocarbonyldiimidazol und dann mit Ammoniak.

Die Ueberführung einer sauren Verbindung der Formel I in ein pharmazeutisch verwendbares Salz nach Variante e) kann durch Behandlung mit einer geeigneten Base in an sich bekannter Weise durchgeführt werden. Geeignete Salze sind solche, die von einer anorganischen Base abgeleitet sind, z.B. Natrium-, Kalium- oder Calciumsalze, oder von einer organischen Base, wie Aethylendiamin oder Mono- oder

Diäthanolamin abgeleitet sind. Die Umwandlung einer basischen Verbindung der Formel I in ein pharmazeutisch verwendbares Salz kann durch Behandlung mit einer geeigneten Säure in an sich bekannter Weise bewerkstelligt werden. Geeignete Salze sind solche, die von einer anorganischen Säure, wie Hydrochloride, Hydrobromide, Phosphate oder Sulfate, oder von einer organischen Säure, z.B. Acetate, Citrate, Fumarate, Tartrate, Maleate, Methansulfonate oder p-Toluolsulfonate, abgeleitet sind.

Die Ausgangsverbindungen der Formel II in der Variante a) sind neu und als solche Gegenstand der Erfindung. Sie können durch Reaktion einer Verbindung der Formel

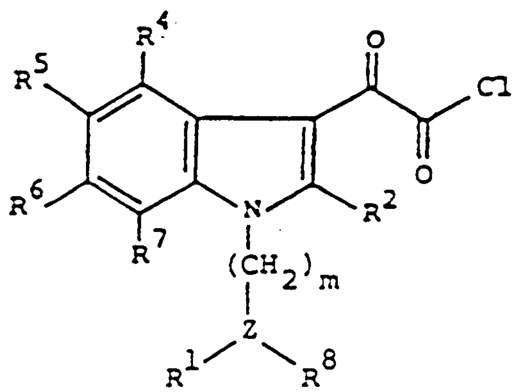

III

worin $R^1$, $R^2$, $R^4$, $R^5$, $R^6$, $R^7$, $R^8$, Z und m die obige Bedeutung haben,
mit einer Verbindung der Formel

$HOOC\text{-}CH_2\text{-}R^3$ IV

worin $R^3$ die obige Bedeutung hat,
hergestellt werden, und gewünschtenfalls durch funktionelle Abwandlung einer reaktionsfähigen Gruppe.

Die Reaktion einer Verbindung der Formel III mit einer solchen der Formel IV wird vorzugsweise in Gegenwart eines säurebindenden Mittels, z.B. eines tertiären Amins, wie einem Trialkylamin, z.B. Triäthylamin oder Diisopropyläthylamin, in einem inerten organischen Lösungsmittel, wie einem halogenierten aliphatischen Kohlenwasserstoff, wie Dichlormethan, bei Raumtemperatur durchgeführt werden.

Die fakultative funktionelle Abwandlung einer reaktionsfähigen Gruppe in einer Verbindung der Formel II kann in der gleichen Weise durchgeführt werden, wie die funktionelle Umwandlung einer solchen Gruppe in einer Verbindung der Formel I.

Die Verbindungen der Formel III können dadurch hergestellt werden, dass man eine Verbindung der Formel

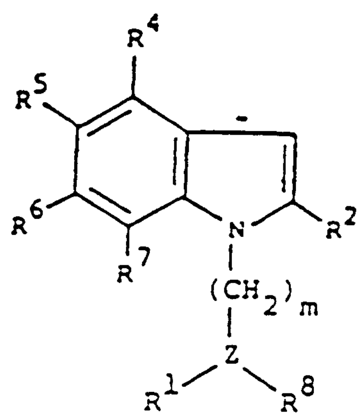

V

worin $R^1$, $R^2$, $R^4$, $R^5$, $R^6$, $R^7$, $R^8$, Z und m
die obige Bedeutung haben,
mit Oxalylchlorid umsetzt, zweckmässig in einem inerten organischen Lösungsmittel, wie einem halogenierten aliphatischen Kohlenwasserstoff, bei einer Temperatur zwischen etwa 0 °C und Rückflusstemperatur des Lösungsmittels, umsetzt. Die entstandene Verbindung der Formel III kann in situ mit der Verbindung der Formel IV umgesetzt oder, z.B. durch Einengen gefolgt durch Kristallisation, vor der Reaktion mit einer Verbindung der Formel IV isoliert und gereinigt werden.

Die Verbindungen der Formel V sind bekannt oder bekannten Verbindungen analog und in ähnlicher Weise wie die bekannten Verbindungen herstellbar.

Die Verbindungen der Formel I und ihre pharmazeutisch verwendbaren Salze sind Inhibitoren der Proteinkinase; sie hemmen cellulare Prozesse, z.B. die Zellproliferation und können in der Behandlung oder Prophylaxe von Krankheiten, z.B. von inflammatorischen Krankheiten, wie Arthritis, von Immunkrankheiten oder bei Organtransplantationen sowie in der Onkologie Verwendung finden. Sie hemmen die Infektion der Zellen durch Humanimmunodefekt-Viren und sind daher in der Behandlung von AIDS verwendbar. Sie hemmen auch die Kontraktion der glatten Muskulatur und können daher gegen cardiovaskuläre und bronchopulmonäre Krankheiten verwendet werden. Ferner sind sie in der Asthmatherapie verwendbar.

Die Aktivität der vorliegenden Verbindungen in der Hemmung der Proteinkinase C kann man z.B. anhand des in BBRC 19 (1979) 1218 beschriebenen Versuchssystems nachweisen. Die $IC_{50}$-Werte in der nachstehenden Tabelle sind diejenigen Konzentrationen der Testsubstanz, die die durch die Proteinkinase induzierte Inkorporation von $^{32}P$ aus $[\gamma\text{-}^{32}P]ATP$ in das Histon um 50% reduziert.

## Tabelle

| Verbindung | $IC_{50}$ |
|---|---|
| 3-[8-(Aminomethyl)-6,7,8,9-tetrahydropyrido[1,2-a]indol-10-yl]-4-(1-methyl-3-indolyl)-1H-pyrrol-2,5-dion-hydrochlorid | 8 nM |
| 3-[7-(Amidinothiomethyl)-6,7,8,9-tetrahydropyrido[1,2-a]indol-10-yl]-4-(1-methyl-3-indolyl)-1H-pyrrol-2,5-dion-methansulfonat | 15 nM |

| | |
|---|---|
| 3-[2-(Aminoacetyl)-1,2,3,4-tetrahydropyrazino[1,2-a]indol-10-yl]-4-(1-methyl-3-indolyl)-1H-pyrrol-2,5-dion-hydrochlorid | 50 nM |
| 3-[7-(2-Aminoäthyl)-6,7,8,9-tetrahydropyrido[1,2-a]-indol-10-yl]-4-(1-methyl-3-indolyl)-1H-pyrrol-2,5-dion-hydrochlorid | 20 nM |
| 3-[6,7,8,9-Tetrahydro-8-[(1-piperidino)-methyl]pyrido[1,2-a]indol-10-yl]-4-(1-methyl-3-indolyl)-1H-pyrrol-2,5-dion | 30 nM |
| 3-[2,3-Dihydro-2-(dimethylaminomethyl)-1H-pyrrolo[1,2-a]indol-9-yl]-4-(1-methyl-3-indolyl)-1H-pyrrol-2,5-dion-trifluormethansulfonat | 20 nM |
| 3-[8-Amidino-6,7,8,9-tetrahydropyrido-[1,2-a]indol-10-yl]-4-(1-methyl-3-indolyl)-1H-pyrrol-2,5-dion-hydrochlorid | 60 nM |
| 3-[7-(Amidinothiomethyl)-6,7,8,9-tetrahydropyrido[1,2-a]indol-10-yl]-4-(1-methyl-3-indolyl)-1H-pyrrol-2,5-dion-methansulfonat | 10 nM |

Die Pyrrole der Formel I und ihre Salze können als Medikamente verwendet werden, z.B. in Form von pharmazeutischen Präparaten, die man oral, z.B. in Form von Tabletten, Dragées, Hart- oder Weichgelatinekapseln, Lösungen, Emulsionen oder Suspensionen, verabreichen kann. Sie können auch rektal, z.B. in Form von Suppositorien, oder parenteral, z.B. in Form von Injektionslösungen verabreicht werden.

Für die Herstellung von pharmazeutischen Präparaten können diese Verbindungen mit therapeutisch inerten anorganischen und organischen Träger verarbeitet werden. Beispiele von solchen Trägern für Tabletten, Dragées und Hartgelatinekapseln sind Lactose, Maisstärke oder Derivate davon, Talk, Stearinsäure oder dessen Salze. Geeignete Träger für Weichgelatinekapseln sind pflanzliche Oele, Wachse, Fette und halbfeste oder flüssige Polyole. Je nach der Natur des Wirkstoffes erübrigen sich jedoch Träger im Fall von Weichgelatinekapseln. Geeignete Träger für die Herstellung von Lösungen und Sirupen sind Wasser, Polyole, Saccharose, Invertzucker und Glucose. Geeignete Träger für Injektionslösungen sind Wasser, Alkohole, Polyole, Glycerol und pflanzliche Oele. Geeignete Träger für Suppositorien sind pflanzliche oder

gehärtete Oele, Wachse, Fette und halbflüssige Polyole.

Die pharmazeutischen Präparate können auch Konservierungsmittel, Lösungsmittel, Stabilisierungsmittel, Netzmittel, Emulgiermittel, Süsstoffe, Farbstoffe, Geschmacksmittel, Salze zur Veränderung des osmotischen Druckes, Puffer, Ueberzugsmittel oder Antioxidantien, sowie gegebenenfalls andere therapeutische Wirkstoffe enthalten.

Wie weiter oben angegeben können die Pyrrole der Formel I und ihre Salze in der Behandlung oder Prophylaxe von Krankheiten speziell von inflammatorischen, immunologischen, bronchopulmonären und cardiovaskulären Krankheiten oder in der Behandlung von Asthma oder von AIDS verwendet werden. Die Dosierung kann in weiten Bereichen variieren, liegt jedoch im allgemeinen bei oraler Verabreichung an Erwachsenen im Bereich von etwa 5 bis 500 mg/Tag, obwohl letzterer Wert, falls nötig, erhöht werden kann. Die tägliche Dosis kann in einer Einzeldosis oder in mehreren Dosen verabreicht werden.

Beispiel 1

Eine Lösung von 2,90 g 3-[8-(Acetoxymethyl)-6,7,8,9-tetrahydropyrido[1,2-a]indol -10-yl]-4-(1-methyl-3-indolyl)furan-2,5-dion in 30 ml DMF und 23 ml 33%ig wässriges Ammoniak wird 7 Stunden bei 140°C erhitzt. Das Gemisch wird mit Aethylacetat extrahiert die vereinigten organischen Extrakte werden mit Wasser gewaschen, getrocknet und eingedampft. Kristallisation des Rückstandes aus Aethylacetat ergibt 1,87 g 3-[6,7,8,9-Tetrahydro-8-(hydroxymethyl)pyrido[1,2-a]indol -10-yl]-4-(1-methyl-3-indolyl)-1H-pyrrol-2,5-dion, Smp. 262-263°C.

a) Eine Lösung von 25 g Aethyl-indol-2-carboxylat in 400 ml DMF wird unter Rühren einer Lösung von 5,5 g einer 60%igen Dispersion von Natriumhydrid in Mineralöl in 40 ml DMF unter einer Stickstoffatomsphäre getropft. 30,9 g Aethylbrombutyrat werden dem Gemisch bei 0°C zugetropft und das resultierende Gemisch wird 18 Stunden bei Raumtemperatur gerührt. Die Reaktion wird durch Zugabe von 100 ml Wasser und 30 ml 2N Salzsäure beendet und das Gemisch wird mit Dichlormethan extrahiert. Die vereinigten organischen Extrakte werden mit Wasser gewaschen, getrocknet und eingedampft. Man erhält 49 g eines Oels, das in Aethylacetat gelöst wird. Die Lösung wird mit Wasser gewaschen, getrocknet und eingedampft. Man erhält 39 g eines Oels. Dieses wird zu einer Suspension von 20,5 g Kalium-t-butoxid in 750 ml THF unter einer Stickstoffatmosphäre unter Rühren getropft. Nach 1 Stunde werden 200 ml Wasser und dann 92 ml 2N Salzsäure zugesetzt. Das Gemisch wird konzentriert und der entstandene Niederschlag abfiltriert und getrocknet. Man erhält 25,3 g Aethyl-6,7-dihydro-9-hydroxypyrido[1,2-a]indol-8-carboxylat,Smp. 101-103°C nach Kristallisation aus Methanol.

b) Eine Suspension von 19,4 g des Carboxylats von a) und 16 Löffelspitzen Raney-Nickel in 480 ml Aethanol und 240 ml Wasser werden 3,5 Stunden unter Rückfluss erhitzt. Weitere 4 Löffelspitzen Raney-Nickel werden dann zugesetzt und das Gemisch wird 1,5 Stunden unter Rückfluss erhitzt. Die obige Schicht wird dekantiert und der Katalysator wird mit Aethylacetat gewaschen. Die vereinigten organischen Phasen werden eingeengt und der Niederschlag wird abfiltriert und getrocknet. Man erhält 16,3 g Aethyl-6,7,8,9-tetrahydropyrido[1,2-a]indol-8-carboxylat, Smp. 70-72°C nach Kristallisation aus Methanol.

c) 16,2 g des Carboxylats von b) in 200 ml THF werden einer Suspension von 2,00 g $LiAlH_4$ in 600 ml THF bei 0°C unter einer Stickstoffatmosphäre zugesetzt. Nach 0,5 Stunden wird die Reaktion durch Zusatz von Aethylacetat, Wasser und 2N Salzsäure beendet und das Gemisch wird mit Diäthyläther extrahiert. Die vereinigten organischen Extrakte werden getrocknet und eingeengt. Kristallisation des Rückstandes aus Diäthyläther/n-Hexan ergibt 11,5 g 6,7,8,9-Tetrahydro-8-(hydroxymethyl)pyrido[1,2-a]-indol, Smp. 110-111°C.

d) 11.4 g Essigsäureanhydrid werden einer Lösung von 11,0 g des Produkts von c) in 100 ml Pyridin zugesetzt und die resultierende Lösung wird 18 Stunden unter Stickstoff gerührt. Der grösste Teil des Pyridins wird abgedampft und der Rückstand mit 2N Salzsäure angesäuert. Das Gemisch wird mit Diäthyläther extrahiert und die vereinigten Extrakte werden mit Natriumbicarbonatlösung und Wasser gewaschen. Die Extrakte werden getrocknet und eingeengt. Man erhält 11,25 g 8-Acetoxymethyl-6,7,8,9-tetrahydropyrido[1,2-a]indol,Smp. 63-64°C.

e) 4,13 g Oxalylchlorid werden einer Lösung von 8,2 g des Tetrahydropyridoindols von d) in 160 ml Diäthyläther unter einer Stickstoffatmosphäre zugetropft. Nach 10 Minuten wird das Lösungsmittel unter vermindertem Druck entfernt und der Rückstand in 330 ml Dichlormethan gelöst. 6,34 g 1-Methyl-3-indolessigsäure und 9,20 ml Triäthylamin werden der Lösung zugesetzt und das Gemisch wird über Nacht gerührt. Weitere 4,60 ml Triäthylamin werden zugesetzt. Nach 48 Stunden wird das Lösungsmittel unter vermindertem Druck entfernt und der Rückstand durch Chromatographie über Silicagel mit Aethylacetat/Petroläther (1:2) gereinigt. Kristallisation aus Aethylacetat ergibt 4,02 g 3-[8-Acetoxymethyl-6,7,8,9-tetrahydropyrido[1,2-a]indol-10-yl]-4-(1-methyl-3-indolyl)furan-2,5-dion, Smp. 174-178°C.

Beispiel 2

2,50 g Trifluormethansulfonsäureanhydrid in 330 ml Dichlormethan werden bei 0 °C unter einer Stickstoffatmosphäre mit einer Suspension von 1,87 g des Pyrroldions von Beispiel 1 und 0,94 g Collidin in 280 ml Dichlormethan behandelt. Nach 2,5 Stunden wird das Gemisch auf 10 °C erwärmen gelassen. 37 ml 33%iges wässriges Ammoniak werden dann zugesetzt und das Gemisch wird auf Raumtemperatur erwärmen gelassen. Das Gemisch wird mit Wasser gewaschen, getrocknet und eingedampft. Der Rückstand wird über Silicagel mit Dichlormethan/Methanol/ Essigsäure/Wasser (90:18:3:2) chromatographiert. Die vereinigten Produkt enthaltenden Fraktionen werden mit 2M Salzsäure behandelt und eingedampft. Man erhält 930 mg 3-[8-Aminomethyl-6,7,8,9-tetrahydropyrido[1,2-a]indol -10-yl]-4-(1-methyl-3-indolyl)-1H-pyrrol -2,5-dion-hydrochlorid, Smp. 310-313 °C.

Beispiel 3

265 mg Trifluormethansulfonsäureanhydrid in 40 ml Dichlormethan werden bei 0 °C unter eine Stickstoffatmosphäre mit einer Suspension von 200 mg des Produkts von Beispiel 1 und 100 mg Collidin in 30 ml Dichlormethan behandelt. Nach 5 Stunden werden 0,5 ml einer 33%igen Lösung von Trimethylamin in Aethanol zugesetzt und das Gemisch wird 18 Stunden gerührt. Der entstandene Niederschlag wird abfiltriert und getrocknet. Man erhält 237 mg 3-[6,7,8,9-Tetrahydro-8 -(trimethylammoniomethyl)pyrido[1,2-a]indol-10-yl] -4-(1-methyl-3-indolyl)-1H-pyrrol-2,5-dion-trifluormethansulfonat, Smp. 320-324 °C.

Beispiel 4

265 mg Trifluormethansulfonsäureanhydrid in 40 ml Dichlormethan werden bei 0 °C unter einer Stickstoffatmosphäre mit einer Suspension von 200 mg des Pyrroldions von Beispiel 1 und 100 mg Collidin in 30 ml Dichlormethan behandelt. Nach 5 Stunden werden 0,75 ml einer 33%igen Lösung von Methylamin in Methanol zugesetzt und das Gemisch wird 18 Stunden gerührt. Weitere 0,5 ml der obigen Methylaminlösung werden zugesetzt. Nach 4 Stunden wird das Lösungsmittel abgedampft und der Niederschlag abfiltriert und durch Chromatographie über Silicagel mit Dichlormethan/Methanol/Essigsäure/Wasser (90:18:3:2) gereinigt. Das Produkt wird mit mit Chlorwasserstoff gesättigtem Aethylacetat 2 Stunden gerührt. Der erhaltene Feststoff wird abfiltriert und getrocknet. Man erhält 55 mg 3-[6,7,8,9-Tetrahydro-8 -(methylaminomethyl)pyrido[1,2-a]indol-10-yl] -4-(1-methyl-3-indolyl)-1H-pyrrol-2,5-dion-hydrochlorid, Smp. 337-340 °C.

Beispiel 5

185 mg Trifluormethansulfonsäureanhydrid in 30 ml Dichlormethan werden bei 0 °C unter einer Stickstoffatmosphäre mit einer Suspension von 140 mg des Pyrroldionprodukts von Beispiel 1 und 70 mg Collidin in 25 ml Dichlormethan behandelt. Nach 1,5 Stunden werden 0,8 ml einer 33%igen Lösung von Dimethylamin in Aethanol zugesetzt und das Gemisch wird 2,5 Stunden gerührt. Das Lösungsmittel wird unter vermindertem Druck entfernt und der Rückstand mit Methanol vermischt. Man erhält einen Feststoff, der mit mit Chlorwasserstoff gesättigtem Aethylacetat gerührt wird. Der Feststoff wird abfiltriert und getrocknet. Man erhält 70 mg 3-[6,7,8,9-Tetrahydro-8-(dimethylaminomethyl)pyrido[1,2-a]indol -10-yl]-4-(1-methyl-3-indolyl)-1H-pyrrol -2,5-dion-hydrochlorid, Smp. 335-336 °C.

Beispiel 6

Eine Lösung von 170 mg des Pyrroldionproducts von Beispiel 1 in 55 ml Dichlormethan wird mit 87 mg Methansulfonsäureanhydrid in 1 ml Pyridin behandelt. Die entstandene Lösung wird 1 Stunde unter Stickstoff gerührt. Weitere 30 mg Methansulfonsäureanhydrid werden dann zugesetzt. Nach 1 Stunde wird das Gemisch mit Wasser gewaschen, getrocknet und eingedampft. Kristallisation des Rückstandes aus Aethylacetat/n-Hexan ergibt 150 mg 3-[6,7,8,9-Tetrahydro-8 -(methylsulfonyloxymethyl)pyrido[1,2-a]indol-10-yl] -4-(1-methyl-3-indolyl)-1H-pyrrol-2,5-dion, Smp. 259-261 °C.

Beispiel 7

Eine Lösung von 120 mg des Pyrroldionprodukts von Beispiel 6 in 6 ml DMF und 6 ml 33%iges wässriges Ammoniak wird 6 Stunden auf 140 °C erhitzt. Das abgekühlte Gemisch wird in Wasser geschüttet

und der Niederschlag abfiltriert. Das Produkt wird durch Chromatographie über Silicagel mit Dichlormethan/Essigsäure/Methanol/Wasser (60:18:2:3) gereinigt. Das Vermischen mit Aethylacetat ergibt 50 mg 3-[8-Formamidomethyl-6,7,8,9-tetrahydropyrido[1,2-a]indol -10-yl]-4-(1-methyl-3-indolyl)-1H-pyrrol-2,5-dion, Smp. 332-334 °C.

Beispiel 8

Eine Lösung von 100 mg des Pyrroldionprodukts von Beispiel 6 und 75 mg Thiourea in 5 ml DMF werden 18 Stunden bei 80 °C unter einer Stickstoffatmosphäre erhitzt. Das Lösungsmittel wird abgedampft und der Rückstand durch Chromatographie über Silicagel mit Dichlormethan/Methanol/Essigsäure/Wasser (90:18:3:2) gereinigt. Der Rückstand wird mit Aethylacetat vermischt. Man erhält 80 mg 3-[8-Amidinothiomethyl-6,7,8,9 -tetrahydropyrido[1,2-a]indol-10-yl]-4-(1-methyl-3-indolyl) -1H-pyrrol-2,5-dion-methansulfonat, Smp. 200-205 °C.

Beispiel 9

Analog Beispiel 1, 1. Absatz erhält man aus 3-[7-Acetoxymethyl-6,7,8,9-tetrahydropyrido[1,2-a]indol -10-yl]-4-(1-methyl-3-indolyl)furan-2,5-dion das 3-[6,7,8,9-Tetrahydro-7-hydroxymethylpyrido[1,2-a]indol -10-yl]-4-(1-methyl-3-indolyl)-1H-pyrrol-2,5-dion, Smp. 239-242 °C.

Das Ausgangsfurandion wird wie folgt hergestellt:

a) 6,6 ml einer 1,6M Lösung von n-Butyllithium in n-Hexan werden unter Rühren einer Lösung von 1,11 g Diisopropylamin in 150 ml THF bei -78 °C unter Stickstoff zugesetzt. Das Gemisch wird 5 Minuten auf -20 °C erwärmen gelassen und dann wieder auf -78 °C abgekühlt. 1,85 g 6,7,8,9-Tetrahydropyrido[1,2-a]-indol-6-on in 10 ml THF werden zugetropft. Nach 0,5 Stunden Rühren bei -78 °C werden 1,19 g Aethylchlorformat zugesetzt und das Gemisch wird auf Raumtemperatur erwärmen gelassen. Das Lösungsmittel wird abgedampft und der Rückstand wird zwischen Diäthyläther und 2M Salzsäure aufgeteilt. Die ätherischen Extrakte werden mit gesättigter Natriumbicarbonatlösung gewaschen, getrocknet und eingeengt. Man erhält ein Oel. Dieses wird durch Chromatographie über Silicagel mit Dichlormethan gereinigt. Kristallisation des Produkts aus Methanol ergibt 1.35 g Aethyl-6,7,8,9-tetrahydro-6-oxopyrido[1,2-a]indol -7-carboxylat, Smp. 82-84 °C.

b) 30 ml einer 1M Lösung von Boron in THF werden unter Rühren einer Lösung von 1,25 g des Carboxylats aus a) zugesetzt und die entstandene Lösung wird 2 Stunden unter einer Stickstoffatmosphäre unter Rückfluss erhitzt. 6 Löffelspitzen Silicagel werden der abgekühlten Lösung zugesetzt und das Lösungsmittel wird abgedampft. Der Rückstand wird durch Chromatographie über Silicagel mit Aethylacetat/n-Hexan (1:1) gereinigt. Man erhält ein Oel, das man in 60 ml Dichlormethan enthaltend 1 ml Pyridin und 2 ml Essigsäureanhydrid löst. Nach 18 Stunden wird die Lösung mit 16 ml 2M Salzsäure und 20 ml gesättigter Natriumbicarbonatlösung gewaschen, getrocknet und eingedampft. Eine Lösung des erhaltenen Oels in 60 ml Diäthyläther wird mit 630 mg Oxalylchlorid unter einer Stickstoffatmosphäre behandelt. Dann wird das Lösungsmittel unter vermindertem Druck entfernt und der Rückstand wird in 100 ml Dichlormethan gelöst. 920 mg 1-Methyl-3-indolylessigsäure und 975 mg Triäthylamin werden dieser Lösung zugesetzt. Nach 72 Stunden wird das Lösungsmittel abgedampft und der Rückstand wird durch Chromatographie über Silicagel mit Aethylacetat/n-Hexan (1:1) gereinigt. Kristallisation aus Aethylacetat ergibt 390 mg 3-[7-Acetoxymethyl-6,7,8,9-tetrahydropyrido[1,2-a]indol -10-yl]-4-(1-methyl-3-indolyl)furan-2,5-dion, Smp. 190-193 °C.

Beispiel 10

200 mg Trifluormethansulfonsäureanhydrid in 50 ml Dichlormethan werden unter einer Stickstoffatmosphäre bei 0 °C mit einer Suspension von 150 mg des Pyrroldionprodukts von Beispiel 9 und 75 mg Collidin in 50 ml Dichlormethan behandelt. Nach 2 Stunden werden 4 ml einer 33%igen wässrigen Ammoniaklösung zugesetzt und das Gemisch wird auf Raumtemperatur erwärmen gelassen. Das Gemisch wird mit Wasser gewaschen, getrocknet und eingedampft. Der Rückstand wird durch Chromatographie über Silicagel mit Dichlormethan/Methanol/Aceton/Wasser (90:18:3:2) gereinigt. Kristallisation aus Dichlormethan/n-Hexan ergibt 85 mg 3-[7-Aminomethyl-6,7,8,9-tetrahydropyrido[1,2-a]indol -10-yl]-4-(1-methyl-3-indolyl)-1H-pyrrol-2,5-dion, Smp. 160-165 °C.

Beispiel 11

Eine Lösung von 120 mg des Pyrroldionprodukts von Beispiel 9 in 80 ml Dichlormethan wird mit 2 ml Pyridin und 100 mg Methansulfonsäureanhydrid unter einer Stickstoffatmosphäre behandelt. Nach 18 Stunden Rühren wird das Gemisch mit 2M Salzsäure und gesättigter Natriumbicarbonatlösung gewaschen, getrocknet und eingedampft. Eine Lösung des erhaltenen Produkts in 40 ml 200 mg Thiourea enthaltendem Aethanol wird 72 Stunden unter Rückfluss erhitzt. Das Lösungsmittel wird abgedampft und der Rückstand durch Chromatographie über Silicagel mit Dichlormethan/Methanol/Aceton/Wasser (90:18:3:2) gereinigt. Kristallisation aus Methanol/Dichlormethan ergibt 30 mg 3-[7-Amidinothiomethyl-6,7,8,9 -tetrahydropyrido-[1,2-a]indol-10-yl]-4-(1-methyl -3-indolyl)-1H-pyrrol-2,5-dion-methansulfonat, Smp. 195-198 °C.

Beispiel 12

Eine Lösung von 72 mg 3-(6,7,8,9-Tetrahydropyrido[1,2-a]indol-10-yl)-4-(1-methyl -3-indolyl)furan-2,5-dion in 5 ml DMF und 5 ml 33%iges wässriges Ammoniak wird 4 Stunden auf 140 °C erhitzt. Die entstandenen Kristalle werden abfiltriert und getrocknet. Man erhält 50 mg 3-(6,7,8,9-Tetrahydropyrido[1,2-a]indol-10-yl)-4-(1-methyl -3-indolyl)-1H-pyrrol-2,5-dion, Smp. 286-289 °C.

Das Ausgangsfurandion wird wie folgt hergestellt:

a) Eine Lösung von 1,03 g Aethyl-6,7-dihydro-9-hydroxypyrido[1,2-a]indol-8-carboxylat in 20 ml Aethanol, 10 ml Wasser und 10 ml konzentrierter Salzsäure wird 3 Stunden auf 80 °C erhitzt. Die Lösungsmittel werden abgedampft. Man erhält 740 mg 7,8-Dihydropyrido[1,2-a]indol-9(6H)-on, Smp. 138-140 °C.

b) Eine Lösung von 740 mg des Produkts von a), 600 mg Hydrazinhydrat und 440 mg Kaliumhydroxid in 2 ml Aethanol und 4 ml Diäthylenglykol werden unter Rückfluss 1,5 Stunden auf 100 °C und dann 2 Stunden auf 180 °C erhitzt. 50 ml Dichlormethan werden zugesetzt und die organische Phase wird mit 2M Salzsäure und Wasser gewaschen. Das Lösungsmittel wird abgedampft. Man erhält 405 mg 6,7,8,9-Tetrahydropyrido[1,2-a]indol.

c) 350 mg Oxalylchlorid werden einer Lösung von 450 mg des Produkts von b) in 13 ml Dichlormethan bei 0 °C zugetropft. Nach 2 Stunden Rühren wird das Lösungsmittel abgedampft und der Rückstand wird in Dichlormethan gelöst. 497 mg 1-Methyl-3-indolylessigsäure und 0,73 ml Triäthylamin werden dieser Lösung zugesetzt und das Gemisch wird 60 Stunden gerührt. Das Lösungsmittel wird abgedampft und der Rückstand wird durch Chromatographie über Silicagel mit Dichlormethan gereinigt. Vermischen des Produkts mit Aethylacetat ergibt 100 mg 3-(6,7,8,9-Tetrahydropyrido[1,2-a]indol-10-yl)-4-(1-methyl -3-indolyl)furan-2,5-dion, Smp. 276-278 °C.

Beispiel 13

Analog Beispiel 1, 1. Absatz erhält man aus
3-[8-(2-Acetoxyäthyl)-6,7,8,9-tetrahydropyrido[1,2-a]indol -10-yl]-4-(1-methyl-3-indolyl)furan-2,5-dion das
3-[6,7,8,9-Tetrahydro-8-(2-hydroxyäthyl)pyrido[1,2-a]indol -10-yl]-4-(1-methyl-3-indolyl)-1H-pyrrol-2,5-dion, Smp. 261-263 °C.

Das Ausgangsfurandion wird wie folgt hergestellt:

a) Eine Lösung von 6,52 g 8-(2-Acetoxyäthyl)-6,7,8,9-tetrahydro -9-oxopyrido[1,2-a]indol in 48 ml Dichlormethan wird mit 2,5 ml Aethandithiol und 3,13 ml Titaniumtetrachlorid behandelt. Die entstandene Lösung wird 18 Stunden unter Stickstoff unter Rückfluss erhitzt. Weitere 4 ml Aethandithiol und 9 ml Titantetrachlorid werden zugesetzt und das Erhitzen wird 4,5 Stunden weitergeführt. Das Gemisch wird mit Wasser gewaschen, getrocknet und eingedampft. Der Rückstand wird durch Chromatographie über Silicagel mit Aethylacetat/Petroläther (1:3) gereinigt. Man erhält 7,7 g 8′-(2-Acetoxyäthyl)-7′,8′-dihydrospiro-[1,3-dithiolan -2′,9′(6′H)-pyrido[1,2-a]indol].

b) Eine Lösung von 5 g des Produkts von a) in 200 ml Aethanol wird 3,5 Stunden zusammen mit 8 Löffelspitzen Raney-Nickel geschüttelt. Das Gemisch wird filtriert und der Filterkuchen mit Aethanol gewaschen. Die vereinigten Filtrate werden eingedampft und der Rückstand wird durch Chromatographie über Silicagel mit Aethylacetat/Petroläther (1:2) gereinigt. Man erhält 620 mg 8-(2-Acetoxyäthyl)-6,7,8,9-tetrahydropyrido[1,2-a]indol.

c) 1.19 g Oxalylchlorid werden einer Lösung von 2,29 g des Produkts von b) in 50 ml Diäthyläther bei 0 °C zugetropft. Nach 2,5 Stunden wird das Lösungsmittel abgedampft und der Rückstand in Dichlormethan gelöst. 1,68 g 1-Methyl-3-indolylessigsäure und 2,45 ml Triäthylamin werden dieser Lösung zugesetzt und das Gemisch wird 18 Stunden unter Stickstoff unter Rückfluss erhitzt. Das Lösungsmittel wird abgedampft und der Rückstand durch Chromatographie über Silicagel mit Aethylacetat/Petroläther

(1:2) gereinigt. Kristallisation aus Aethylacetat ergibt 625 mg 3-[8-(2-Acetoxyäthyl)-6,7,8,9-tetrahydropyrido[1,2-a]indol -10-yl]-4-(1-methyl-3-indolyl)-furan-2,5-dion, Smp. 159-161 °C.

Beispiel 14

Eine Lösung von 115 mg des Produkts von Beispiel 13c) in 1 ml DMF und 2 ml 33%igem wässrigem Ammoniak wird 4 Stunden auf 140 °C erhitzt. Das abgekühlte Gemisch wird eingedampft und der Rückstand durch Chromatographie über Silicagel mit Aethylacetat/Petroläther (2:1) gereinigt. Kristallisation aus Aethylacetat/Petroläther ergibt 13 mg 3-[8-(2-Acetoxyethyl)-6,7,8,9-tetrahydropyrido[1,2-a]indol -10-yl]-4-(1-methyl-3-indolyl)-1H-pyrrol-2,5-dion, Smp. 272-274 °C.

Beispiel 15

Eine Lösung von 500 mg des Pyrroldionprodukts von Beispiel 13 in 50 ml Dichlormethan wird mit 218 mg Methansulfonsäureanhydrid und 1 ml Pyridin behandelt. Die entstandene Lösung wird 1 Stunde unter einer Stickstoffatmosphäre gerührt. Weitere 20 mg Methansulfonsäureanhydrid werden zugesetzt und das Rühren wird 0,5 Stunden fortgesetzt. Das Gemisch wird mit Wasser gewaschen, getrocknet und eingedampft. Kristallisation des Rückstandes aus Aethylacetat/Petroläther ergibt 540 mg 3-[6,7,8,9-Tetrahydro-8 -(2-methylsulfonyloxyäthyl)pyrido[1,2-a]indol-10-yl]-4 -(1-methyl-3-indolyl)-1H-pyrrol-2,5-dion, Smp. 244-245 °C.

Beispiel 16

Eine Lösung von 500 mg des Pyrroldionprodukts von Beispiel 15 und 250 mg Natriumazid in 10 ml DMF wird 3 Stunden bei 70 °C erhitzt. Das Lösungsmittel wird abgedampft und der Feststoff zwischen Aethylacetat und Wasser verteilt. Unlösliches Material wird abfiltriert und getrocknet. Man erhält 425 mg 3-[8-(2-Azidoäthyl)-6,7,8,9-tetrahydropyrido[1,2-a]indol -10-yl]-4-(1-methyl-3-indolyl)-1H-pyrrol-2,5-dion, Smp. 262-264 °C.

Beispiel 17

200 mg des Pyrroldionprodukts von Beispiel 16 in 70 ml Methanol enthaltend 40 mg 10%iges Pd/C wird 48 Stunden unter einer Wasserstoffatmosphäre unter einem Druck von 3 Atmosphären geschüttelt. Die Oberfläche wird abdekantiert und eingedampft. Der Rückstand wird mit 50 ml gesättiger Salzsäurelösung in Aethylacetat behandelt und durch Chromatographie über Silicagel mit Dichlormethan/Methanol/Essigsäure/Wasser (60:18:2:3) gereinigt. Kristallisation aus Aethylacetat ergibt 20 mg 3-[8-(2-Aminoäthyl)-6,7,8,9-tetrahydropyrido[1,2-a]indol -10-yl]-4-(1-methyl-3-indolyl)-1H-pyrrol-2,5-dion, Smp. 160-165 °C.

Beispiel 18

Analog Beispiel 12, 1. Absatz, erhält man aus
3-[2,3-Dihydro-1H-pyrrolo[1,2-a]indol-9-yl]-4-(1-methyl -3-indolyl)furan-2,5-dion das 3-[2,3-Dihydro-1H-pyrrolo[1,2-a]indol-9-yl]-4-(1-methyl -3-indolyl)-1H-pyrrol-2,5-dion, Smp. 260-270 °C.

Das Ausgangsfurandion wird wie folgt hergestellt:

175 mg Oxalylchlorid werden einer Lösung von 200 mg 2,3-Dihydro-1H-pyrrolo[1,2-a]indol in 7 ml Diäthyläther bei 0 °C unter einer Stickstoffatmosphäre zugetropft. Nach 1 Stunde wird das Lösungsmittel unter vermindertem Druck entfernt und der Rückstand in 14 ml Dichlormethan gelöst. 245 mg 1-Methyl-3-indolylessigsäure und 265 mg Triäthylamin werden dieser Lösung zugesetzt und das Gemisch wird 72 Stunden gerührt. Das Lösungsmittel wird unter vermindertem Druck entfernt und der Rückstand durch Chromatographie über Silicagel mit Aethylacetat/Petroläther (1:2) gereinigt. Kristallisation aus Aethylacetat ergibt 70 mg 3-[2,3-Dihydro-1H-pyrrolo[1,2-a]indol-9-yl]-4-(1-methyl -3-indolyl)furan-2,5-dion, Smp. 125-130 °C.

Beispiel 19

Analog Beispiel 1, 1. Absatz, erhält man aus
3-[2-Acetoxymethyl-2,3-dihydro-1H-pyrrolo[1,2-a]indol -9-yl]-4-(1-methyl-3-indolyl)furan-2,5-dion das

3-[2,3-Dihydro-2-hydroxymethyl-1H-pyrrolo[1,2-a]indol -9-yl]-4-(1-methyl-3-indolyl)-1H-pyrrol-2,5-dion, Smp. 238-240 °C.

Das Ausgangsfurandion wird wie folgt hergestellt:

a) 6 Löffelspitzen Raney-Nickel werden einer Lösung von 5,08 g of Aethyl-2,3-dihydro-1-oxo-1H-pyrrolo-[1,2-a]indol-2-carboxylat in 180 ml Aethanol und 90 ml Wasser zugesetzt. Das Gemisch wird 10 Stunden unter Rückfluss erhitzt und dann werden weitere 3 Löffelspitzen Raney-Nickel zugesetzt. Das Erhitzen wird 5,5 Stunden fortgesetzt, worauf man das Gemisch abkühlt und filtriert. Der Filterkuchen wird mit Aethylacetat und Dichlormethan gewaschen. Die vereinigten Filtrate werden eingedampft und der Rückstand wird durch Chromatographie über Silicagel mit Diäthyläther/Petroläther (1:2) gereinigt. Kristallisation aus Methanol ergibt 635 mg Aethyl-2,3-dihydro-1H-pyrrolo[1,2-a]indol-2-carboxylat, Smp. 55-57 °C.

b) 4 ml einer 1M Lösung von $LiAlH_4$ in THF werden einer Lösung von 750 mg des Produkts von a) in 30 ml THF zugesetzt. Nach 1 Stunde werden 30 ml gesättigter Ammoniumchloridlösung zugesetzt und das Gemisch wird eingedampft. Der Rückstand wird mit Dichlormethan extrahiert und der organische Extrakt getrocknet und eingedampft. Kristallisation des Rückstandes aus Diäthyläther/Petroläther ergibt 355 mg 2,3-Dihydro-2-hydroxymethyl-1H-pyrrolo[1,2-a]indol, Smp. 76-78 °C.

c) Eine Lösung von 355 mg des Produkts von b) in 20 ml Dichlormethan enthaltend 2 ml Essigsäureanhydrid und 2 ml Pyridin wird 2 Stunden gerührt. Die Lösungsmittel werden abgedampft und der Rückstand wird zwischen Dichlormethan und Wasser aufgeteilt. Die organische Phase wird getrocknet und eingedampft. Man erhält 420 mg 2-Acetoxymethyl-2,3-dihydro-1H-pyrrolo[1,2-a]indol.

d) 290 mg Oxalylchlorid werden einer Lösung von 420 mg des Produkts von c) in 40 ml Diäthyläther unter einer Stickstoffatmosphäre zugetropft. Nach 1 Stunde wird das Lösungsmittel unter vermindertem Druck entfernt und der Rückstand in Dichlormethan gelöst. 420 mg 1-Methyl-3-indolylessigsäure und 485 mg Triäthylamin werden dieser Lösung zugesetzt und das Gemisch wird 72 Stunden gerührt. Das Lösungsmittel wird abgedampft und der Rückstand durch Chromatographie über Silicagel mit Aethylacetat/Petroläther (1:1) gereinigt. Kristallisation aus Aethylacetat ergibt 90 mg 3-[2-Acetoxymethyl-2,3-dihydro-1H-pyrrolo[1,2-a]indol -9-yl]-4-(1-methyl-3-indolyl)furan-2,5-dion, Smp. 208-211 °C.

Beispiel 20

Eine Lösung von 150 mg 3-[2-t-Butoxycarbonyl-1,2,3,4 -tetrahydropyrazino[1,2-a]indol-10-yl]-4-(1-methyl -3-indolyl)furan-2,5-dion in 4 ml DMF und 8 ml einer 33%igen wässrigen Ammoniaklösung wird 4 Stunden auf 140 °C erhitzt. Das Gemisch wird mit Aethylacetat extrahiert und der organische Extrakt mit Wasser gewaschen, getrocknet und eingedampft. Das entstandene Produkt wird durch Chromatographie über Silicagel mit Dichlormethan/Methanol/Essigsäure/Wasser gereinigt. Das entstandene Imid wird in 30 ml Aethanol und 5 ml 2M Salzsäure gelöst und die entstandene Lösung 2 Stunden unter Rückfluss erhitzt. Abdampfen des Lösungsmittels und Mischen des Rückstandes mit Aethylacetat ergeben 35 mg 3-[1,2,3,4-Tetrahydropyrazino[1,2-a]indol-10-yl]-4 -(1-methyl-3-indolyl)-1H-pyrrol-2,5-dion-hydrochloride, Smp. 268-270 °C.

Das Ausgangsfurandion wird wie folgt hergestellt:

a) Eine Lösung von 450 mg 1,2,3,4-Tetrahydropyrazino[1,2-a]indol in 30 ml Dichlormethan wird bei 0 °C unter einer Stickstoffatmosphäre mit 303 mg Triäthylamin und 615 mg Di(t-butyl)dicarbonat behandelt. Das Gemisch wird 4 Stunden bei 0 °C gerührt und dann mit gesättigter Natriumbicarbonatlösung gewaschen, getrocknet und eingedampft. Kristallisation des erhaltenen Oels aus Methanol ergibt 580 mg t-Butyl-1,2,3,4-tetrahydropyrazino[1,2-a]indol-2-carboxylat, Smp. 103-105 °C.

b) 230 mg Oxalylchlorid werden unter Rühren einer Lösung von 450 mg des Produkts von a) in 30 ml Diäthyläther bei 0 °C zugetropft. Nach Rühren wird die Lösung eingedampft und der Rückstand in 50 ml Dichlormethan gelöst. 360 mg 1-Methyl-3-indolylessigsäure und 350 mg Triäthylamin werden zugesetzt und das Gemisch wird 90 Stunden gerührt. Das Lösungsmittel wird abgedampft und der Rückstand durch Chromatographie über Silicagel mit Aethylacetat/Petroläther (2:3) gereinigt. Man erhält 180 mg 3-[2-t-Butoxycarbonyl-1,2,3,4 -tetrahydropyrazino[1,2-a]indol-10-yl]-4-(1-methyl -3-indolyl)furan-2,5-dion, Smp. 125-127 °C nach Kristallisation aus Aethylacetat/n-Hexan.

Beispiel 21

Analog Beispiel 12, 1. Absatz, erhält man aus
3-(5,6-Dihydro-4H-pyrrolo[3,2,1-ij]quinolin-1-yl) -4-(1-methyl-3-indolyl)furan-2,5-dion das 3-(5,6-Dihydro-4H-pyrrolo[3,2,1-ij]quinolin-1-yl) -4-(1-methyl-3-indolyl)-1H-pyrrol-2,5-dion, Smp. 285-288 °C.

Das Ausgangsfurandion wird wie folgt hergestellt:

1,22 g Oxalylchlorid werden einer Lösung von 1,5 g 5,6-Dihydro-4H-pyrrolo[3,2,1-ij]chinolin in 60 ml Dichlormethan unter einer Stickstoffatmosphäre zugetropft. Nach 1 Stunde wird das Lösungsmittel unter vermindertem Druck entfernt und der Rückstand in 120 ml Dichlormethan gelöst. 1,9 g 1-Methyl-3-indolylessigsäure und 2,02 g Triäthylamin werden dieser Lösung zugesetzt und das Gemisch wird 18 Stunden gerührt. Das Lösungsmittel wird unter vermindertem Druck entfernt und der Rückstand durch Chromatographie über Silicagel mit Aethylacetat/Petroläther (1:2) gereinigt. Weitere Reinigung durch Chromatographie mittels Dichlormethan und Kristallisation aus Aethylacetat ergeben 690 mg 3-(5,6-Dihydro-4H-pyrrolo[3,2,1-ij]chinolin-1-yl) -4-(1-methyl-3-indolyl)furan-2,5-dion, Smp. 217-219 °C.

Beispiel 22

Analog Beispiel 1, 1. Absatz, erhält man aus
3-[5-Acetoxymethyl-5,6-dihydro -4H-pyrrolo[3,2,1-ij]chinolin-1-yl]-4-(1-methyl -3-indolyl)furan-2,5-dion das
3-[5,6-Dihydro-5-hydroxymethyl-4H-pyrrolo[3,2,1-ij]chinolin -1-yl]-4-(1-methyl-3-indolyl) -1H-pyrrol-2,5-dion, Smp. 223-225 °C.

Dse Ausgangsfurandion wird wie folgt hergestellt:

a) 33,4 ml einer 1,6M Lösung von n-Butyllithium in Hexan werden einer Lösung von 8,13 ml Diisopropylamin in 420 ml THF bei -78 °C unter einer Stickstoffatmosphäre zugesetzt. Nach 0,5 Stunden werden 4,6 g 1,2,5,6-Tetrahydro-4-oxo-4H-pyrrolo[3,2,1-ij]chinolin zugesetzt und das Gemisch wird 0,5 Stunden bei -78 °C gerührt. 2,77 ml Aethylchloroformat werden zugesetzt und das Rühren wird 1 Stunde fortgesetzt. Die Reaktion wird durch Zusatz von Wasser unterbrochen und das Gemisch wird eingedampft. Der Rückstand wird durch Chromatographie über Silicagel mit Aethylacetat/Petroläther (1:2) gereinigt. Kristallisation aus Diäthyläther ergibt 2,8 g Aethyl-1,2,5,6-tetrahydro-4-oxo -4H-pyrrolo[3,2,1-ij]-chinolin-5-carboxylat, Smp. 88-90 °C.

b) 15 ml einer 1M Lösung von Boran in THF werden einer Lösung von 2,8 g des Produkts von a) in 100 ml THF zugesetzt und die entstandene Lösung wird 2 Stunden unter Rückfluss erhitzt. Weitere 55 ml Boran werden zugesetzt und das Erhitzen wird 12 Stunden fortgeführt. Das Lösungsmittel wird unter vermindertem Druck entfernt. Wasser und 2M Salzsäure werden zugesetzt und das Gemisch wird mit Dichlormethan extrahiert. Das Lösungsmittel wird abgedampft und der Rückstand in Diäthyläther gelöst. Die Lösung wird mit 12 ml einer 1M Lösung von $LiAlH_4$ in Diäthyläther behandelt und das Gemisch 18 Stunden unter einer Stickstoffatmosphäre gerührt. Wasser wird zugesetzt und das Gemisch mit Dichlormethan extrahiert. Entfernen des Lösungsmittel unter vermindertem Druck ergibt 1,4 g 1,2,5,6-Tetrahydro-4H-pyrrolo[3,2,1-ij]chinolin-5-methanol.

c) Eine Lösung von 1,4 g des Produkts von b) in 50 ml Dichlormethan wird mit 4 ml Essigsäureanhydrid und 2 ml Pyridin behandelt. Nach 4 Stunden werden weitere 4 ml Essigsäureanhydrid zugesetzt und das Gemisch wird 18 Stunden gerührt, das Lösungsmittel wird unter vermindertem Druck entfernt und der Rückstand zwischen Wasser und Dichlormethan verteilt. Die organische Phase wird eingedampft und der Rückstand in Toluol gelöst und 18 Stunden in Toluol in Gegenwart von 250 mg 10%igem Pd/C unter Rückfluss erhitzt. Weitere 250 mg 10%iges Pd/C werden zugesetzt und das Erhitzen wird 20 Stunden weitergeführt. Das Gemisch wird filtriert und das Filtrat eingedampft. Der Rückstand wird durch Chromatographie über Silicagel mit Aethylacetat/Petroläther (1:2) gereinigt. Man erhält 350 mg 5-Acetoxymethyl-5,6-dihydro-4H-pyrrolo[3,2,1-ij]chinolin.

d) 315 mg Oxalylchlorid werden einer Lösung von 570 mg des Produkts von c) in 15 ml Dichlormethan und unter einer Stickstoffatmosphäre zugetropft. Das Lösungsmittel wird unter vermindertem Druck entfernt und der Rückstand in Dichlormethan gelöst. 472 mg 1-Methyl-3-indolylessigsäure und 505 mg Triäthylamin werden zugesetzt und das Gemisch wird 72 Stunden gerührt. Das Lösungsmittel wird unter vermindertem Druck entfernt und der Rückstand durch Chromatographie über Silicagel mit Dichlormethan gereinigt. Kristallisation aus Aethylacetat/n-Hexan ergibt 140 mg 3-[5-Acetoxymethyl-5,6-dihydro -4H-pyrrolo[3,2,1-ij]chinolin-1-yl]-4-(1-methyl -3-indolyl)furan-2,5-dion, Smp. 198-200 °C.

Beispiel 23

Analog Beispiel 11 erhält man aus dem Pyrroldionprodukt von Beispiel 22 3-[5-Amidinothiomethyl-5,6-dihydro-4H-pyrrolo[3,2,1-ij]chinolin-1-yl]-4-(1-methyl-3-indolyl) -1H-pyrrol-2,5-dion-methansulfonat, Smp. 190-195 °C.

Beispiel 24

Analog Beispiel 2 erhält man aus dem Pyrroldionprodukt von Beispiel 22 3-[5-Aminomethyl-5,6-dihydro -4H-pyrrolo[3,2,1-ij]chinolin-1-yl]-4-(1-methyl-3-indolyl) -1H-pyrrol-2,5-dion-hydrochlorid, Smp. 248-250 °C.

Beispiel 25

Analog Beispiel 1, 1. Absatz, erhält man aus 3-[8-Acetoxymethyl-6,7,8,9-tetrahydropyrido[1,2-a]indol -10-yl]-4-phenylfuran-2,5-dion (erhalten wie beschrieben im letzten Absatz von Beispiel 1, jedoch unter Verwendung von Phenylessigsäure anstelle von 1-Methyl-3-indolylessigsäure) das 3-[6,7,8,9-Tetrahydro-8-hydroxymethylpyrido[1,2-a]indol -10-yl]-4-phenyl-1H-pyrrol-2,5-dion, Smp. 276-278 °C.

Beispiel 26

Analog Beispiel 1, 1. Absatz, erhält man aus 4-[8-Acetoxymethyl-6,7,8,9-tetrahydropyrido[1,2-a]indol -10-yl]-3-(3-benzo[b]thienyl)furan-2,5-dion (erhalten wie beschrieben im letzten Absatz von Beispiel 1, jedoch unter Verwendung von 3-Benzo[b]-thienylessigsäure anstelle von 1-Methyl-3-indolylessigsäure) das 3-(3-Benzo[b]thienyl)-4-[6,7,8,9-tetrahydro -8-hydroxymethylpyrido[1,2-a]indol-10-yl]-1H-pyrrol -2,5-dion, Smp. 226-227 °C.

Beispiel 27

Analog Beispiel 1, 1. Absatz, erhält man aus 3-[8-Acetoxymethyl-6,7,8,9-tetrahydropyrido[1,2-a]indol -10-yl]-4-(1-naphthyl)furan-2,5-dion (erhalten wie beschrieben im letzten Absatz von Beispiel 1, jedoch unter Verwendung von 1-Naphthylessigsäure anstelle von 1-Methyl-3-indolylessigsäure) das 3-[6,7,8,9-Tetrahydro-8-hydroxymethylpyrido[1,2-a]indol -10-yl]-4-(1-naphthyl)-1H-pyrrol-2,5-dion, Smp. 221-222 °C.

Beispiel 28

Analog Beispiel 10 erhält man aus dem Produkt von Beispiel 19 3-[2-Aminomethyl-2,3-dihydro-1H-pyrrolo[1,2-a]indol-9-yl] -4-(1-methyl-3-indolyl)-1H-pyrrol-2,5-dion, Smp. 208-211 °C.

Beispiel 29

Analog Beispiel 10 erhält man aus dem Pyrroldionprodukt von Beispiel 25 3-[8-Aminomethyl-6,7,8,9-tetrahydropyrido[1,2-a]indol -10-yl]-4-phenyl-1H-pyrrol-2,5-dion, Smp. 249-250 °C.

Beispiel 30

Eine Suspension von 100 mg des Pyrroldionprodukts von Beispiel 20 in 10 ml Dichlormethan wird unter Stickstoff mit 0,08 ml Triäthylamin und 86 mg Phenylchloroformat behandelt. Das Gemisch wird 2 Stunden gerührt und dann das Lösungsmittel abgedampft. Chromatographie des Rückstandes über Silicagel mit Aethylacetat/n-Hexan (1:1) ergibt ein gummiartiges Produkt, das in einem Gemisch von 5 ml Isopropanol und 10 ml 33%igem wässrigem Ammoniak gelöst wird. Das Gemisch wird mit Wasser verdünnt und mit Dichlormethan extrahiert. Die vereinigten Dichlormethanextrakte werden getrocknet und eingedampft. Kristallisation des Rückstandes aus Aethylacetat/n-Hexan ergibt 45 mg 3-[1,2,3,4-Tetrahydro -2-phenoxycarbonylpyrazino[1,2-a]indol-10-yl] -4-(1-methyl-3-indolyl)-1H-pyrrol-2,5-dion, Smp. 160-165 °C.

Beispiel 31

a) Eine Lösung von 80 mg des Pyrroldionprodukts von Beispiel 20 in 20 ml Dichlormethan wird mit 10 ml einer 5%igen wässrigen Natriumbicarbonatlösung behandelt. Das Gemisch wird unter Rückfluss mit einer Lösung von 125 mg Trifluoracetamidoacetylchlorid in 5 ml Dichlormethan behandelt. Nach 10 Stunden werden die Phasen getrennt und die organische Phase wird getrocknet und eingedampft. Chromatographie des Rückstandes über Silicagel mit Aethylacetat/n-Hexan (2:1) und Kristallisation aus Aethylacetat/n-Hexane ergeben 70 mg 3-[2-Trifluoracetamidoacetyl-1,2,3,4 -tetrahydropyrazino[1,2-a]-

indol-10-yl]-4-(1-methyl -3-indolyl)-1H-pyrrol-2,5-dion, Smp. 170-172 ° C.

b) Eine Lösung von 65 mg des Produkts von a) in 10 ml Methanol wird mit 5 ml einer 33%igen wässrigen Ammoniaklösung behandelt. Nach 4 Stunden wird das Lösungsmittel abgdampft und der Rückstand zwischen Dichlormethan und Wasser verteilt. Die organische Phase wird mit Wasser gewaschen, getrocknet und eingedampft. Chromatographie des Rückstandes über Silicagel mit Chloroform/Methanol/Essigsäure/Wasser (60:18:2:3) ergibt ein gummiartiges Produkt, das in Eisessig gelöst und mit 20 ml 1M Salzsäure behandelt wird. Abdampfen des Lösungsmittels und Mischen des Rückstandes mit Diäthyläther ergeben 35 mg 3-[2-Aminoacetyl-1,2,3,4-tetrahydropyrazino[1,2-a]indol -10-yl]-4-(1-methyl-3-indolyl)-1H-pyrrol-2,5-dion, Smp. 235 ° C (Zersetzung).

Beispiel 32

a) Eine Lösung von 100 mg des Pyrroldionprodukts von Beispiel 20 in 40 ml Dichlormethan wird unter einer Stickstoffatmosphäre mit 125 mg 1,1-Carbonyldiimidazol behandelt und das Gemisch 24 Stunden gerührt. Die Lösung wird mit Wasser gewaschen, getrocknet und eingedampft. Vermischen des Rückstandes mit Aethylacetat ergibt 84 mg 3-[1,2,3,4-Tetrahydro-2 -1-(imidazolylcarbonyl)pyrazino[1,2-a]indol-10-yl] -4-(1-methyl-3-indolyl)-1H-pyrrol-2,5-dion, Smp. 295 ° C (Zersetzung).

b) 80 mg des Produkts von a) werden in einem Gemisch von 20 ml DMF und 20 ml 33%igem wässrigem Ammoniak gelöst. Das Gemisch wird 17 Stunden gerührt und das Lösungsmittel eingedampft. Chromatographie des Rückstandes über Silicagel mit Methanol/Aethylacetat (1:9) ergibt 45 mg 3-[2-Carbamoyl-1,2,3,4-tetrahydropyrazino[1,2-a]indol -10-yl]-4-(1-methyl-3-indolyl)-1H-pyrrol-2,5-dion, Smp. 295 ° C (Zersetzung) nach Kristallisation aus Methanol.

Beispiel 33

Eine Lösung von 505 mg des Pyrroldionprodukts von Beispiel 2 in 20 ml DMF wird mit einer Lösung von 222 mg 1,1-Thiocarbonyldiimidazol in 5 ml THF behandelt. Nach 17 Stunden wird das Lösungsmittel abgedampft und der Rückstand durch Chromatographie über Silicagel mit Methanol/Dichlormethan (1:99) gereinigt. Vermischen mit n-Hexan ergibt 297 mg 3-[6,7,8,9-Tetrahydro-8-isothiocyanatopyrido[1,2-a]indol -10-yl]-4-(1-methyl-3-indolyl)-1H-pyrrol-2,5-dion, Smp. 285-287 ° C.

Beispiel 34

250 mg des Pyrroldionprodukts von Beispiel 2 werden unter Rühren in ein Gemisch von 25 ml Dichlormethan und 15 ml 5%igem wässrigem Natriumbicarbonat zugegeben. Das Gemisch wird mit 1 ml Benzoylchlorid behandelt und 10 Stunden gerührt. Die Phasen werden getrennt und die organische Phase wird getrocknet und eingedampft. Chromatographie des Rückstandes über Silicagel mit Methanol/Dichlormethan (7:93) gefolgt durch Vermischen mit n-Hexan ergibt 220 mg 3-[8-Benzamidomethyl-6,7,8,9-tetrahydropyrido[1,2-a]indol-10-yl]-4-(1-methyl-3-indolyl)-1H-pyrrol-2,5-dion, Smp. 297-303 ° C.

Beispiel 35

Eine Lösung von 150 mg 3-[7-Acetoxy-6,7,8,9-tetrahydropyrido[1,2-a]indol-10-yl] -4-(1-methyl-3-indolyl)-furan-2,5-dion in 6 ml DMF und 6 ml 33%igem wässrigem Ammoniak wird 6 Stunden auf 150 ° C erhitzt. Das Gemisch wird mit Aethylacetat extrahiert und die organischen Extrakte werden mit Wasser gewaschen, getrocknet und eingedampft. Kristallisation des Rückstandes aus Aethylacetat ergibt 120 mg 3-[6,7,8,9-Tetrahydro-7-hydroxypyrido[1,2-a]indol-10-yl] -4-(1-methyl-3-indolyl)-1H-pyrrol-2,5-dion, Smp. 252-255 ° C.

Das Ausgangsfurandion wird wie folgt hergestellt:

a) Eine Lösung von 14,0 g Indol-2-methanol in 500 ml Dichlormethan wird mit 76,4 g aktiviertem Mangan-IV-oxid gerührt. Nach 1 Stunde wird der Feststoff abfiltriert und mit Dichlormethan gewaschen. Die vereinigten Filtrate werden konzentriert und 33 g Carbäthoxymethylen-triphenylphosphoran werden zugesetzt. Die entstandene Lösung wird unter Rückfluss unter einer Stickstoffatmosphäre erhitzt. Das Lösungsmittel wird abgedampft. Das erhaltene Oel wird durch Chromatographie über Silicagel mit Aethylacetat/n-Hexan (1:3) gereinigt. Das Produkt ist ein 20:1-Gemisch der E/Z-Isomeren. Kristallisation aus Methanol ergibt 11,3 g Aethyl-(E)-2-indolyl-2-propenoat, Smp. 120-122 ° C.

b) Eine Lösung von 7,2 g des Produkts von a) in 120 ml DMF wird mit 1,47 g einer 60%igen Dispersion von Natriumhydrid in Mineralöl behandelt. Die entstandene Lösung wird auf 0 ° C abgekühlt und 7,17 g t-Butylbromacetat werden unter einer Stickstoffatmosphäre zugesetzt. Nach 2 Stunden wird das Gemisch

in 100 ml 2M Salzsäure geschüttet und mit Aethylacetat extrahiert. Die vereinigten organischen Extrakte werden mit Wasser gewaschen, getrocknet und eingedampft. Das erhaltene Oel wird durch Chromatographie über Silicagel mit Diäthyläther/Petroläther (1:3) gereinigt. Kristallisation aus Diäthyläther/n-Hexan ergibt 8,1 g Aethyl-(E)-3-[1-t-butoxycarbonylmethyl-2-indolyl] -2-propenoat, Smp. 66-68°C.

c) Eine Lösung von 8,0 g des Produkts von b) in 300 ml Aethanol wird unter einer Wasserstoffatmosphäre mit 800 mg 10%igem Pd/C geschüttelt. Der Katalysator wird abfiltriert und mit Aethylacetat gewaschen. Die vereinigten Filtrate werden eingedampft. Man erhält ein Oel, das in THF gelöst wird. Die Lösung wird einer Lösung von 2,8 g Kalium-t-butoxid in THF unter einer Stickstoffatmosphäre zugesetzt. Dann wird das Gemisch 1 Stunde gerührt und das Lösungsmittel eingedampft. Der Rückstand wird zwischen Aethylacetat und 2M Salzsäure aufgeteilt. Die organische Phase wird mit Wasser gewaschen, getrocknet und eingedampft. Der Rückstand wird durch Chromatographie über Silicagel mit Diäthyläther/n-Hexan (1:4) gereinigt. Man erhält 4,55 g t-Butyl-6,7,8,9-tetrahydro-7-oxopyrido[1,2-a]indol -6-carboxylat.

d) Eine Lösung von 4,5 g des Produkts von c) in 200 ml Toluol wird mit 4 Löffelspitzen Silicagel behandelt und das Gemisch 3 Stunden unter einer Stickstoffatmosphäre unter Rückfluss erhitzt. Der Feststoff wird abfiltriert und mit Toluol gewaschen. Die vereinigten Filtrate werden eingedampft. Man erhält 2,5 g 8,9-Dihydropyrido[1,2-a]indol-7(6H)-on, Smp. 126-128°C nach Kristallisation aus Diäthyläther/n-Hexan.

e) 190 mg Natriumborhydrid werden unter Rühren einer Lösung von 650 mg des Produkts von d) in 50 ml Methanol unter einer Stickstoffatmosphäre zugegeben. Das Gemisch wird gerührt und dann in 100 ml gesättigte Ammoniumchloridlösung geschüttet. Das Gemisch wird mit Aethylacetat extrahiert und die vereinigten Extrakte werden getrocknet und eingedampft. Nach Kristallisation des Produktes aus Diäthyläther/n-Hexan erhält man 500 mg 6,7,8,9-[Tetrahydro-7-hydroxypyrido[1,2-a]indol, Smp. 99-100°C.

f) Eine Lösung von 500 mg des Produkts von e) in 5 ml Pyridin und 2 ml Essigsäureanhydrid wird 8 Stunden gerührt. Das Gemisch wird in 50 ml 2M Salzsäure geschüttet und mit Aethylacetat extrahiert. Die vereinigten organischen Extrakte werden mit 5%iger Natriumbicarbonatlösung und Wasser gewaschen, getrocknet und eingedampft. Man erhält 25 mg 7-Acetoxy-6,7,8,9-tetrahydropyrido[1,2-a]indol, Smp. 90-95°C nach Kristallisation aus Diäthyläther/n-Hexan.

g) 320 mg Oxalylchlorid werden einer Lösung von 500 mg des Produkts von f) in 50 ml Diäthyläther unter einer Stickstoffatmosphäre zugegeben. Dann wird das Lösungsmittel unter vermindertem Druck entfernt und der Rückstand in 50 ml Dichlormethan gelöst. 378 mg 1-Methyl-3-indolylessigsäure und 505 mg Triäthylamin werden dieser Lösung zugesetzt und das Gemisch wird 72 Stunden gerührt. Das Lösungsmittel wird unter vermindertem Druck entfernt und der Rückstand durch Chromatographie über Silicagel mit Aethylacetat/n-Hexan (1:1) gereinigt. Nach Kristallisation aus Aethylacetat erhält man 160 mg 3-[7-Acetoxy-6,7,8,9-tetrahydropyrido[1,2-a]indol-10-yl] -4-(1-methyl-3-indolyl)furan-2,5-dion, Smp. 272-275°C.

Beispiel 36

Eine Lösung von 85 mg 3-[7-t-Butoxyformamido-6,7,8,9-tetrahydropyrido[1,2-a]indol -10-yl]-4-(1-methyl-3-indolyl)furan-2,5-dion in 5 ml DMF und 5 ml einer 33%igen wässrigen Ammoniaklösung wird 1 Stunde auf 100°C erhitzt. Das abgekühlte Gemisch wird zwischen Aethylacetat und Wasser verteilt. Die organische Phase wird mit Wasser gewaschen, getrocknet und eingedampft. Nach Kristallisation aus Aethylacetat/n-Hexan erhält man 70 mg 3-[7-t-Butoxyformamido-6,7,8,9-tetrahydropyrido[1,2-a]indol -10-yl]-4-(1-methyl-3-indolyl)-1H-pyrrol-2,5-dion, Smp. 159-163°C.

Das Ausgangsfurandion wird wie folgt hergestellt:

a) Eine Suspension von 555 mg 8,9-Dihydropyrido[1,2-a]indol-7(6H)-on und 4,62 g Ammoniumacetat in 15 ml Methanol wird mit 250 mg Natriumcyanoborhydride behandelt. Das Gemisch wird gerührt und dann zwischen Aethylacetat und Wasser verteilt. Die organische Phase wird getrocknet und das Lösungsmittel unter vermindertem Druck entfernt. Das zurückbleibende Oel wird über Silicagel mit 10% Methanol in Dichlormethan chromatographiert. Das erhaltene Indolin wird in Toluol gelöst und mit 50 mg 10%igem Pd/C unter Rückfluss erhitzt. Der Katalysator wird abfiltriert und mit Toluol gewaschen. Die vereinigten Filtrate werden eingedampft. Man erhält 170 mg 7-Amino-6,7,8,9-tetrahydropyrido[1,2-a]indol.

b) 225 mg Di-t-butyldicarbonat werden unter Rühren einer Lösung von 175 mg des Produkts von a) und 112 mg Triäthylamin in 20 ml Dichlormethan bei 0°C unter einer Stickstoffatmosphäre zugefügt. Nach 18 Stunden wird die Lösung mit gesättigter Natriumbicarbonatlösung gewaschen, getrocknet und eingedampft. Nach Kristallisation aus Diäthyläther erhält man 240 mg 7-t-Butoxyformamido-6,7,8,9-tetrahydropyrido[1,2-a]indol, Smp. 137-139°C.

c) 127 mg Oxalylchlorid werden einer Lösung von 240 mg des Produkts von b) in 30 ml Diäthyläther unter einer Stickstoffatmosphäre zugegeben. Nach 10 Minuten wird das Lösungsmittel unter vermindertem Druck entfernt und der Rückstand in 30 ml Dichlormethan gelöst. 170 mg 1-Methyl-3-indolylessigsäureund 200 mg Triäthylamin werden der entstandenen Lösung zugesetzt und das Gemisch wird 72 Stunden gerührt. Das Lösungsmittel wird unter vermindertem Druck entfernt und der Rückstand wird durch Chromatographie über Silicagel mit Aethylacetat/n-Hexan (1:2) gereinigt. Kristallisation aus Aethylacetat/n-Hexan ergibt 100 mg 3-[7-t-Butoxyformamido-6,7,8,9-tetrahydropyrido[1,2-a]indol -10-yl]-4-(1-methyl-3-indolyl)furan-2,5-dion, Smp. 141-145 ° C.

Beispiel 37

Eine gesättigte Lösung von Chlorwasserstoff in 30 ml Aethylacetat wird unter Rühren einer Suspension von 60 mg des Pyrroldionprodukts von Beispiel 36 in 50 ml Aethylacetat zugesetzt und das Gemisch 18 Stunden gerührt. Das Lösungsmittel wird unter vermindertem Druck entfernt und der Rückstand mit Aethylacetat vermischt. Man erhält 35 mg 3-[7-Amino-6,7,8,9-tetrahydropyrido[1,2-a]indol-10-yl] -4-(1-methyl-3-indolyl)-1H-pyrrol-2,5-dion-hydrochlorid, Smp. 260-265 ° C.

Beispiel 38

Eine Lösung von 80 mg 3-[8-t-Butoxyformamido-6,7,8,9-tetrahydropyrido[1,2-a]indol -10-yl]-4-(1-methyl-3-indolyl)-furan-2,5-dion in 2 ml DMF und 2 ml 33%igem wässrigem Ammoniak wird 1 Stunde auf 100 ° C erhitzt. Die Lösung wird abgekühlt und man erhält 60 mg 3-[8-t-Butoxyformamido-6,7,8,9-tetrahydropyrido-[1,2-a]indol -10-yl]-4-(1-methyl-3-indolyl)1H-pyrrol-2,5-dion, Smp. 153-155 ° C.

Das Ausgangsfurandion wird wie folgt hergestellt:

a) Eine Lösung von 300 mg Natriumhydroxid in 5 ml Wasser wird unter Rühren einer Lösung von 1,35 g des Carboxylatprodukts von Beispiel 1b) in 25 ml Aethanol zugesetzt und das Gemisch 15 Minuten untr Rückfluss erhitzt. 2 ml 2M Salzsäure und 10 ml Wasser werden zugesetzt und der Niederschlag wird abfiltriert und getrocknet. Man erhält 1,14 g 6,7,8,9-Tetrahydropyrido[1,2-a]indol-8-carbonsäure, Smp. 244-246 ° C.

b) Eine Suspension von 900 mg des Produkts von a) in 1 ml Wasser und 20 ml Aceton wird auf 0 ° C abgekühlt und mit 490 mg Triäthylamin gefolgt von 576 mg Aethylchorofomat behandelt. Nach 0,5 Stunden werden 345 mg Natriumazid in 1 ml Wasser zugesetzt und das Gemisch wird 1 Stunde bei 0 ° C gerührt. Das Lösungsmittel wird unter vermindertem Druck entfernt und der Rückstand mit Dichlormethan extrahiert. Die Extrakte werden eingedampft und der Rückstand wird durch Chromatographie über Silicagel mit Dichlormethan gereinigt. Der erhaltene Feststoff wird in 10 ml Toluol gelöst und 4 Stunden unter einer Stickstoffatmosphäre auf 100 ° C erhitzt. Das Lösungsmittel wird abgedampft. Man erhält 700 mg 6,7,8,9-Tetrahydropyrido[1,2-a]indol-8-isocyanat, Smp. 87-89 ° C.

c) 4 ml einer 2M Natriumhydroxidlösung werden einer Lösung von 700 ml des Produkts von b) in 50 ml THF zugesetzt und die Lösung wird über Nacht gerührt. Das Lösungsmittel wird unter vermindertem Druck entfernt und der Rückstand mit Dichlormethan extrahiert. Der Dichlormethanextrakt wird eingedampft und man erhält ein Amin, das in Dichlormethan gelöst wird. 645 mg Di-t-butylcarbonat und 300 ml Triäthylamin werden bei 0 ° C zugesetzt und das Gemisch wird 22 Stunden unter Rühren auf Raumtemperatur erwärmen gelassen. Das Gemisch wird mit Natriumbicarbonatlösung gewaschen und die organische Phase getrocknet. Das Lösungsmittel wird unter vermindertem Druck entfernt und der Rückstand mit Diäthyläther extrahiert. Die ätherischen Extrakte werden eingedampft und der Feststoff mit Petroläther vermischt. Man erhält 550 mg 8-t-Butoxyformamido-6,7,8,9-tetrahydropyrido[1.2-a]indol, Smp. 155-157 ° C.

d) 256 mg Oxalylchlorid werden einer Lösung von 550 mg des Produkts von c) in 10 ml Diäthyläther bei 0 ° C unter einer Stickstoffatmosphäre zugetropft. Nach 1 Stunde wird das Lösungsmittel unter vermindertem Druck entfernt und der Rückstand in Dichlormethan gelöst. 363 mg 1-Methyl-3-indolylessigsäure und 390 mg Triäthylamin werden zugesetzt und das Gemisch wird 40 Stunden gerührt. Das Lösungsmittel wird unter vermindertem Druck entfernt und der Rückstand durch Chromatographie über Silicagel mit Aethylacetat/Petroläther (1:2) gereinigt. Nach Kristallisation aus Diäthyläther/Petroläther erhält man 220 mg 3-[8-t-Butoxyformamido-6,7,8,9-tetrahydropyrido[1,2-a]indol -10-yl]-4-(1-methyl-3-indolyl)furan-2,5-dion, Smp. 155-160 ° C.

Beispiel 39

Analog Beispiel 37 erhält man aus dem Pyrroldionprodukt von Beispiel 38 3-[8-Amino-6,7,8,9-tetrahydropyrido[1,2-a]indol-10-yl] -4-(1-methyl-3-indolyl)-1H-pyrrol-2,5-dion-hydrochlorid, Smp. 310-315 °C.

Beispiel 40

Eine Lösung von 320 mg 3-[4-(2-Acetoxyethyl)-5,6-dihydro -4H-pyrrolo[3,2,1-ij]chinolin-1-yl]-4-(1-methyl -3-indolyl)furan-2,5-dion in 2 ml DMF und 2 ml 33%igem wässrigem Ammoniak wird 12 Stunden auf 140 °C erhitzt. Wasser wird dem abgekühlten Gemisch zugesetzt. Nach Filtrierung erhält man 210 mg 3-[4-(2-Hydroxyäthyl)-5,6-dihydro -4H-pyrrolo[3,2,1-ij]chinolin-1-yl)-4-(1-methyl-3-indolyl) -1H-pyrrol-2,5-dion, Smp. 214-215 °C nach Kristallisation aus Aethylacetat.

Das Ausgangsfurandion wird wie folgt hergestellt:

a) 25 ml einer 1,6M Lösung von n-Butyllithium in n-Hexan werden einer Lösung von 4,04 g Diisopropylamin in 20 ml THF bei 0 °C unter Stickstoff zugesetzt. Nach 10 Minuten wird die Lösung unter Rühren auf -78 °C abgekühlt und es wird eine Lösung von 9,28 g t-Butylacetat in 20 ml THF zugesetzt. Nach 10 Minuten werden 3,46 g 1,2,5,6-Tetrahydro-4H-pyrrolo[3,2,1-ij]chinolin-4-on in 20 ml THF gefolgt von 8 ml Bortrifluorid-diäthylätherat zugesetzt. Das Gemisch wird bei -78 °C gerührt und dann werden 20 ml Pyrrolidin zugesetzt. Das Gemisch wird zwischen Aethylacetat und Wasser verteilt und die organischen Extrakte werden mit Wasser und Natriumchloridlösung gewaschen, getrocknet und eingedampft. Der Rückstand wird durch Chromatographie über Silicagel mit Aethylacetat/Petroläther (1:3) gereinigt. Man erhält 4,1 g t-Butyl-(E)-(1,2,5,6-tetrahydro-4H-pyrrolo[3,2,1-ij]chinolin -4-yliden)acetat, Smp. 105-107 °C.

b) Eine Lösung von 4 g des Produkts von a) in 400 ml Methanol wird 18 Stunden unter einer Stickstoffatmosphäre mit 280 mg 10%igem Pd/C geschüttelt. Der Katalysator wird abfiltriert und die Filtrate werden eingedampft. 1,99 g des entstandenen Oels in 100 ml Diäthyläther werden mit 5 ml einer 1M Lösung von $LiAlH_4$ in Diäthyläther behandelt und das Gemisch wird 2 Stunden gerührt. Wasser wird zugegeben und das Produkt wird mit Aethylacetat extrahiert. Die Aethylacetatextrakte werden getrocknet und unter vermindertem Druck eingeengt. Man erhält 1,44 g 1,2,5,6-Tetrahydro-4H-pyrrolo[3,2,1-ij]-chinolin-4-äthanol.

c) 1.44 g des Produkts von b) in 40 ml Dichlormethan werden mit 10 ml Essigsäureanhydrid und 5 ml Pyridin behandelt. Die Lösung wird gerührt und dann eingedampft. Der Rückstand wird in Dichlormethan gelöst, die Lösung mit Wasser gewaschen und die organische Phase abgetrennt, getrocknet und eingeengt. Man erhält 1,65 g 4-(2-Acetoxyäthyl)-1,2,5,6-tetrahydro -4H-pyrrolo[3,2,1-ij]chinolin.

d) Eine Lösung von 1,6 g des Produkts von c) in 50 ml Xylol und 100 mg 10%iges Pd/C wird 12 Stunden unter Rückfluss erhitzt. Der Katalysator wird abfiltriert und das Filtrat eingedampft. Man erhält 1,7 g 4-(2-Acetoxyäthyl)-5,6-dihydro-4H-pyrrolo[3,2,1-ij]chinolin.

e) 935 mg Oxalylchlorid werden unter Rühren einer Lösung von 1,7 g des Produkts von d) in 45 ml Dichlormethan unter einer Stickstoffatmosphäre zugestzt. Nach 1 Stunde wird das Lösungsmittel unter vermindertem Druck entfernt und der Rückstand in 90 ml Dichlormethan gelöst. 1,38 g 1-Methyl-3-indolylessigsäure und 1,48 g Triäthylamin werden der Lösung zugesetzt und das Gemisch wird 18 Stunden gerührt. Das Lösungsmittel wird unter vermindertem Druck entfernt und der Rückstand durch Chromatographie über Silicagel mit Aethylacetat/Petroläther (1:2) gereinigt. Kristallisation aus Methanol/Wasser ergibt 280 mg 3-[4-(2-Acetoxyäthyl)-5,6-dihydro -4H-pyrrolo[3,2,1-ij]chinolin-1-yl]-4-(1-methyl -3-indolyl)furan-2,5-dion, Smp. 143-146 °C.

Beispiel 41

Eine Lösung von 400 mg 3-[8-(t-Butoxyformamidomethyl)-6,7,8,9 -tetrahydropyrido[1,2-a]indol-10-yl]-4-(1-methyl -3-indolyl)furan-2,5-dion in 50 ml DMF und 50 ml Wasser wird mit 2,5 g Hydroxylaminhydrochlorid und 2,5 g Kaliumcarbonat behandelt und die Lösung auf 100 °C erhitzt. Die Lösungsmittel werden abgedampft und der Rückstand in Dichlormethan gelöst, mit Wasser gewaschen und getrocknet. Das Lösungsmittel wird unter vermindertem Druck entfernt und der Rückstand aus Aethylacetat/Petroläther kristallisiert. Man erhält 190 mg 3-[8-(t-Butoxyformamidomethyl)-6,7,8,9 -tetrahydropyrido[1,2-a]indol-10-yl]-1-hydroxy -4-(1-methyl-3-indolyl)pyrrol-2,5-dion, Smp. 238-240 °C.

Das Ausgangsfurandion wird wie folgt hergestellt:

a) 2,4 g Methansulfonsäureanhydrid und 2 ml Triäthylamin werden unter Rühren einer Lösung von 2.01 g 6,7,8,9-Tetrahydropyrido[1,2-a]indol-8-methanol in 40 ml Dichlormethan unter einer Stickstoffatmosphäre zugegeben. Nach 18 Stunden wird das Gemisch mit gesättigter Natriumbicarbonatlösung gewaschen,

getrocknet und eingedampft. 1,8 g des erhaltenen Oels werden in 10 ml Isopropanol und 5 ml 33%igem wässrigem Ammoniak gelöst und das Gemisch wird 10 Stunden auf 80 °C erhitzt. Das Lösungsmittel wird unter vermindertem Druck entfernt und der Rückstand zwischen Dichlormethan und gesättigter Natriumbicarbonatlösung verteilt. Die organische Phase wird getrocknet und eingedampft. Man erhält 1,3 g 8-Aminomethyl-6,7,8,9-tetrahydropyrido[1,2-a]indol, Smp. 85-90 °C.

b) 1,09 g Di-t-butyldicarbonat werden unter Rühren einer Lösung von 890 mg 8-Aminomethyl-6,7,8,9-tetrahydropyrido[1,2-a]indol und 920 mg Triäthylamin in 60 ml Dichlormethan bei 0 °C unter einer Stickstoffatmosphäre zugegeben. Nach 72 Stunden wird die organische Phase mit gesättigter Natriumbicarbonatlösung gewaschen, getrocknet und eingedampft. Der Rückstand wird aus Petroläther kristallisiert. Man erhält 1,03 g 8-(t-Butoxyformamidomethyl)-6,7,8,9 -tetrahydropyrido[1,2-a]indol, Smp. 80-85 °C.

c) 445 mg Oxalylchlorid werden einer Lösung von 1 g des Produkts von b) in 20 ml Diäthyläther unter einer Stickstoffatmosphäre bei 0 °C zugetropft. Nach 1 Stunde wird das Lösungsmittel unter vermindertem Druck entfernt und der Rückstand in Dichlormethan gelöst. 630 mg 1-Methyl-3-indolylessigsäure und 920 µl Triäthylamin werden dieser Lösung zugegeben und das Gemisch wird 72 Stunden gerührt. Das Lösungsmittel wird unter vermindertem Druck entfernt und der Rückstand durch Chromatographie über Silicagel mit Aethylacetat/Petroläther (1:2) gereinigt. Der entstandene Feststoff wird aus Diäthyläther kristallisiert. Man erhält 315 mg 3-[8-(t-Butoxyformamidomethyl)-6,7,8,9 -tetrahydropyrido[1,2-a]-indol-10-yl]-4-(1-methyl -3-indolyl)furan-2,5-dion, Smp. 124-126 °C.

## Beispiel 42

Analog Beispiel 37 erhält man aus dem Produkt von Beispiel 41 3-[8-Aminomethyl-6,7,8,9-tetrahydropyrido[1,2-a]indol -10-yl]-1-hydroxy-4-(1-methyl-3-indolyl)pyrrol -2,5-dion-hydrochlorid, Smp. 280-282 °C.

## Beispiel 43

Analog Beispiel 11 erhält man aus dem Produkt von Beispiel 40 3-[4-(2-Amidinothioäthyl)-5,6-dihydro -4H-pyrrolo[3,2,1-ij]chinolin-1-yl]-4-(1-methyl-3-indolyl) -1H-pyrrol-2,5-dion-methansulfonat, Smp. 185-190 °C.

## Beispiel 44

Analog Beispiel 2 erhält man aus dem Produkt von Beispiel 40 3-[4-(2-Aminoäthyl)-5,6-dihydro -4H-pyrrolo[3,2,1-ij]chinolin-1-yl]-4-(1-methyl -3-indolyl)-1H-pyrrol-2,5-dion-hydrochlorid, Smp. 193-195 °C.

## Beispiel 45

Analog Beispiel 2 erhält man aus dem Produkt von Beispiel 26 3-[8-Aminomethyl-6,7,8,9-tetrahydropyrido[1,2-a]indol -10-yl]-4-(3-benzo[b]thienyl)-1H-pyrrol -2,5-dion-hydrochlorid, Smp. 285-287 °C.

## Beispiel 46

Analog Beispiel 1, 1. Absatz, erhält man aus 3-[8-Acetoxymethyl-6,7,8,9-tetrahydropyrido[1,2-a]indol -10-yl]-4-(2-naphthyl)furan-2,5-dion (erhalten wie beschrieben im letzten Absatz von Beispiel 1, jedoch unter Verwendung von 2-Naphthylessigsäure anstelle von 1-Methyl-3-indolylessigsäure) 3-[6,7,8,9-Tetrahydro-8-hydroxymethylpyrido[1,2-a]indol -10-yl]-4-(2-naphthyl)-1H-pyrrol-2,5-dion, Smp. 260-263 °C.

## Beispiel 47

Analog Beispiel 2 erhält man aus dem Produkt von Beispiel 46 3-[8-Aminomethyl-6,7,8,9-tetrahydropyrido[1,2-a]indol -10-yl]-4-(2-naphthyl)-1H-pyrrol-2,5-dion-hydrochlorid, Smp. >300 °C.

## Beispiel 48

Analog Beispiel 10 erhält man aus dem Produkt von Beispiel 27 3-[8-Aminomethyl-6,7,8,9-tetrahydropyrido[1,2-a]indol -1-yl]-4-(1-naphthyl)-1H-pyrrol-2,5-dion, Smp. 167-169 °C.

Beispiel 49

Analog Beispiel 1, 1. Absatz, erhält man aus 1,3 g 3-[9-Acetoxymethyl-7,8,9,10-tetrahydro -6H-azepino-[1,2-a]indol-11-yl]-4-(1-methyl-3-indolyl)furan -2,5-dion 520 mg 3-[7,8,9,10-Tetrahydro-9-hydroxymethyl-6H -azepino[1,2-a]indol-11-yl]-4-(1-methyl-3-indolyl) -1H-pyrrol-2,5-dion, Smp. 268-270 °C.

Das Ausgangsfurandion wird wie folgt hergestellt:

a) Eine Lösung von 18,9 g Aethylindol-2-carboxylat in 100 ml DMF wird einer Suspension von 2,64 g Natriumhydrid in 50 ml DMF zugegeben. Nach 1 Stunde wird eine Lösung von 20,9 g Aethyl-5-bromvalerat in 100 ml DMF zugetropft. Nach 48 Stunden wird das Gemisch in Wasser geschüttet, mit Dichlormethan extrahiert und die vereinigten Dichlormethanextrakte werden mit Wasser gewaschen, getrocknet und eingeengt. Man erhält 26,2 g Aethyl-1-(4-äthoxycarbonylbutyl)indol-2-carboxylat.

b) Eine Lösung des Produkts von a) in 50 ml THF wird unter Rühren einer Suspension von 11,2 g Kalium-t-butoxid in 150 ml THF zugegeben. Nach 36 Stunden wird das Gemisch eingeengt und der Rückstand in ein Gemisch von Wasser und Diäthyläther geschüttet. Die organische Phase wird getrocknet und eingeengt. Chromatographie des Rückstandes über Silicagel mit Dichlormethan/Methanol (9:1) ergibt ein Feststoff, welches aus Aethylacetat/n-Hexan umkristallisiert wird. Man erhält 6,1 g Aethyl-7,8-dihydro-10-hydroxy-6H-azepino[1,2-a]indol -9-carboxylat, Smp. 74-81 °C.

c) Eine Lösung von 5,5 g des Produkts von b) in 200 ml Aethanol wird mit 11 Löffelspitzen Raney-Nickel und 400 ml Wasser behandelt. Das Gemisch wird 4 Stunden unter Rückfluss erhitzt. Das abgekühlte Gemisch wird filtriert und der Rückstand mit Aethylacetat gewaschen. Das Filtrat wird mit Aethylacetat extrahiert. Die vereinigten Extrakte werden getrocknet und eingedampft. Man erhält ein Oel, welches durch Chromatographie über Silicagel mit Dichlormethan gereinigt wird. Man erhält 2,5 g Aethyl-7,8,9,10-tetrahydro-6H-azepino[1,2-a]indol -9-carboxylat, Smp. 69-70 °C.

d) Eine Lösung des Produkts von c) in 50 ml THF wird einem Gemisch von 0,45 g $LiAlH_4$ in 20 ml THF zugetropft. Das Gemisch wird 2 Stunden gerührt und nach Zugabe von Wasser mit Diäthyläther extrahiert. Die vereinigten Extrakte werden getrocknet und eingedampft. Chromatographie des Rückstandes über Silicagel mit Dichlormethan ergibt 1,90 g 7,8,9,10-Tetrahydro-9-hydroxymethyl -6H-azepino[1,2-a]indol, Smp. 109-111 °C.

e) Eine Lösung von 1,8 g des Produkts von d) in 100 ml Diäthyläther wird bei 0 °C mit 1,70 g Essigsäureanhydrid und 0,66 g Pyridin behandelt. Nach 8 Stunden werden weitere 5 g Pyridin zugegeben und das Gemisch wird 76 Stunden gerührt. Die Lösungsmittel werden unter vermindertem Druck entfernt und der Rückstand wird über Silicagel mit Dichlormethan chromatographiert. Man erhält 1,98 g 9-Acetoxymethyl-7,8,9,10-tetrahydro -6H-azepino[1,2-a]indol, Smp. 65 °C.

f) Eine Lösung von 1,90 g des Produkts von e) in 50 ml Dichlormethan wird auf 0 °C abgekühlt und mit 1,03 g Oxalylchlorid behandelt. Nach 2 Stunden wird das Lösungsmittel abgedampft, der Rückstand in Dichlormethan gelöst und einer Lösung von 1,5 g 1-Methylindol-3-essigsäure und 1,86 g Triäthylamin in Dichlormethan zugetropft. Das Gemisch wird eingedampft und der Rückstand über Silicagel mit Dichlormethan enthaltend 5% Methanol/Volumen chromatographiert. Der erhaltene Feststoff wird aus Aethylacetat/n-Hexan kristallisiert. Man erhält 1,55 g 3-[9-Acetoxymethyl-7,8,9,10-tetrahydro -6H-azepino[1,2-a]-indol-11-yl]-4-(1-methyl-3-indolyl)furan -2,5-dion, Smp. 164-166 °C.

Beispiel 50

Analog Beispiel 12 erhält man aus 0,50 g 3-[7,8,9,10-Tetrahydro-6H-azepino[1,2-a]indol-11-yl] -3-(1-methyl-3-indolyl)furan-2,5-dion 0,43 g 3-[7,8,9,10-Tetrahydro-6H-azepino[1,2-a]indol-11-yl] -3-(1-methyl-3-indolyl)-1H-pyrrol-2,5-dion, Smp. >300 °C.

Das Ausgangsfurandion erhält man wie folgt:

1,5 g Oxalylchlorid werden einer eiskalten Lösung von 2,0 g 7,8,9,10-Tetrahydro-6H-azepino[1,2-a]indol (J. Org. Chem. 33, 1968, 4286) in 50 ml Dichlormethan zugetropft. Das Gemisch wird 2 Stunden gerührt. Das Lösungsmittel wird im Vakuum entfernt und der Rückstand in Dichlormethan gelöst. Die Lösung wird einer Lösung von 2,2 g 1-Methyl-3-indolylessigsäureund 2,73 g Triäthylamin in 50 ml Dichlormethan zugegeben. Das Gemisch wird gerührt und dann eingedampft. Der Rückstand wird über Silicagel mit Dichlormethan chromatographiert und man erhält 1,0 g 3-[7,8,9,10-Tetrahydro-6H-azepino[1,2-a]indol-11-yl] -3-(1-methyl-3-indolyl)furan-2,5-dion, Smp. 257-259 °C.

Beispiel 51

Eine Lösung von 150 mg des Produkts von Beispiel 49 und 146 mg 2,6-Lutidin in 50 ml Dichlormethan wird einer Lösung von 290 mg Trifluormethansulfonsäureanhydrid bei 0 °C zugetropft. Nach 3 Stunden werden 25 ml 33%iges wässriges Ammoniak zugesetzt, das Gemisch wird 16 Stunden gerührt, dann mit Dichlormethan extrahiert und die vereinigten Extrakte werden getrocknet und eingedampft. Chromatographie des Rückstandes über Silicagel mit Dichlormethan/Methanol/Essigsäure/Wasser (90:18:3:2) ergibt 50 mg 3-[9-Aminomethyl-7,8,9,10-tetrahydro -6H-azepino[1,2-a]indol-11-yl]-4-(1-methyl-3-indolyl) -1H-pyrrol-2,5-dion-acetat, Smp. 215 °C (Zersetzung).

Beispiel 52

Ein Gemisch von 40 mg des Produkts von Beispiel 51, 20 ml Natriumbicarbonat und 25 mg 3,5-Dimethyl-$N^2$-nitro-1-pyrazol-1-carboxamid in 10 ml Aethanol wird 16 Stunden unter Rückfluss erhitzt, dann eingedampft und der Rückstand über Silicagel mit Dichlormethan/Methanol (9:1) chromatographiert. Man erhält 15 mg 3-(1-Methyl-3-indolyl)-4-[7,8,9,10-tetrahydro -9-(2-nitroguanidinomethyl)-6H-azepino[1,2-a]indol-11-yl] -1H-pyrrol-2,5-dion, Smp. 177-178 °C.

Beispiel 53

Analog Beispiel 1, 1. Absatz, erhält man aus 0,20 g 3-[8-Acetoxymethyl-7,8,9,10-tetrahydro -6H-azepino[1,2-a]indol-11-yl]-4-(1-methyl-3-indolyl)furan -2,5-dion 60 mg 3-[7,8,9,10-Tetrahydro-8-hydroxymethyl-6H -azepino[1,2-a]indol-11-yl]-4-(1-methyl-3-indolyl) -1H-pyrrol-2,5-dion, Smp. 109-111 °C.

Das Ausgangsfurandion erhält man wie folgt:

a) Eine Lösung von 5 g Aethyl-6,7-dihydro-9-hydroxypyrido[1,2-a]indol-8-carboxylat (hergestellt wie in Beispiel 1 beschrieben) in 200 ml DMF wird mit 550 mg Natriumhydrid behandelt. Das Gemisch wird unter einer Stickstoffatmosphäre gerührt und dann wird eine Lösung von 3,6 g Aethylbromacetat in 50 ml DMF zugegeben. Nach 16 Stunden wird das Gemisch in Wasser geschüttet und mit Diäthyläther extrahiert. Die vereinigten Extrakte werden mit Wasser gewaschen, getrocknet und eingedampft. Man erhält 4,4 g Aethyl-8-äthoxycarbonyl-6,7,8,9-tetrahydro -9-oxopyrido[1,2-a]indol-8-acetat.

b) Eine Lösung von 5,0 g des Produkts von a) in 200 ml THF wird unter Rühren einer Lösung von 2,0 g Kalium-t-butoxid in 50 ml THF zugetropft. Das Gemisch wird gerührt und dann wird 1 ml Eisessig zugegeben. Das Gemisch wird in Wasser geschüttet und mit Dichlormethan extrahiert. Die vereinigten Extrakte werden getrocknet und eingedampft. Der Rückstand wird über Silicagel mit Dichlormethan/Methanol (95:5) chromatographiert. Man erhält 3,0 g Diäthyl-7,8-dihydro-10-hydroxy-6H-azepino[1,2-a]indol -8,9-dicarboxylat.

c) Ein Gemisch von 2,8 g des Produkts von b) und 0,5 g Borsäure wird auf 150 °C und dann auf 170 °C erhitzt. Eiswasser wird dem abgekühlten Gemisch zugegeben und das Gemisch mit Dichlormethan extrahiert. Die vereinigten Dichlormethanextrakte werden getrocknet und eingedampft. Der Rückstand wird über Silicagel mit Dichlormethan/Methanol (95:5) chromatographiert. Man erhält 2,1 g Aethyl-7,8,9,10-tetrahydro-10-oxo-6H-azepino[1,2-a]indol -8-carboxylat.

d) 2,1 g des Produkts von c) werden in 80 ml Aethanol gelöst und mit 4 Löffelspitzen Raney-Nickel und 50 ml Wasser behandelt. Das Gemisch wird 4 Stunden unter Rückfluss erhitzt, dann abgekühlt und filtriert. Der Rückstand wird mit Aethylacetat gewaschen, die Filtrate werden mit Aethylacetat extrahiert, die vereinigten Extrakte getrocknet und eingedampft. Chromatographie des Rückstandes über Silicagel mit Dichlormethan ergibt 0,89 g Aethyl-7,8,9,10-tetrahydro-6H-azepino[1,2-a]indol -8-carboxylat.

e) 0,85 g des Produkts von d) werden in 50 ml THF gelöst und unter Rühren einer Suspension von 140 mg $LiAlH_4$ in 50 ml THF zugetropft. Nach Zusatz von Wasser wird das Gemisch mit Diäthyläther extrahiert. Die vereinigten Extrakte werden getrocknet und eingedampft. Chromatographie des Rückstandes über Silicagel mit Dichlormethan/Methanol (95:5) ergibt 0,70 g 7,8,9,10-Tetrahydro-8-hydroxymethyl-6H-azepino[1,2-a]indol, Smp. 90-91 °C.

f) 0,70 g des Produkts von e) werden mit 0,66 g Essigsäureanhydrid und 0,39 g Pyridin in 50 ml Diäthyläther behandelt. 1 g Pyridin und 1 g Essigsäure werden zugegeben und das Gemisch wird 16 Stunden gerührt, dann eingedampft und der Rückstand über Silicagel mit Dichlormethan chromatographiert. Man erhält 0,60 g 8-Acetoxymethyl-7,8,9,10-tetrahydro-6H-azepino[1,2-a]indol, Smp. 77-79 °C.

g) Einer Lösung von 0,60 g des Produkts von f) in 50 ml Dichlormethan werden 0,33 g Oxalylchlorid zugetropft. Nach 2 Stunden bei 10 °C wird die Lösung eingedampft und der Rückstand in Dichlormethan gelöst und diese Lösung einer solchen von 0,49 g 1-Methylindol-3-essigsäure und 0,59 g Triäthylamin in

Dichlormethan zugegeben. Nach 16 Stunden wird das Gemisch eingedampft und der Rückstand über Silicagel mit Dichlormethan/Methanol (95:5) chromatographiert. Man erhält 0,51 g 3-[8-Acetoxymethyl-7,8,9,10-tetrahydro -6H-azepino[1,2-a]indol-11-yl]-4-(1-methyl-3-indolyl)furan -2,5-dion, Smp. 70 °C.

Beispiel 54

Eine Lösung von 60 mg des Produkts von Beispiel 53 und 60 mg 2,6-Lutidin in 25 ml Dichlormethan wird einer Lösung von 116 mg Trifluormethansulfonsäureanhydrid in 25 ml Dichlormethan bei 0 °C zugetropft. Nach 3 Stunden werden 25 ml wässriges Ammoniak der Lösung zugegeben. Die organische Phase wird getrocknet und eingeengt. Chromatographie des Rückstandes über Silicagel ergibt 30 mg 3-[8-Aminomethyl-7,8,9,10-tetrahydro-6H -azepino[1,2-a]indol-11-yl]-4-(1-methyl-3-indolyl)-1H-pyrrol-2,5-dion-acetat, Smp. 162-163 °C.

Beispiel 55

Einer Lösung von 0,64 g des Produkts von Beispiel 1 und 0,4 ml 2,4,6-Collidin in 20 ml Dichlormethan wird eine Lösung von 0,75 g Trifluormethansulfonsäureanhydrid in 10 ml Dichlormethan bei 0 °C zugetropft. Nach 2,5 Stunden wird das Gemisch mit 3 ml Piperidin behandelt und 16 Stunden gerührt. Eindampfen und Chromatographie des Rückstandes über Silicagel mit Dichlormethan/Methanol (von 98:2 zu 50:50) ergeben 340 mg 3-[6,7,8,9-Tetrahydro-8 -(1-piperidinomethyl)pyrido[1,2-a]indol-10-yl] -4-(1-methyl-3-indolyl)-1H-pyrrol-2,5-dion. Behandlung dieses Produkts mit gesättigter Chlorwasserstofflösung in Aethylacetat ergibt das Hydrochlorid, Smp. 294 °C (Zersetzung).

Beispiel 56

Einer Lösung von 0,8 g des Produkts von Beispiel 1 und 0,44 g 2,4,6-Collidin in 30 ml Dichlormethan wird einer Lösung von 0,9 g Trifluormethansulfonsäureanhydrid in 10 ml Dichlormethan bei 0 °C zugetropft. Nach 1,5 Stunden wird das Gemisch mit 3,64 g Diisopropylamin behandelt, 16 Stunden gerührt und dann mit Wasser und gesättigter wässriger Natriumbicarbonatlösung gewaschen, getrocknet und eingedampft. Der Feststoff wird in Aethylacetat gelöst und mit einer gesättigten Chlorwasserstofflösung in Aethylacetat behandelt. Entfernen des Lösungsmittels im Vakuum ergibt 260 mg 3-[6,7,8,9-Tetrahydro -8-(diisopropylaminomethyl)pyrido[1,2-a]indol-10-yl] -4-(1-methyl-3-indolyl)-1H-pyrrol-2,5-dion-hydrochlorid, Smp. 187 °C (Zersetzung).

Beispiel 57

Eine Lösung von 1,0 g 3-[8-Acetoxymethyl-6,7,8,9-tetrahydropyrido[1,2-a]indol -10-yl]-4-(3-benzofuranyl)furan-2,5-dion in 100 ml Chloroform wird mit 13,8 ml Hexamethyldisilazan und 2,73 ml Methanol behandelt und 6 Stunden unter Rühren unter einer Stickstoffatmosphäre auf 50 °C erhitzt. Weitere 13,8 g ml Hexamethyldisilazan und 2,73 ml Methanol werden zugesetzt und das Erhitzen wird 16 Stunden fortgesetzt. Es werden noch zweimal die gleichen Mengen Hexamethyldisilazan und Methanol zugesetzt und das Gemisch wird 24 Stunden bei 50 °C gehalten. 20 ml Methanol werden dann zugesetzt und das Gemisch wird 15 Minuten unter Rückfluss erhitzt, dann abgekühlt und eingedampft, der Niederschlag abfiltriert und nacheinander mit Aethylacetat und Methanol vermischt. Man erhält 630 mg 3-[8-Acetoxymethyl-6,7,8,9-tetrahydropyrido[1,2-a]indol -10-yl]-4-(3-benzofuranyl)-1H-pyrrol-2,5-dion, Smp. 234-237 °C.

Das Ausgangsfurandion erhält man wie folgt:

1.7 g Oxalylchlorid wird eine Lösung von 3,3 g 8-Acetoxymethyl-6,7,8,9-tetrahydropyrido[1,2-a]indol in 200 ml Diäthyläther unter einer Stickstoffatmosphäre zugetropft. Nach 15 Minuten wird das Lösungsmittel unter vermindertem Druck entfernt und der Rückstand in Dichlormethan gelöst. 2,4 g 3-Benzofuranylessigsäure und 5,6 ml Triäthylamin werden dieser Lösung zugegeben und das Gemisch wird über Nacht gerührt, das Lösungsmittel unter vermindertem Druck entfernt und der Rückstand durch Chromatographie über Silicagel mit Aethylacetat/Petroläther (1:2) chromatographiert. Kristallisation des Rückstandes aus Aethylacetat/Petroläther ergibt 1,62 g 3-[8-Acetoxymethyl-6,7,8,9-tetrahydropyrido[1,2-a]indol -10-yl]-4-(3-benzofuranyl)furan-2,5-dion, Smp. 214-215 °C.

Beispiel 58

Eine Lösung von 300 mg des Produkts von Beispiel 57 in 40 ml Methanol wird mit 5 ml 2M Natriumhydroxid behandelt. Nach 10 Minuten wird das Gemisch mit 5 ml 2M Salzsäure angesäuert und das Methanol unter vermindertem Druck entfernt und der Rückstand zwischen Aethylacetat und Wasser aufgeteilt. Die organische Phase wird mit Natriumbicarbonatlösung gewaschen und dann getrocknet, die Lösung eingedampft und der Niederschlag abfiltriert. Man erhält 190 mg 3-(3-Benzofuranyl)-4-[6,7,8,9-tetrahydro -8-hydroxymethylpyrido[1,2-a]indol-10-yl] -1H-pyrrol-2,5-dion, Smp. 246-248 °C.

Beispiel 59

Analog Beispiel 2 erhält man aus dem Produkt von Beispiel 58 3-[8-Aminomethyl-6,7,8,9-tetrahydropyrido[1,2-a]indol -10-yl]-4-(3-benzofuranyl)-1H-pyrrol-2,5-dion-hydrochlorid, Smp. 210-212 °C.

Beispiel 60

118 mg Trifluormethansulfonsäureanhydrid in 20 ml Dichlormethan werden bei 0 °C unter einer Stickstoffatmosphäre mit eine Suspension von 90 mg des Produkts von Beispiel 26 und 45 mg Collidin in 20 ml Dichlormethan behandelt. Nach 45 Minuten werden 0,41 ml eine 40%igen Lösung von Dimethylamin in Wasser zugegeben, das Gemisch wird 1,5 Stunden gerührt, die Lösung mit Wasser und Natriumbicarbonatlösung gewaschen und dann getrocknet und eingedampft und die entstandenen Kristalle werden abfiltriert und getrocknet. Man erhält 60 mg 3-(3-Benzo[b]thienyl)-4-[6,7,8,9-tetrahydro -8-dimethylaminomethylpyrido[1,2-a]indol-10-yl] -1H-pyrrol-2,5-dion, Smp. 285-286 °C.

Beispiel 61

546 mg Trifluormethansulfonsäureanhydrid in 80 ml Dichlormethan werden bei 0 °C unter einer Stickstoffatmosphäre mit einer Suspension von 400 mg des Produkts von Beispiel 19 und 208 mg Collidin in 120 ml Dichlormethan behandelt. Nach 1 Stunde werden 1,9 ml 50%iges wässriges Dimethylamin zugesetzt und das Gemisch wird 3 Stunden gerührt, das Lösungsmittel entfernt und der Rückstand einer Chromatographie über Silicagel mit Dichlormethan/Methanol/Aceton (88:10:2) unterworfen. Vermischen mit Aethylacetat gefolgt durch Umkristallisation aus Methanol ergeben 295 mg 3-[2,3-Dihydro-2-dimethylaminomethyl -1H-pyrrolo[1,2-a]indol-9-yl]-4-(1-methyl-3-indolyl) -1H-pyrrol-2,5-dion-trifluormethansulfonat, Smp. 323-325 °C.

Beispiel 62

Eine Lösung von 400 mg 3-[8-Cyano-6,7,8,9-tetrahydropyrido[1,2-a]indol-10-yl] -4-(1-methyl-3-indolyl)-furan-2,5-dion in 12 ml DMF und 12 ml 33%iges wässriges Ammoniak wird 3 Stunden auf 140 °C erhitzt. Das Gemisch wird abgekühlt und der entstandene Feststoff abfiltriert und getrocknet. Man erhält 275 mg 3-[8-Cyano-6,7,8,9-tetrahydropyrido[1,2-a]indol-10-yl] -4-(1-methyl-3-indolyl)-1H-pyrrol-2,5-dion, Smp. 312-313 °C.

Das Ausgangsfurandion erhält man wie folgt:

a) Eine Suspension von 4,0 g des Produkts von Beispiel 38a) in 4,4 ml Wasser und 84 ml Aceton wird auf 0 °C abgekühlt und 2,18 g Triäthylamin und dann 2,56 g Aethylchloroformat werden zugegeben. Die entstandene Lösung wird unter einer Stickstoffatmosphäre gerührt. 0,9 ml 33%iges wässriges Ammoniak werden zugesetzt und das Gemisch wird auf Raumtemperatur erwärmen gelassen. Weitere 0,5 ml 33%iges wässriges Ammoniak werden zugesetzt und das Rühren wird fortgesetzt. Das Lösungsmittel wird abgedampft, der Rückstand mit Dichlormethan extrahiert und die organische Phase mit Wasser gewaschen, getrocknet und eingedampft. Man erhält 2,8 g 6,7,8,9-Tetrahydropyrido[1,2-a]indol-8-carboxamid, Smp. 179-181 °C.

b) 991 mg Trifluoressigsäureanhydrid werden einer Suspension von 1,0 g des Produkts von a) in 15 ml Dioxan bei 10 °C zugetropft. Das Gemisch wird zwischen Dichlormethan und Wasser verteilt, die organische Phase getrocknet und das Lösungsmittel unter vermindertem Druck entfernt. Das eerhaltene Oel wird über Silicagel mit Aethylacetat/Petroläther (1:1) chromatographiert. Man erhält 740 mg 6,7,8,9-Tetrahydropyrido[1,2-a]indol-8-carbonitril, Smp. 116-118 °C.

c) 518 mg Oxalylchlorid werden einer Lösung von 800 mg des Produkts von b) in 100 ml Diäthyläther unter einer Stickstoffatmosphäre zugesetzt. Das Lösungsmittel wird abgedampft und der Rückstand in

Dichlormethan gelöst. 771 mg 1-Methyl-3-indolylessigsäure und 1,24 g Triäthylamin werden dieser Lösung zugegeben, das Gemisch wird über Nacht gerührt, das Lösungsmittel dann unter vermindertem Druck entfernt und der Rückstand durch Chromatographie über Silicagel mit 10% Methanol in Dichlormethan gereinigt. Die das gewünschte Produkt enthaltenden Fraktionen werden eingedampft und die Kristalle abfiltriert und getrocknet. Man erhält 560 mg 3-[8-Cyano-6,7,8,9-tetrahydropyrido[1,2-a]indol-10-yl] -4-(1-methyl-3-indolyl)furan-2,5-dion, Smp. 309-311 °C.

Beispiel 63

Gasförmiger Chlorwasserstoff wird einer Lösung von 200 mg des Produkts von Beispiel 62 in 250 ml Methanol bei 0 °C eingeblasen. Das Lösungsmittel wird dann unter vermindertem Druck entfernt und der Rückstand in 50 ml Dichlormethan und 250 ml Aethanol gelöst. Ammoniak wird in die Lösung eingeblasen und das Lösungsmittel dann abgedampft. Der Rückstand wird durch Chromatographie über Silicagel mit Dichlormethan/Methanol/Essigsäure/Wasser (90:18:3:2) gereinigt. Vermischen mit Aethylacetat ergibt 75 mg 3-[8-Amidino-6,7,8,9-tetrahydropyrido[1,2-a]indol-10-yl] -4-(1-methyl-3-indolyl)-1H-pyrrol-2,5-dion-hydrochlorid, Smp. 237-239 °C.

Beispiel 64

Eine Lösung von 50 mg 3-[8-Carbamoyl-6,7,8,9-tetrahydropyrido[1,2-a]indol-10-yl] -4-(1-methyl-3-indolyl)furan-2,5-dion in 4 ml DMF und 4 ml 33%iges wässriges Ammoniak wird auf 140 °C erhitzt. Das Gemisch wird mit Aethylacetat extrahiert und die organische Phase mit Wasser gewaschen und dann getrocknet. Der grösste Teil des Lösungsmittels wird abgedampft und der entstandene Niederschlag abfiltriert und getrocknet. Man erhält 20 mg 3-[8-[Carbamoyl-6,7,8,9-tetrahydropyrido[1,2-a]indol-10-yl] -4-(1-methyl-3-indolyl)-1H-pyrrol-2,5-dion, Smp. 315-316 °C.

Das Ausgangsfurandion erhält man wie folgt:

178 mg Oxalylchlorid werden einer Lösung von 300 mg des Produkts von Beispiel 62a) in 40 ml Dichlormethan unter einer Stickstoffatmosphäre zugegeben. Dann wird das Lösungsmittel unter vermindertem Druck entfernt und der Rückstand in Dichlormethan gelöst. 265 mg 1-Methyl-3-indolylessigsäure und 424 mg Triäthylamin werden zugesetzt und das Gemisch wird 60 Stunden gerührt, das Lösungsmittel dann unter vermindertem Druck entfernt und der Rückstand durch Chromatographie über Silicagel mit 10% Methanol in Dichlormethan gereinigt. Kristallisation aus Aethylacetat ergibt 70 mg 3-[8-Carbamoyl-6,7,8,9-tetrahydropyrido[1,2-a]indol-10-yl] -4-(1-methyl-3-indolyl)furan-2,5-dion, Smp. 307-309 °C.

Beispiel 65

Analog Beispiel 1, 1. Absatz, erhält man aus 3-[8-Acetoxymethyl-6,7,8,9-tetrahydropyrido[1,2-a]indol -10-yl]-4-(5-methoxy-1-methyl-3-indolyl)furan-2,5-dion 3-[6,7,8,9-Tetrahydro-8-hydroxymethylpyrido[1,2-a]-indol -10-yl]-4-(5-methoxy-1-methyl-3-indolyl) -1H-pyrrol-2,5-dion, Smp. 300-303 °C.

Das Ausgangsfurandion erhält man wie folgt:

0,4 ml Oxalylchlorid werden einer Lösung von 906 mg 8-Acetoxymethyl-6,7,8,9-tetrahydropyrido[1,2-a]-indol in 35 ml Diäthyläther unter einer Stickstoffatmosphäre zugetropft. Dann wird das Lösungsmittel unter vermindertem Druck entfernt und der Rückstand in Dichlormethane gelöst. 940 mg 5-Methoxy-1-methyl-3-indolylessigsäure und 1,16 ml Triäthylamin werden zugesetzt und das Gemisch wird 40 Stunden gerührt, das Lösungsmittel dann unter vermindertem Druck entfernt und der Rückstand durch Chromatographie über Silicagel mit Aethylacetat/n-Hexan (1:2) gereinigt. Kristallisation aus Aethylacetat/Petroläther ergibt 250 mg 3-[8-Acetoxymethyl-6,7,8,9-tetrahydropyrido[1,2-a]indol -10-yl]-4-(5-methoxy-1-methyl-3-indolyl)furan-2,5-dion, Smp. 259-261 °C.

Beispiel 66

Analog Beispiel 2 erhält man aus dem Produkt von Beispiel 65 3-[8-Aminomethyl-6,7,8,9-tetrahydropyrido[1,2-a]indol -10-yl]-4-(5-methoxy-1-methyl-3-indolyl)-1H-pyrrol -2,5-dion-hydrochlorid, Smp. 268-270 °C.

Beispiel 67

Analog Beispiel 1, 1. Absatz, erhält man aus
3-[8-Acetoxymethyl-6,7,8,9-tetrahydropyrido[1,2-a]indol -10-yl]-4-(5-brom-1-methyl-3-indolyl)furan-2,5-dion
3-[6,7,8,9-Tetrahydro-8-hydroxymethylpyrido[1,2-a]indol -10-yl]-4-(5-brom-1-methyl-3-indolyl)-1H-pyrrol-2,5-dion, Smp. 316-318 °C.

Das Ausgangsfurandion erhält man wie folgt:

a) 500 mg einer Dispersion von Natriumhydrid in Mineralöl werden einer Lösung von 1 g 5-Bromindol-3-essigsäure in 50 ml THF zugegeben und das Gemisch wird unter einer Stickstoffatmosphäre 1 Stunde gerührt. 820 mg Methyljodid werden zugesetzt und das Gemisch wird 24 Stunden unter einer Stickstoffatmosphäre gerührt. 5 ml Wasser werden zugesetzt und das Lösungsmittel wird unter vermindertem Druck entfernt, der Rückstand mit 2M Salzsäure behandelt und der entstandene Niederschlag abfiltriert, mit n-Hexan gewaschen und getrocknet. Der entstandene Feststoff wird aus Diäthyläther umkristallisiert. Man erhält 5-Brom-1-methyl-3-indolylessigsäure, Smp. 192-194 °C.

b) 500 mg Oxalylchlorid werden einer Lösung von 900 mg des Produkts von a) in 100 ml Diäthyläther unter einer Stickstoffatmosphäre zugegeben. Dann wird das Lösungsmittel eingedampft und der Rückstand in Dichlormethan gelöst. 800 mg des Produkts von a) und 810 mg Triäthylamin werden zugesetzt und das Gemisch wird 48 Stunden gerührt, das Lösungsmittel dann unter vermindertem Druck entfernt und der Rückstand durch Chromatographie über Silicagel mit Aethylacetat/n-Hexan (1:1) gereinigt. Kristallisation des erhaltenen Feststoffs aus Aethylacetat ergibt 400 mg 3-[8-Acetoxymethyl-6,7,8,9-tetrahydropyrido[1,2-a]indol -10-yl]-4-(5-brom-1-methyl-3-indolyl)furan-2,5-dion, Smp. 215-220 °C.

Beispiel 68

Analog Beispiel 2 erhält man aus dem Produkt von Beispiel 67 3-[8-Aminomethyl-6,7,8,9-tetrahydropyrido[1,2-a]indol -10-yl]-4-(5-brom-1-methyl-3-indolyl)-1H-pyrrol -2,5-dion-hydrochlorid, Smp. >310 °C.

Beispiel 69

Eine Lösung von 200 mg 3-[7-(2-Acetoxyäthyl)-6,7,8,9-tetrahydropyrido[1,2-a]indol -10-yl]-4-(1-methyl-3-indolyl)-furan-2,5-dion in 2 ml DMF und 1 ml 33%iges wässriges Ammoniak wird auf 140 °C erhitzt, dann 1 ml einer 2M Natriumhydroxidlösung der abgekühlten Lösung zugegeben und das Gemisch 2 Stunden gerührt, dann mit 2M Salzsäure angesäuert und eingedampft. Der Rückstand wird zwischen Aethylacetat und Wasser verteilt und die organische Phase getrocknet, das Lösungsmittel eingedampft und der entstandene Feststoff mit Aethylacetat vermischt. Man erhält 115 mg 3-[6,7,8,9-Tetrahydro-7-(2-hydroxyäthyl)pyrido[1,2-a]indol -10-yl]-4-(1-methyl-3-indolyl)-1H-pyrrol-2,5-dion, Smp. 236-238 °C.

Das Ausgangsfurandion erhält man wie folgt:

a) 400 mg einer 60%igen Dispersion von Natriumhydrid in Mineralöl werden einer Lösung von 2,24 g Triäthylphosphonoacetat in 40 ml Dimethoxyäthan unter einer Stickstoffatmosphäre zugegeben. Dann wird die Lösung auf 0 °C abgekühlt und 1,85 g des Produkts von Beispiel 35d) in 10 ml Dimethoxyäthan werden zugegeben. Das Gemisch wird über Nacht gerührt und dann eingedampft, der Rückstand in Dichlormethan gelöst und dann mit Wasser gewaschen, getrocknet und eingedampft. Der Rückstand wird durch Chromatographie über Silicagel mit Diäthyläther/Petroläther (1:3) gereinigt. Man erhält 1,55 g eines Gemisches von Aethyl-(E) und (Z)-(6,7,8,9-Tetrahydropyrido[1,2-a]indol-7-yliden)acetat. Eine Lösung von 1,4 g dieses Produkts in Aethanol wird mit 280 mg 10% Pd/C unter einer Wasserstoffatmosphäre geschüttelt, der Katalysator dann abfiltriert und das Filtrat eingedampft. Man erhält 1.2 g Aethyl-6,7,8,9-tetrahydropyrido[1,2-a]indol-7-acetat, Smp. 66-68 °C nach Kristallisation aus Diäthyläther/Petroläther.

b) Eine Lösung von 1,2 g des Produkts von a) in 100 ml Diäthyläther wird mit 3,5 ml einer 1M Lösung von $LiAlH_4$ in Diäthyläther behandelt und nach 1 Stunde Rühren mit 50 ml wässriges Ammoniumchlorid versetzt. Das Gemisch wird mit Diäthyläther extrahiert und die organische Phase getrocknet und eingedampft. Man erhält 1,01 g 6,7,8,9-Tetrahydro-7-(2-hydroxyäthyl)pyrido[1,2-a]indol, Smp. 70-72 °C nach Kristallisation aus Diäthyläther/Petroläther.

c) Eine Lösung von 1,04 g des Produkts von b) in 30 ml Dichlormethan wird mit 6 ml Essigsäureanhydrid in 3 ml Pyridin behandelt und die Lösung unter einer Stickstoffatmosphäre gerührt, das Gemisch dann zur Trockene eingedampft und der Rückstand in Dichlormethan gelöst. Die organische Phase wird mit 2M Salzsäure und mit Wasser gewaschen, getrocknet und eingedampft. Der Rückstand wird durch

Chromatographie über Silicagel mit Diäthyläther/Petroläther (1:4) gereinigt. Man erhält 550 mg 7-(2-Acetoxyäthyl)-6,7,8,9-tetrahydropyrido[1,2-a]indol.

d) 250 µl Oxalylchlorid werden einer Lösung von 670 mg des Produkts von c) in 12 ml Dichlormethan unter einer Stickstoffatmosphäre zugegeben. Dann wird das Lösungsmittel unter vermindertem Druck entfernt und der Rückstand in Dichlormethan gelöst. 493 mg 1-Methyl-3-indolylessigsäure und 527 mg Triäthylamin werden zugesetzt und das Gemisch wird gerührt, dann das Lösungsmittel unter vermindertem Druck entfernt und der Rückstand durch Chromatographie über Silicagel mit Aethylacetat/Petroläther (1:2) gereinigt. Man erhält 350 mg 3-[7-(2-Acetoxyäthyl)-6,7,8,9-tetrahydropyrido[1,2-a]indol -10-yl]-4-(1-methyl-3-indolyl)furan-2,5-dion, Smp. 182-184 °C nach Kristallisation aus Aethylacetat.

Beispiel 70

Analog Beispiel 2 erhält man aus dem Produkt von Beispiel 69 3-[7-(2-Aminoäthyl)-6,7,8,9-tetrahydropyrido[1,2-a]indol-10-yl]-4-(1-methyl-3-indolyl)-1H-pyrrol -2,5-dion-hydrochlorid, Smp. 240-242 °C.

Beispiel 71

Analog Beispiel 1, 1. Absatz, erhält man aus 3-[8-Acetoxymethyl-6,7,8,9-tetrahydro -2-methoxypyrido-[1,2-a]indol-10-yl]-4-(1-methyl -3-indolyl)furan-2,5-dion 3-[6,7,8,9-Tetrahydro-8-hydroxymethyl -2-methoxypyrido[1,2-a]indol-10-yl]-4-(1-methyl-3-indolyl) -1H-pyrrol-2,5-dion, Smp. 195-197 °C.

Das Ausgangsfurandion erhält man wie folgt:

a) 2 g einer 60%igen Natriumhydrid-Dispersion in Mineralöl werden durch Dekantierung mit n-Hexan gewaschen und in 100 ml DMF unter einer Stickstoffatmosphäre suspendiert. Eine Lösung von 10 g Aethyl-5-methoxyindol-2-carboxylat in 100 ml DMF wird zugesetzt und das Gemisch gerührt. Dann werden 9,8 g Aethyl-4-brombutyrat zugegeben und das Gemisch wird 2 Stunden gerührt, dann abgekühlt und mit 50 ml 1M Salzsäure und 400 ml Wasser behandelt. Das Gemisch wird mit Diäthyläther extrahiert und die vereinigten Extrakte werden mit Natriumchloridlösung gewaschen. Die organische Phase wird getrocknet und eingedampft. Eine Lösung des erhaltenen Oels in THF wird einem Gemisch von 5,2 g Kalium-t-butoxid in 200 ml THF unter einer Stickstoffatmosphäre zugegeben. Dann wird das Gemisch abgekühlt und mit 1M Salzsäure neutralisiert. Wasser wird zugegeben und das Gemisch mit Diäthyläther extrahiert. Die vereinigten Extrakte werden mit Wasser und Natriumchloridlösung gewaschen und dann getrocknet. Abdampfen des Lösungsmittels und Kristallisation des Rückstandes aus Aethylacetat ergeben 6,7 g Aethyl-6,7-dihydro-9-hydroxy-2-methoxypyrido[1,2-a]indol -8-carboxylat, Smp. 157-160 °C.

b) 5 g des Produkts von a) in 200 ml Aethanol werden unter einer Stickstoffatmosphäre mit 10 Löffelspitzen Raney-Nickel und 100 ml Wasser behandelt. Die Suspension wird unter Rückfluss erhitzt, dann abgekühlt und filtriert. Der Feststoff wird mit Aethylacetat gewaschen und die flüchtigen Bestandteile werden im Vakuum von den vereinigten Filtraten entfernt. Die wässrige Suspension wird mit Aethylacetat extrahiert, die vereinigten Extrakte werden mit Natriumchloridlösung gewaschen und getrocknet. Abdampfen des Lösungsmittels und Kristallisation des Rückstandes aus Methanol ergeben 2,41 g Aethyl-6,7,8,9-tetrahydro-2-methoxypyrido[1,2-a]indol -8-carboxylat, Smp. 104-105 °C.

c) Eine Lösung von 2,3 g des Produkts von b) in 25 ml THF wird einer Suspension von 260 mg $LiAlH_4$ in 20 ml THF unter einer Stickstoffatmosphäre zugegeben. Dann wird das Gemisch mit 10 ml Aethylacetat gefolgt von 20 ml Wasser behandelt, mit 1M Salzsäure auf pH 3 angesäuert und mit Diäthyläther extrahiert. Die vereinigten Extrakte werden mit Wasser gewaschen und getrocknet. Abdampfen des Lösungsmittels ergibt 1,85 g 6,7,8,9-Tetrahydro-2-methoxypyrido[1,2-a]indol-8-methanol, Smp. 95-96 °C nach Kristallisation aus Aethylacetat/n-Hexan.

d) 1 g des Produkts von c) in 10 ml Pyridin wird mit 1,5 g Essigsäureanhydrid behandelt. Dann wird das Lösungsmittel abgedampft, der Rückstand zwischen Diäthyläther und 5%igem wässrigem Ammoniumchlorid verteilt, die organische Phase mit Natriumchloridlösung gewaschen, getrocknet und eingedampft. Kristallisation des Rückstandes aus Diäthyläther/n-Hexan ergibt 0,84 g 8-Acetoxymethyl-6,7,8,9-tetrahydro -2-methoxypyrido[1,2-a]indol, Smp. 98-100 °C.

e) Eine Suspension von 800 mg des Produkts von d) in 25 ml Diäthyläther wird mit 0,27 ml Oxalylchlorid unter einer Stickstoffatmosphäre behandelt, dann das Lösungsmittel abgedampft, der Rückstand in 20 ml Dichlormethan gelöst und mit 555 mg N-Methylindol-3-essigsäure und 0,8 ml Triäthylamin behandelt. Das Gemisch wird 65 Stunden gerührt und dann das Lösungsmittel unter vermindertem Druck abgedampft. Chromatographie des Rückstandes über Silicagel mit Aethylacetat/n-Hexan (1:1) ergibt 380 mg 3-[8-Acetoxymethyl-6,7,8,9-tetrahydro -2-methoxypyrido[1,2-a]indol-10-yl]-4-(1-methyl -3-indolyl)furan-2,5-dion, Smp. 131-133 °C (Zersetzung) nach Kristallisation aus Toluol/n-Hexan.

Beispiel 72

Analog Beispiel 2 erhält man aus dem Produkt von Beispiel 71 3-[8-Aminomethyl-6,7,8,9-tetrahydro -2-methoxypyrido[1,2-a]indol-10-yl]-4-(1-methyl -3-indolyl)-1H-pyrrol-2,5-dion-hydrochlorid, Smp. 235-238 °C (Zersetzung).

Beispiel 73

a) Eine Lösung von 150 mg des Produkts von Beispiel 20 in Dichlormethan wird unter einer Stickstoffatmosphäre mit 135 mg 1,1'-Thiocarbonyldiimidazol behandelt und nach 17 Stunden mit Wasser gewaschen und getrocknet. Das Lösungsmittel wird abgedampft und der Rückstand aus Aethylacetat kristallisiert. Man erhält 150 mg 3-[1,2,3,4-Tetrahydro -2-(1-imidazolylthiocarbonyl)pyrazino[1,2-a]indol-10-yl] -4-(1-methyl-3-indolyl)-1H-pyrrol-2,5-dion, Smp. 244-247 °C.

b) Eine Lösung von 140 mg des Produkts von a) in 10 ml DMF wird mit 20 ml 33%igem wässrigem Ammoniak behandelt. Nach 17 Stunden wird die Suspension filtriert und der Feststoff mit Wasser gewaschen und dann getrocknet. Man erhält 95 mg 3-[1,2,3,4-Tetrahydro-2-thiocarbamoylpyrazino[1,2-a]indol-10-yl]-4-(1-methyl-3-indolyl)-1H-pyrrol-2,5-dion, Smp. 278 °C (Zerstzung).

Beispiel 74

Eine Lösung von 150 mg des Produkts von Beispiel 20 in 50 ml Dichlormethan wird mit 3 ml Essigsäureanhydrid und 3 ml Triäthylamin behandelt und nach 17 Stunden mit Wasser gewaschen. Die organische Phase wird getrocknet und eingedampft, der Rückstand in Dichlormethan gelöst und mit 0,08 ml Diäthylamin behandelt. Nach 17 Stunden wird die Lösung eingedampft. Kristallisation des Rückstandes aus Dichlormethan/n-Hexan ergibt 80 mg 3-[2-Acetyl-1,2,3,4-tetrahydropyrazino[1,2-a]indol-10-yl] -4-(1-methyl-3-indolyl)-1H-pyrrol-2,5-dion, Smp. 308-310 °C.

Beispiel 75

Analog Beispiel 74 erhält man aus dem Produkt von Beispiel 2 3-[8-Acetamidomethyl-6,7,8,9-tetrahydropyrido[1,2-a]indol -10-yl]-4-(1-methyl-3-indolyl)-1H-pyrrol-2,5-dion, Smp. 270-273 °C.

Beispiel 76

Eine Lösung von 150 mg des Produkts von Beispiel 20 in 40 ml Dichlormethan wird mit 40 mg Triäthylamin und 44 mg Methansulfonylchlorid behandelt. Nach 17 Stunden wird das Gemisch mit Wasser gewaschen, die organische Phase getrocknet und eingedampft. Chromatographie des Rückstandes über Silicagel mit Aethylacetat/n-Hexan (2:1) und Aethylacetat ergibt 95 mg 3-[1,2,3,4-Tetrahydro-2-methansulfonylpyrazino[1,2-a]indol -10-yl]-4-(1-methyl-3-indolyl)-1H-pyrrol-2,5-dion, Smp. 298-301 °C (Zersetzung).

Beispiel 77

Eine Lösung von 3,0 g des Produkts von Beispiel 1 in 100 ml THF werden einer Suspension von 1,8 g Lithiumaluminiumhydrid in 50 ml THF bei 0 °C zugegeben. Das Gemisch wird 16 Stunden auf Rückfluss erhitzt, dann abgekühlt, mit 10 ml Wasser behandelt und mit Dichlormethan extrahiert. Die vereinigten Dichlormethanextrakte werden mit wässrigem Natriumbicarbonat gewaschen, getrocknet und eingedampft. Chromatographie des erhaltenen Feststoffs über Silicagel mit Dichlormethan/Methanol (95:5) ergibt

a) 400 mg 1,5-Dihydro-3-[6,7,8,9-tetrahydro -8-hydroxymethylpyrido[1,2-a]indol-10-yl]-4-(1-methyl -3-indolyl)-2H-pyrrol-2-on, Smp. 205-207 °C und

b) 160 mg 1,5-Dihydro-4-[6,7,8,9-tetrahydro -8-hydroxymethylpyrido[1,2-a]indol-10-yl]-3-(1-methyl -3-indolyl)-2H-pyrrol-2-on, Smp. 201-203 °C.

Beispiel 78

Analog Beispiel 1, 1. Absatz, erhält man aus 0,5 g 3-[8-Acetoxymethyl-6,7,8,9-tetrahydropyrido[1,2-a]-indol -10-yl]-4-(3-trifluormethylphenyl)furan-2,5-dion 110 mg 3-[6,7,8,9-Tetrahydro-8-hydroxymethylpyrido-[1,2-a]indol -10-yl]-4-(3-trifluormethylphenyl)-1H-pyrrol-2,5-dion, Smp. 77-79 °C.

Das Ausgangsfurandion erhält man wie folgt:

1,7 g Oxalylchlorid werden einer auf 0-4°C abgekühlten Lösung von 3,0 g 8-Acetoxymethyl-6,7,8,9-tetrahydropyrido[1,2-a]indol in 50 ml Dichlormethan zugetropft. Nach 2 Stunden wird das Lösungsmittel abgedampft und der Rückstand in Dichlormethan gelöst. Die Lösung wird einer Lösung von 2,7 g (α,α,α-Trifluor-m-tolyl)essigsäure und 3,2 g Triäthylamin in 70 ml Dichlormethan zugegeben. Das Gemisch wird 16 Stunden gerührt und dann eingedampft. Der Rückstand wird über Silicagel mit Dichlormethan/Methanol (95:5) chromatographiert. Man erhält 700 mg 3-[8-Acetoxymethyl-6,7,8,9-tetrahydropyrido[1,2-a]indol -10-yl]-4-(3-trifluormethylphenyl)furan-2,5-dion, Smp. 176-177°C.

Beispiel 79

Analog Beispiel 1, 1. Absatz, erhält man aus 1,0 g 3-[8-Acetoxymethyl-6,7,8,9-tetrahydropyrido[1,2-a]-indol -10-yl]-4-(4-methoxyphenyl)furan-2,5-dion 150 mg 3-[6,7,8,9-Tetrahydro-8-hydroxymethylpyrido[1,2-a]-indol -10-yl]-4-(4-methoxyphenyl)-1H-pyrrol-2,5-dion, Smp. 228°C (Zersetzung).

Das Ausgangsfurandion erhält man wie folgt:

1,7 g Oxalylchlorid werden einer auf 0-4°C abgekühlten Lösung von 3,0 g 8-Acetoxymethyl-6,7,8,9-tetrahydropyrido[1,2-a]indol in 50 ml Dichlormethan zugetropft. Nach 2 Stunden wird das Lösungsmittel abgedampft und der Rückstand in Dichlormethan gelöst. Die Lösung wird einer Lösung von 2,24 g p-Methoxyphenylessigsäure und 3,2 g Triäthylamin in 70 ml Dichlormethan zugesetzt, das Gemisch 16 Stunden gerührt und dann eingedampft. Chromatographie des Rückstandes über Silicagel mit Dichlormethan/Methanol (95:5) ergibt 2 g 3-[8-Acetoxymethyl-6,7,8,9-tetrahydropyrido[1,2-a]indol -10-yl]-4-(4-methoxyphenyl)furan-2,5-dion, Smp. 79-82°C.

Beispiel 80

Analog Beispiel 1, 1. Absatz, erhält man aus 0,8 g 3-[8-Acetoxymethyl-6,7,8,9-tetrahydropyrido[1,2-a]-indol -10-yl]-4-(2-chlorphenyl)furan-2,5-dion 120 mg 3-(2-Chlorphenyl)-4-[6,7,8,9-tetrahydro -8-hydroxymethylpyrido[1,2-a]indol-10-yl] -1H-pyrrol-2,5-dion, Smp. 232-233°C.

Das Ausgangsfurandion erhält man wie folgt:

2,2 g Oxalylchlorid werden einer eiskalten Lösung von 4 g 8-Acetoxymethyl-6,7,8,9-tetrahydropyrido-[1,2-a]indol in 50 ml Dichlormethan zugetropft. Nach 2 Stunden wird das Lösungsmittel abgedampft und der Rückstand in Dichlormethan gelöst. Diese Lösung wird einer Lösung von 3,0 g 2-Chlorphenylessigsäure und 4,0 g Triäthylamin in Dichlormethan zugegeben. Das Gemisch wird 16 Stunden gerührt und dann eingedampft. Chromatographie des Rückstandes über Silicagel mit Dichlormethan/Methanol (95:5) ergibt 0,9 g 3-[8-Acetoxymethyl-6,7,8,9-tetrahydropyrido[1,2-a]indol -10-yl]-4-(2-chlorphenyl)furan-2,5-dion, Smp. 168-171°C.

Beispiel 81

Analog Beispiel 51 erhält man aus 80 mg des Produkts von Beispiel 78 30 mg 3-[8-Aminomethyl-6,7,8,9-tetrahydropyrido[1,2-a]indol -10-yl]-4-(3-trifluormethylphenyl)-1H-pyrrol-2,5-dion, Smp. 202-204°C.

Beispiel 82

Analog Beispiel 51 erhält man aus 100 mg des Produkts von Beispiel 79 88 mg 3-[8-Aminomethyl-6,7,8,9-tetrahydropyrido[1,2-a]indol -10-yl]-4-(4-methoxyphenyl)-1H-pyrrol-2,5-dion, Smp. 195-196°C.

Beispiel 83

Analog Beispiel 53 erhält man aus 80 mg des Produkts von Beispiel 80 57 mg 3-[8-Aminomethyl-6,7,8,9-tetrahydropyrido[1,2-a]indol -10-yl]-4-(2-chlorphenyl)-1H-pyrrol-2,5-dion, Smp. 206-208°C (Zersetzung).

Die folgenden galenischen Präparate werden in an sich bekannter Weise hergestellt:

| Beispiel A | |
|---|---|
| Bestandteile | Pro Tablette |
| Verbindung der Formel I | 5,0 mg |
| Lactose | 125,0 mg |
| Maisstärke | 75,0 mg |
| Talk | 4,0 mg |
| Magnesiumstearat | 1,0 mg |
| | Tablettengewicht 210,0 mg |

| Beispiel B | |
|---|---|
| Bestandteile | Pro Kapsel |
| Verbindung der Formel I | 10,0 mg |
| Lactose | 165,0 mg |
| Maisstärke | 20,0 mg |
| Talk | 5,0 mg |
| | Kapselfüllgewicht 200,0 mg |

**Patentansprüche**

**Patentansprüche für folgende Vertragsstaaten : AT, BE, CH, DE, DK, FR, GB, IT, LI, LU, NL, SE**

**1.** Verbindungen der allgemeinen Formel

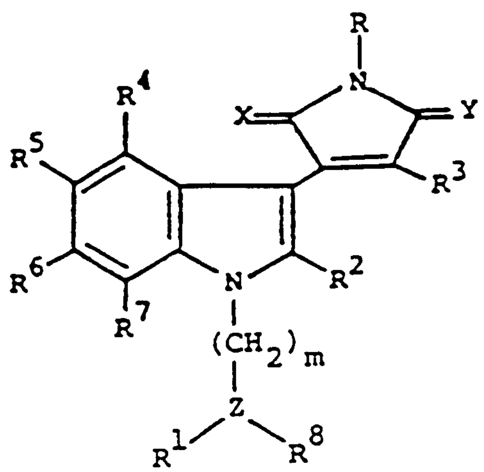

I

worin R Wasserstoff oder Hydroxy, $R^1$ und $R^2$ zusammen eine Gruppe der Formel $-(CH_2)_n-$ und $R^7$ Wasserstoff, oder $R^1$ und $R^7$ zusammen eine Gruppe der Formel $-(CH)_n-$ und $R^2$ Wasserstoff; $R^3$ Aryl oder Heteroaryl; $R^4$, $R^5$ und $R^6$ Wasserstoff, Halogen, Alkyl, Hydroxy, Alkoxy, Haloalkyl, Nitro, Amino, Acylamino, Alkylthio, Alkylsulfinyl oder Alkylsulfonyl; $R^8$ eine Gruppe der Formel $-(CH_2)_p-R^9$ oder $-(CH_2)_q-R^{10}$; $R^9$ Wasserstoff, Alkylcarbonyl, Aminoalkylcarbonyl, Cyano, Amidino, Alkoxycarbonyl, Aryloxycarbonyl, Alkylsulfonyl, Aminocarbonyl oder Aminothiocarbonyl; $R^{10}$ Hydroxy, Alkoxy, Halogen, Amino, Monoalkylamino, Dialkylamino, Trialkylamino, Azido, Acylamino, Alkylsulfonylamino, Arylsulfonylamino, Alkylthio, Alkoxycarbonylamino, Aminoacylamino, Aminocarbonylamino, Isothiocyanato, Alkylcarbonyloxy, Alkylsulfonyloxy oder Arylsulfonyloxy, eine 5- oder 6-gliedrige gesättigte stickstoffhaltige heterocyclische Gruppe, die über das N-Atom gebunden ist oder eine Gruppe der Formel -U-C(V)-W; U ein S-Atom oder NH; V die Gruppen NH, $NNO_2$, NCN oder $CHNO_2$; W Amino, Mono- oder Dialkylamino; eins von X und Y ein O-Atom und das andere O oder (H,H); Z die Gruppe CH oder ein N-Atom; m, p und q eine Zahl von 0 bis 5 sind und n eine Zahl von 1 bis 5 ist, mit der Bedingung, dass m und q

eine Zahl von 2 bis 5 sind, wenn Z ein N-Atom ist;
sowie pharmazeutisch verwendbare Salze von sauren Verbindungen der Formel I mit Basen und von basischen Verbindungen der Formel I mit Säuren.

**2.** Verbindungen nach Anspruch 1, worin R Wasserstoff, $R^9$ Wasserstoff, Alkylcarbonyl, Cyano, Amidino, Alkoxycarbonyl, Alkylsulfonyl, Aminocarbonyl oder Aminothiocarbonyl und $R^{10}$ Hydroxy, Alkoxy, Halogen, Amino, Monoalkylamino, Dialkylamino, Trialkylamino, Azido, Acylamino, Alkylsulfonylamino, Arylsulfonylamino, Alkylthio, Aminocarbonylamino, Isothiocyanato, Alkylcarbonyloxy, Alkylsulfonyloxy oder Arylsulfonyloxy oder eine Gruppe der Formel -U-C(V)-W und $R^1$, $R^2$, $R^3$, $R^4$, $R^5$, $R^6$, $R^7$, $R^8$, U, V, W, X, Y, Z, m, n, p und q die in Anspruch 1 angegebene Bedeutung haben.

**3.** Verbindungen nach Anspruch 1 oder 2, worin $R^1$ und $R^2$ zusammen $-CH_2-$ und $R^7$ Wasserstoff, m die Zahl 1 oder 2 und Z die Gruppe CH sind.

**4.** Verbindungen nach Anspruch 1 oder 2, worin $R^1$ und $R^2$ zusammen $-(CH_2)_2-$ und $R^7$ Wasserstoff, m die Zahl 1 und Z die Gruppe CH sind.

**5.** Verbindungen nach Anspruch 1 oder 2, worin $R^1$ und $R^2$ zusammen $-CH_2-$ und $R^7$ Wasserstoff, m die Zahl 2 und Z ein N-Atom sind.

**6.** Verbindungen nach Anspruch 1 oder 2, worin $R^1$ und $R^7$ zusammen $-CH_2-$ und $R^2$ Wasserstoff, m die Zahl 1 und Z die Gruppe CH sind.

**7.** Verbindungen nach Anspruch 1 oder 2, worin $R^1$ und $R^7$ zusammen $-(CH_2)_2-$ und $R^2$ Wasserstoff, m O und Z die Gruppe CH sind.

**8.** Verbindungen nach einem der Ansprüche 1-6, worin $R^3$ Phenyl, Naphthyl, 3-Benzothienyl, 3-Benzofuranyl oder 3-Indolyl ist, das gewünschtenfalls durch einen oder mehreren Substituenten aus der Gruppe von Halogen, Alkyl, Hydroxy, Alkoxy, Haloalkyl, Nitro, Amino, Acylamino, Alkylthio, Alkylsulfinyl und Alkylsulfonyl substituiert ist.

**9.** Verbindungen nach Anspruch 8, worin $R^3$ 1-Methyl-3-indolyl ist.

**10.** Verbindungen nach einem der Ansprüche 1-9, worin $R^4$, $R^5$ und $R^6$ Wasserstoff sind.

**11.** Verbindungen nach einem der Ansprüche 1-10, worin $R^8$ eine Gruppe der Formel $-(CH_2)_q-R^{10}$ ist.

**12.** Verbindungen nach Anspruch 11, worin q die Zahl 1 oder 2 ist.

**13.** Verbindungen nach Anspruch 11 oder 12, worin $R^{10}$ Hydroxy, Amino, Monoalkylamino, Dialkylamino, Trialkylamino, Azido, Acylamino, Alkylcarbonyloxy oder Alkylsulfonyloxy oder eine Gruppe der Formel -U-C(V)-W.

**14.** Verbindungen nach Anspruch 13, worin U ein S-Atom, V die Gruppe NH und W Amino ist.

**15.** Verbindungen nach einem der Ansprüche 1-14, worin X und Y Sauerstoff sind.

**16.** Verbindungen nach Anspruch 1 oder 2 aus der Gruppe der folgenden:
3-[8-(Aminomethyl)-6,7,8,9-tetrahydropyrido[1,2-a]indol-10-yl]-4-(1-methyl-3-indolyl)-1H-pyrrol-2,5-dion,
3-[7-(Amidinothiomethyl)-6,7,8,9-tetrahydropyrido[1,2-a]indol-10-yl]-4-(1-methyl-3-indolyl)-1H-pyrrol-2,5-dion,
3-[6,7,8,9-Tetrahydro-8-[(dimethylamino)methyl]pyrido[1,2-a]indol-10-yl]-4-(1-methyl-3-indolyl)-1H-pyrrol-2,5-dion
und pharmazeutisch verwendbare Salze davon.

**17.** Verbindungen der allgemeinen Formel

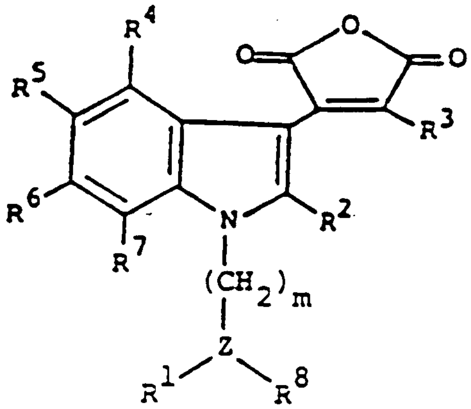

II

wherein $R^1$, $R^2$, $R^3$, $R^4$, $R^5$, $R^6$, $R^7$, $R^8$, Z und m die in Anspruch 1 angegebene Bedeutung haben.

**18.** Verbindungen nach einem der Ansprüche 1-16 zur Verwendung als therapeutisch aktive Substanzen, insbesondere zur Verwendung als antiinflammatorisch, immunologisch, onkologisch, bronchopulmonär und kardiovaskulär aktive Substanzen oder als Wirkstoffe in der Behandlung von Asthma oder AIDS.

**19.** Verfahren zur Herstellung der Verbindungen nach einem der Ansprüche 1-16, dadurch gekennzeichnet, dass man

a) zur Herstellung einer Verbindung der Formel I, in der X und Y Sauerstoff sind, eine Verbindung der allgemeinen Formel

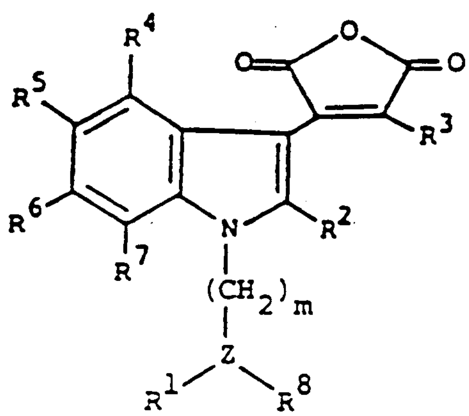

II

worin $R^1$, $R^2$, $R^3$, $R^4$, $R^5$, $R^6$, $R^7$, $R^8$, Z und m die in Anspruch 1 angegebene Bedeutung haben, mit Ammoniak unter Druck oder mit Hexamethyldisilazan und Methanol zu einer Verbindung der Formel I, in der R Wasserstoff ist, oder mit Hydroxylamin zu einer Verbindung der Formel, in der R Hydroxy ist, umsetzt, oder

b) zur Herstellung einer Verbindung der Formel I, in der eins von X und Y Sauerstoff und das andere (H,H) ist, eine Verbindung der Formel I, in der X und X Sauerstoff sind, mit Lithiumaluminiumhydrid reduziert, und

c) gewünschtenfalls eine in einer erhaltenen Verbindung der Formel I enthaltene reaktionsfähige Gruppe funktionell abwandelt, und

d) gewünschtenfalls eine saure Verbindung der Formel I in ein pharmazeutisch verwendbares Salz mit einer Base oder eine basische Verbindung der Formel I in ein pharmazeutisch verwendbares Salz mit einer Säure umwandelt.

**20.** Pharmazeutische Präparate, insbesondere antiinflammatorische, immunologische, onkologische, bronchopulmonäre oder kardiovaskuläre Präparate oder Präparate zur Behandlung von Asthma oder AIDS enthaltend eine Verbindung nach einem der Ansprüche 1-16 und ein inertes Trägermaterial.

**21.** Verwendung einer Verbindung nach einem der Ansprüche 1-16 für die Herstellung eines pharmazeutischen Präparates gegen inflammatorische, immunologische, onkologische, bronchopulmonäre oder kardiovaskuläre Krankheiten oder gegen Asthma oder AIDS.

**Patentansprüche für folgende Vertragsstaaten : ES, GR**

**1.** Verfahren zur Herstellung der Verbindungen der allgemeinen Formel

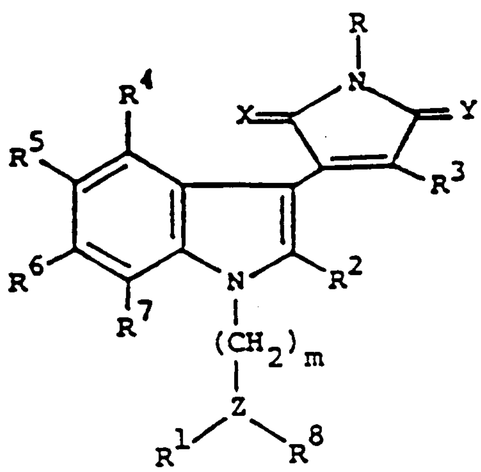

I

worin R Wasserstoff oder Hydroxy, $R^1$ und $R^2$ zusammen eine Gruppe der Formel $-(CH_2)_n-$ und $R^7$ Wasserstoff, oder $R^1$ und $R^7$ zusammen eine Gruppe der Formel $-(CH_2)_n-$ und $R^2$ Wasserstoff; $R^3$ Aryl oder Heteroaryl; $R^4$, $R^5$ und $R^6$ Wasserstoff, Halogen, Alkyl, Hydroxy, Alkoxy, Haloalkyl, Nitro, Amino, Acylamino, Alkylthio, Alkylsulfinyl oder Alkylsulfonyl; $R^8$ eine Gruppe der Formel $-(CH_2)_p-R^9$ oder $-(CH_2)_q-R^{10}$; $R^9$ Wasserstoff, Alkylcarbonyl, Aminoalkylcarbonyl, Cyano, Amidino, Alkoxycarbonyl, Aryloxycarbonyl, Alkylsulfonyl, Aminocarbonyl der Aminothiocarbonyl; $R^{10}$ Hydroxy, Alkoxy, Halogen, Amino, Monoalkylamino, Dialkylamino, Trialkylamino, Azido, Acylamino, Alkylsulfonylamino, Arylsulfonylamino, Alkylthio, Alkoxycarbonylamino, Aminoacylamino, Aminocarbonylamino, Isothiocyanato, Alkylcarbonyloxy, Alkylsulfonyloxy oder Arylsulfonyloxy, eine 5- oder 6-gliedrige gesättigte stickstoffhaltige heterocyclische Gruppe, die über das N-Atom gebunden ist oder eine Gruppe der Formel -U-C(V)-W; U ein S-Atom oder NH; V die Gruppen NH, $NNO_2$, NCN oder $CHNO_2$; W Amino, Mono- oder Dialkylamino; eins von X und Y ein O-Atom und das andere O oder (H,H); Z die Gruppe CH oder ein N-Atom; m, p und q eine Zahl von 0 bis 5 sind und n eine Zahl von 1 bis 5 ist, mit der Bedingung, dass m und q eine Zahl von 2 bis 5 sind, wenn Z ein N-Atom ist;
sowie von pharmazeutisch verwendbaren Salzen von sauren Verbindungen der Formel I mit Basen und von basischen Verbindungen der Formel I mit Säuren, dadurch gekennzeichnet, dass man

a) zur Herstellung einer Verbindung der Formel I, in der X und Y Sauerstoff sind, eine Verbindung der allgemeinen Formel

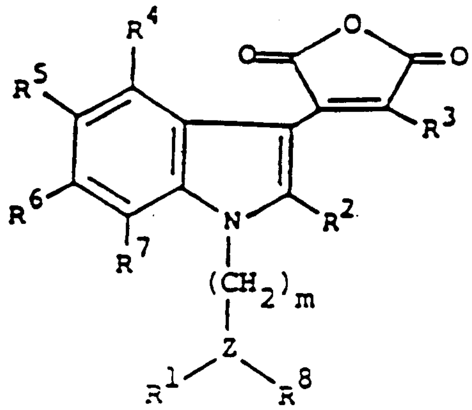

II

worin $R^1$, $R^2$, $R^3$, $R^4$, $R^5$, $R^6$, $R^7$, $R^8$, Z und m die in Anspruch 1 angegebene Bedeutung haben, mit Ammoniak unter Druck oder mit Hexamethyldisilazan und Methanol zu einer Verbindung der Formel I, in der R Wasserstoff ist, oder mit Hydroxylamin zu einer Verbindung der Formel, in der R Hydroxy ist, umsetzt, oder

b) zur Herstellung einer Verbindung der Formel I, in der eins von X und Y Sauerstoff und das andere (H,H) ist, eine Verbindung der Formel I, in der X und X Sauerstoff sind, mit Lithiumaluminiumhydrid reduziert, und

c) gewünschtenfalls eine in einer erhaltenen Verbindung der Formel I enthaltene reaktionsfähige Gruppe funktionell abwandelt, und

d) gewünschtenfalls eine saure Verbindung der Formel I in ein pharmazeutisch verwendbares Salz mit einer Base oder eine basische Verbindung der Formel I in ein pharmazeutisch verwendbares Salz mit einer Säure umwandelt.

**2.** Verfahren nach Anspruch 1, worin R Wasserstoff, $R^9$ Wasserstoff, Alkylcarbonyl, Cyano, Amidino, Alkoxycarbonyl, Alkylsulfonyl, Aminocarbonyl oder Aminothiocarbonyl und $R^{10}$ Hydroxy, Alkoxy, Halogen, Amino, Monoalkylamino, Dialkylamino, Trialkylamino, Azido, Acylamino, Alkylsulfonylamino, Arylsulfonylamino, Alkylthio, Aminocarbonylamino, Isothiocyanato, Alkylcarbonyloxy, Alkylsulfonyloxy oder Arylsulfonyloxy oder eine Gruppe der Formel -U-C(V)-W und $R^1$, $R^2$, $R^3$, $R^4$, $R^5$, $R^6$, $R^7$, $R^8$, U, V, W, X, Y, Z, m, n, p und q die in Anspruch 1 angegebene Bedeutung haben.

**3.** Verfahren nach Anspruch 1 oder 2, worin $R^1$ und $R^2$ zusammen $-CH_2-$ und $R^7$ Wasserstoff, m die Zahl 1 oder 2 und Z die Gruppe CH sind.

**4.** Verfahren nach Anspruch 1 oder 2, worin $R^1$ und $R^2$ zusammen $-(CH_2)_2-$ und $R^7$ Wasserstoff, m die Zahl 1 und Z die Gruppe CH sind.

**5.** Verfahren nach Anspruch 1 oder 2, worin $R^1$ und $R^2$ zusammen $-CH_2-$ und $R^7$ Wasserstoff, m die Zahl 2 und Z ein N-Atom sind.

**6.** Verfahren nach Anspruch 1 oder 2, worin $R^1$ und $R^7$ zusammen $-CH_2-$ und $R^2$ Wasserstoff, m die Zahl 1 und Z die Gruppe CH sind.

**7.** Verfahren nach Anspruch 1 oder 2, worin $R^1$ und $R^7$ zusammen $-(CH_2)_2-$ und $R^2$ Wasserstoff, m O und Z die Gruppe CH sind.

**8.** Verfahren nach einem der Ansprüche 1-6, worin $R^3$ Phenyl, Naphthyl, 3-Benzothienyl, 3-Benzofuranyl oder 3-Indolyl ist, das gewünschtenfalls durch einen oder mehreren Substituenten aus der Gruppe von Halogen, Alkyl, Hydroxy, Alkoxy, Haloalkyl, Nitro, Amino, Acylamino, Alkylthio, Alkylsulfinyl und Alkylsulfonyl substituiert ist.

**9.** Verfahren nach Anspruch 8, worin $R^3$ 1-Methyl-3-indolyl ist.

**10.** Verfahren nach einem der Ansprüche 1-9, worin $R^4$, $R^5$ und $R^6$ Wasserstoff sind.

**11.** Verfahren nach einem der Ansprüche 1-10, worin $R^8$ eine Gruppe der Formel $-(CH_2)_q-R^{10}$ ist.

**12.** Verfahren nach Anspruch 11, worin q die Zahl 1 oder 2 ist.

**13.** Verfahren nach Anspruch 11 oder 12, worin $R^{10}$ Hydroxy, Amino, Monoalkylamino, Dialkylamino, Trialkylamino, Azido, Acylamino, Alkylcarbonyloxy oder Alkylsulfonyloxy oder eine Gruppe der Formel -U-C(V)-W.

**14.** Verfahren nach Anspruch 13, worin U ein S-Atom, V die Gruppe NH und W Amino ist.

**15.** Verfahren nach einem der Ansprüche 1-14, worin X und Y Sauerstoff sind.

**16.** Verfahren nach Anspruch 1 oder 2, worin man eine Verbindung aus der Gruppe der folgenden herstellt:
3-[8-(Aminomethyl)-6,7,8,9-tetrahydropyrido[1,2-a]indol-10-yl]-4-(1-methyl-3-indolyl)-1H-pyrrol-2,5-dion,
3-[7-(Amidinothiomethyl)-6,7,8,9-tetrahydropyrido[1,2-a]indol-10-yl]-4-(1-methyl-3-indolyl)-1H-pyrrol-2,5-dion,
3-[6,7,8,9-Tetrahydro-8-[(dimethylamino)methyl]pyrido[1,2-a]indol-10-yl]-4-(1-methyl-3-indolyl)-1H-pyrrol-2,5-dion
und pharmazeutisch verwendbare Salze davon.

**17.** Verfahren nach Anspruch 1, worin man
a) die Verbindung der Formel II mit Ammoniak bei 100-150 °C, mit Hexamethyldisilazan und Methanol bei 40-110 °C oder mit Hydroxylamin bei 100 °C jeweils in einem inerten organischen Lösungsmittel umsetzt.
b) die Reduktion mit $LiAlH_4$ in einem inerten organischen Lösungsmittel bei einer Temperatur zwischen 0 °C und Rückflusstemperatur durchführt.
c) gewünschtenfalls eine Alkoxycarbonylgruppe $R^8$ mit einer Säure in ein H-Atom umwandelt; eine Gruppe Alkyl-COO-$(CH_2)_q$- mit einer Base in eine Gruppe HO-$(CH_2)_q$- umwandelt; letztere Gruppe durch Behandlung mit Trifluormethansulfonsäureanhydrid und dann mit Ammoniak, einem Mono-, Di- oder Trialkylamin bzw. einem geeigneten Heterocyclus in eine Gruppe $-(CH_2)_q-R^{10}$ umwandelt, in der $R^{10}$ gegebenenfalls mono-, di- oder trialkyliertes Amino oder ein 5- oder 6-gliedriger Heterocyclus ist; eine Gruppe $-(CH_2)_q$-OH mit einem Alkansulfonsäureanhydrid umsetzt; eine erhaltene Gruppe Alkyl-$SO_3$-$(CH_2)_q$- mit Ammoniak in Dimethylformamid, mit einem Alkalimetallazid bzw. mit Thioharnstoff in eine Gruppe $-(CH_2)_q-R^{10}$ umwandelt, in der $R^{10}$ Formamido, Azido bzw. $-SNHNH_2$ ist; eine Gruppe $-(CH_2)_q-N_3$ zu einer Gruppe $-(CH_2)_q-NH_2$ katalytisch hydriert; eine Gruppe Alkyl-OCONH-$(CH_2)_q$- mit einer Säure in eine Gruppe $-(CH_2)_q-NH_2$ umwandelt; letztere Gruppe N-acyliert oder mittels 3,5-Dimethyl-$N^2$-nitro-1-pyrrazolyl-1-carboxamid bzw. mittels 1,1-Thiocarbonyldiimidazol in eine Gruppe $-(CH_2)_q-NHC(NH)NNO_2$ bzw. $-(CH_2)_q$-NCS überführt; eine Gruppe $-(CH_2)_p$-CN mit Chlorwasserstoff und dann Ammoniak in eine Gruppe $-(CH_2)_p-C(NH)NH_2$ überführt; eine Verbindung, in der Z ein N-Atom und $R^8$ Wasserstoff ist, zu einer Verbindung, in der $R^8$ Alkyl-CO-, Alkyl-OCO oder Aryl-OCO- ist, acyliert oder mittels eines Alkansulfonylchlorids, mittels eines Trifluoracetamidoalkanoylchlorids und Ammoniak, mittels 1,1-Carbonyldiimidazol und Ammoniak bzw. mittels 1,1-Thiocarbonyldiimidazol und Ammoniak in eine Verbindung überführt, in der $R^8$ Alkyl-$SO_2$-,-CO-Alkyl-$NH_2$, $-CONH_2$ bzw. $-CSNH_2$ ist.

**18.** Verfahren zur Herstellung von pharmazeutischen Präparaten, insbesondere antiinflammatorischen, immunologischen, onkologischen, bronchopulmonären oder kardiovaskulären Präparaten oder Präparaten zur Behandlung von Asthma oder AIDS, dadurch gekennzeichnet, dass man ein Produkt nach Anspruch 1 und ein inertes Trägermaterial in eine galenische Darreichungaform bringt.

**19.** Verwendung eines Produkts nach Anspruch 1 für die Herstellung eines pharmazeutischen Präparates gegen inflammatorische, immunologische, onkologische, bronchopulmonäre oder kardiovaskuläre Krankheiten oder gegen Asthma oder AIDS.

**20.** Verbindungen der allgemeinen Formel

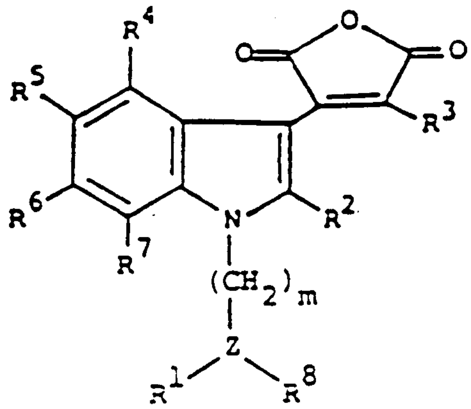

II

wherein $R^1$, $R^2$, $R^3$, $R^4$, $R^5$, $R^6$, $R^7$, $R^8$,
Z und m die in Anspruch 1 angegebene Bedeutung haben.

## Claims

### Claims for the following Contracting States : AT, BE, CH, DE, DK, FR, GB, IT, LI, LU, NL, SE

**1.** Compounds of the general formula

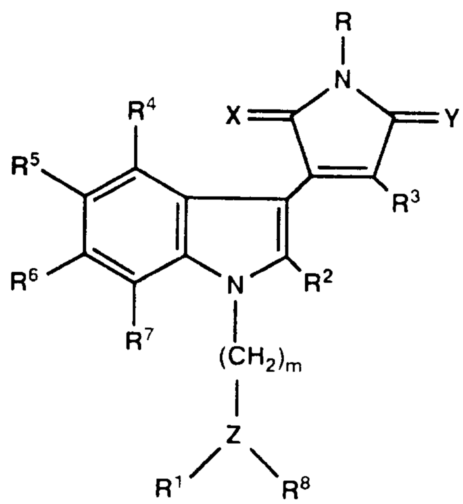

I

wherein R is hydrogen or hydroxy, $R^1$ and $R^2$ together are a group of the formula $-(CH_2)_n-$ and $R^7$ is hydrogen or $R^1$ and $R^7$ together are a group of the formula $-(CH_2)_n-$ and $R^2$ is hydrogen; $R^3$ is aryl or heteroaryl; $R^4$, $R^5$ and $R^6$ are hydrogen, halogen, alkyl, hydroxy, alkoxy, haloalkyl, nitro, amino, acylamino, alkylthio, alkylsulphinyl or alkylsulphony; $R^8$ is a group of the formula $-(CH_2)_p-R^9$ or $-(CH_2)_q-R^{10}$;$R^9$ is hydrogen, alkylcarbonyl, aminoalkylcarbonyl, cyano, amidino, alkoxycarbonyl, aryloxycarbonyl, alkylsulphonyl, aminocarbonyl or aminothiocarbonyl; $R^{10}$ is hydroxy, alkoxy, halogen, amino, monoalkylamino, dialkylamino, trialkylamino, azido, acylamino, alkylsulphonylamino, arylsulphonylamino, alkylthio, alkoxycarbonylamino, aminoacylamino, aminocarbonylamino, isothiocyanato, alkylcarbonyloxy, alkylsulphonyloxy or arylsulphonyloxy, a 5- or 6-membered saturated nitrogen-containing heterocycle attached via the N atom or a group of the formula -U-C(V)-W; U is a S atom or NH; V is the group NH, $NNO_2$, NCN or $CHNO_2$; W is amino, mono- or dialkylamino; one of X and Y is an O atom and the other is O or (H,H); Z is the group CH or a N atom; m, p and q are a number from 0 to 5 and n is a number from 1 to 5, with the proviso that m and q are a number from 2 to 5 when Z is a N atom;
as well as pharmaceutically usable salts of acidic compounds of formula I with bases and of basic compounds of formula I with acids.

2. Compounds according to claim 1, wherein R is hydrogen, $R^9$ is hydrogen, alkylcarbonyl, cyano, amidino, alkoxycarbonyl, alkylsulphonyl, aminocarbonyl or aminothiocarbonyl and $R^{10}$ is hydroxy, alkoxy, halogen, amino, monoalkylamino, dialkylamino, trialkylamino, azido, acylamino, alkylsulphonylamino, arylsulphonylamino, alkylthio, aminocarbonylamino, isothiocyanato, alkylcarbonyloxy, alkylsulphonyloxy or arylsulphonyloxy or a group of the formula -U-C(V)-W and $R^1$, $R^2$, $R^3$, $R^4$, $R^5$, $R^6$, $R^7$, $R^8$, U, V, W, X, Y, Z, m, n, p and q have the significance given in claim 1.

3. Compounds according to claim 1 or 2, wherein $R^1$ and $R^2$ together are $-CH_2-$ and $R^7$ is hydrogen, m is the number 1 or 2 and Z is the group CH.

4. Compounds according to claim 1 or 2, wherein $R^1$ and $R^2$ together are $-(CH_2)_2-$ and $R^7$ is hydrogen, m is the number 1 and Z is the group CH.

5. Compounds according to claim 1 or 2, wherein $R^1$ and $R^2$ together are $-CH_2-$ and $R^7$ is hydrogen, m is the number 2 and Z is a N atom.

6. Compounds according to claim 1 or 2, wherein $R^1$ and $R^7$ together are $-CH_2-$ and $R^2$ is hydrogen, m is the number 1 and Z is the group CH.

7. Compounds according to claim 1 or 2, wherein $R^1$ and $R^7$ together are $-(CH_2)_2-$ and $R^2$ is hydrogen, m is 0 and Z is the group CH.

8. Compounds according to any one of claims 1 to 6, wherein $R^3$ is phenyl, naphthyl, 3-benzothienyl, 3-benzofuranyl or 3-indolyl which is optionally substituted by one or more substituents from the group of halogen, alkyl, hydroxy, alkoxy, haloalkyl, nitro, amino, acylamino, alkylthio, alkylsulphinyl and alkylsulphonyl.

9. Compounds according to claim 8, wherein $R^3$ is 1-methyl-3-indolyl.

10. Compounds according to any one of claims 1-9, wherein $R^4$, $R^5$ and $R^6$ are hydrogen.

11. Compounds according to any one of claims 1-10, wherein $R^8$ is a group of the formula $-(CH_2)_q-R^{10}$.

12. Compounds according to claim 11, wherein q is the number 1 or 2.

13. Compounds according to claim 11 or 12, wherein $R^{10}$ is hydroxy, amino, monoalkylamino, dialkylamino, trialkylamino, azido, acylamino, alkylcarbonyloxy or alkylsulphonyloxy or a group of the formula -U-C-(V)-W.

14. Compounds according to claim 13, wherein U is a S atom, V is the group NH and W is amino.

15. Compounds according to any one of claims 1-14, wherein X and Y are oxygen.

16. Compounds according to claim 1 or 2 from the following group:
3-[8-(Aminomethyl)-6,7,8,9-tetrahydropyrido[1,2-a]indol-10-yl]-4-(1-methyl-3-indolyl)-1H-pyrrole-2,5-dione,
3-[7-(amidinothiomethyl)-6,7,8,9-tetrahydropyrido[1,2-a]indol-10-yl]-4-(1-methyl-3-indolyl)-1H-pyrrole-2,5-dione,
3-[6,7,8,9-tetrahydro-8-[(dimethylamino)methyl]pyrido[1,2- a]indol-10-yl]-4-(1-methyl-3-indolyl)-1H-pyrrole-2,5-dione and pharmaceutically usable salts thereof.

**17.** Compounds of the general formula

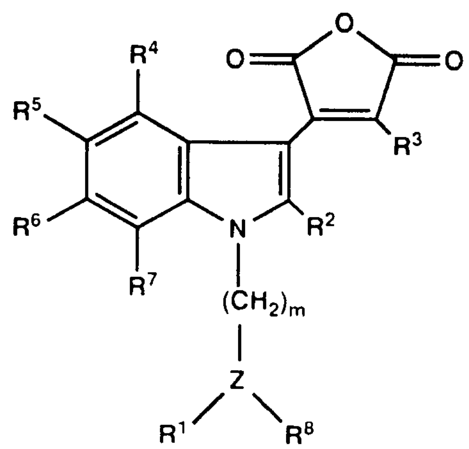

II

wherein $R^1$, $R^2$ $R^3$, $R^4$, $R^5$, $R^6$, $R^7$, $R^8$, Z and m have the significance given in claim 1.

**18.** Compounds according to any one of claims 1-16 for use as therapeutically active substances, especially for use as antiinflammatory, immunological, oncological, bronchopulmonary and cardiovascular active substances or as active substances in the treatment of asthma or AIDS.

**19.** A process for the manufacture of the compounds according to any one of claims 1-16, characterized by

(a) for the manufacture of a compound of formula I in which X and Y are oxygen, reacting a compound of the general formula

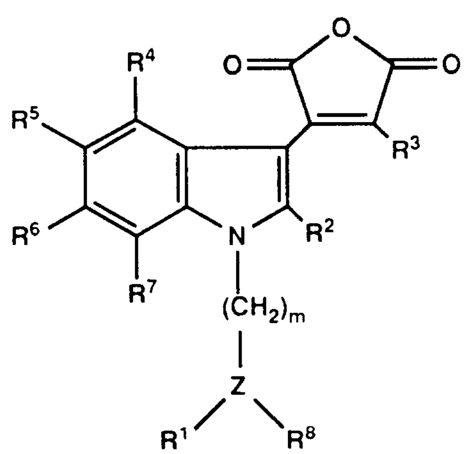

II

wherein $R^1$, $R^2$, $R^3$, $R^4$, $R^5$, $R^6$, $R^7$, $R^8$, Z, and m have the significance given in claim 1, with ammonia under pressure or with hexamethyldisilazane and methanol to give a compound of formula I in which R is hydrogen or with hydroxylamine to give a compound of formula I in which R is hydroxy, or

(b) for the manufacture of a compound of formula I in which one of X and Y is oxygen and the other is (H,H), reducing a compound of formula I in which X and Y are oxygen with lithium aluminum hydride, or

(c) if desired, functionally modifying a reactive group present in a compound of formula I obtained, and

(d) if desired, converting an acidic compound of formula I into a pharmaceutically usable salt with a base or converting a basic compound of formula I into a pharmaceutically usable salt with an acid.

**20.** A pharmaceutical preparation, especially an antiinflammatory, immunological, oncological, bronchopulmonary or cardiovascular preparation or a preparation for the treatment of asthma or of AIDS,

containing a compound according to any one of claims 1-16 and an inert carrier material.

**21.** The use of a compound according to any one of claims 1-16 for the manufacture of a pharmaceutical preparation against inflammatory, immunological, oncological, bronchopulmonary or cardiovascular disorders or against asthma or AIDS.

**Claims for the following Contracting States : ES, GR**

**1.** A process for the manufacture of compounds of the general formula

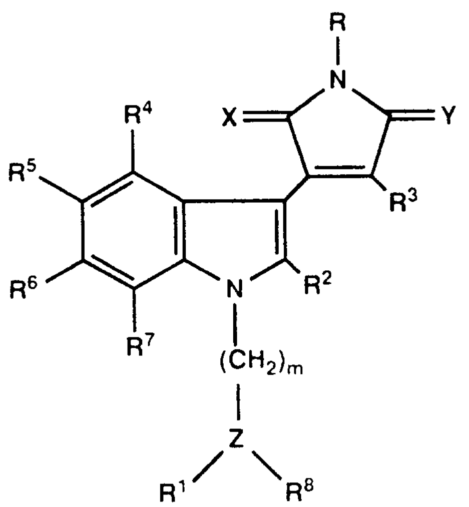

I

wherein R is hydrogen or hydroxy, $R^1$ and $R^2$ together are a group of the formula $-(CH_2)_n-$ and $R^7$ is hydrogen or $R^1$ and $R^7$ together are a group of the formula $-(CH_2)_n-$ and $R^2$ is hydrogen; $R^3$ is aryl or heteroaryl; $R^4$, $R^5$ and $R^6$ are hydrogen, halogen, alkyl, hydroxy, alkoxy, haloalkyl, nitro, amino, acylamino, alkylthio, alkylsulphinyl or alkylsulphonyl; $R^8$ is a group of the formula $-(CH_2)_p-R^9$ or $-(CH_2)_q-R^{10}$;$R^9$ is hydrogen, alkylcarbonyl, aminoalkylcarbonyl, cyano, amidino, alkoxycarbonyl, aryloxycarbonyl, alkylsulphonyl, aminocarbonyl or aminothiocarbonyl; $R^{10}$ is hydroxy, alkoxy, halogen, amino, monoalkylamino, dialkylamino, trialkylamino, azido, acylamino, alkylsulphonylamino, arylsulphonylamino, alkylthio, alkoxycarbonylamino, aminoacylamino, aminocarbonylamino, isothiocyanato, alkylcarbonyloxy, alkylsulphonyloxy or arylsulphonyloxy, a 5- or 6-membered saturated nitrogen-containing heterocycle attached via the N atom or a group of the formula -U-C(V)-W; U is a S atom or NH; V is the group NH, $NNO_2$, NCN or $CHNO_2$; W is amino, mono- or dialkylamino; one of X and Y is an O atom and the other is O or (H,H); Z is the group CH or a N atom; m, p and q are a number 0 to 5 and n is a number from 1 to 5, with the proviso that m and q are a number from 2 to 5 when Z is a N atom;

as well as of pharmaceutically usable salts of acidic compounds of formula I with bases and of basic compounds of formula I with acids, characterized by

(a) for the manufacture of a compound of formula I in which X and Y are oxygen, reacting a compound of the general formula

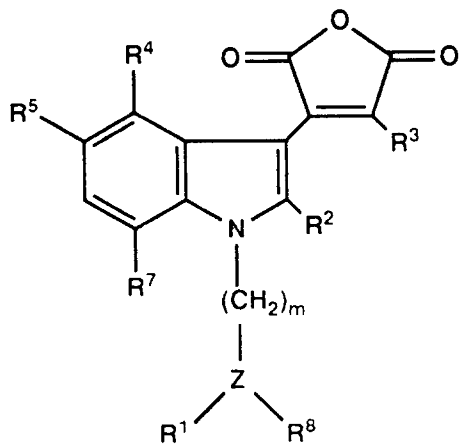

II

wherein $R^1$, $R^2$, $R^3$, $R^4$, $R^5$, $R^6$, $R^7$, $R^8$, Z and m have the significance given in claim 1, with ammonia under pressure or with hexamethyldisilazane and methanol to give a compound of formula I in which R is hydrogen or with hydroxylamine to give a compound of formula I in which R is hydroxy, or

(b) for the manufacture of a compound of formula I in which one of X and Y is oxygen and the other is (H,H), reducing a compound of formula I in which X and Y are oxygen with lithium aluminium hydride, or

(c) if desired, functionally modifying a reactive group present in a compound of formula I obtained, and

(d) if desired, converting an acidic compound of formula I into a pharmaceutically usable salt with a base or converting a basic compound of formula I into a pharmaceutically usable salt with an acid.

**2.** A process according to claim I, wherein R is hydrogen, $R^9$ is hydrogen, alkylcarbonyl, cyano, amidino, alkoxycarbonyl, alkylsulphonyl, aminocarbonyl or aminothiocarbonyl and $R^{10}$ is hydroxy, alkoxy, halogen, amino, monoalkylamino, dialkylamino, trialkylamino, azido, acylamino, alkylsulphonylamino, arylsulphonylamino, alkylthio, aminocarbonylamino, isothiocyanato, alkylcarbonyloxy, alkylsulphonyloxy or arylsulphonyloxy or a group of the formula -U-C(V)-W and $R^1$, $R^2$, $R^3$, $R^4$, $R^5$, $R^6$, $R^7$, $R^8$, U, V, W, X, Y, Z, m, n, p and q have the significance given in claim 1.

**3.** A process according to claim 1 or 2, wherein $R^1$ and $R^2$ together are $-CH_2-$ and $R^7$ is hydrogen, m is the number 1 or 2 and Z is the group CH.

**4.** A process according to claim 1 or 2, wherein $R^1$ and $R^2$ together are $-(CH_2)_2-$ and $R^7$ hydrogen, m is the number 1 and Z is the group CH.

**5.** A process according to claim 1 or 2, wherein $R^1$ and $R^2$ together are $-CH_2-$ and $R^7$ is hydrogen, m is the number 2 and Z is a N atom.

**6.** A process according to claim 1 or 2, wherein $R^1$ and $R^7$ together are $-CH_2-$ and $R^2$ is hydrogen, m is the number 1 and Z is the group CH.

**7.** A process according to claim 1 or 2, wherein $R^1$ and $R^7$ together are $-(CH_2)_2-$ and $R^2$ is hydrogen, m is 0 and Z is the group CH.

**8.** A process according to any one of claims 1 to 6, wherein $R^3$ is phenyl, naphthyl, 3-benzothienyl, 3-benzofuranyl or 3-indolyl which is optionally substituted by one or more substituents from the group of halogen, alkyl, hydroxy, alkoxy, haloalkyl, nitro, amino, acylamino, alkylthio, alkylsulphinyl and alkylsulphonyl.

**9.** A process according to claim 8, wherein $R^3$ is 1-methyl-3-indolyl.

**10.** A process according to any one of claims 1-9, wherein $R^4$, $R^5$ and $R^6$ are hydrogen.

**11.** A process according to any one of claims 1-10, wherein $R^8$ is a group of the formula $-(CH_2)_q-R^{10}$.

**12.** A process according to claim 11, wherein q is the number 1 or 2.

**13.** A process according to claim 11 or 12, wherein $R^{10}$ is hydroxy, amino, monoalkylamino, dialkylamino, trialkylamino, azido, acylamino, alkylcarbonyloxy or alkylsulphonyloxy or a group of the formula -U-C-(V)-W.

**14.** A process according to claim 13, wherein U is a S atom, V is the group NH and W is amino.

**15.** A process according to any one of claims 1-14, wherein X and Y are oxygen.

**16.** A process according to claim 1 or 2, wherein a compound from the following group is manufactured:
3-[8-(Aminomethyl)-6,7,8,9-tetrahydropyrido[1,2-a]indol-10-yl]-4-(1-methyl-3-indolyl)-1H-pyrrole-2,5-dione,
3-[7-(amidinothiomethyl)-6,7,8,9-tetrahydropyrido[1,2-a]indol-10-yl]-4-(1-methyl-3-indolyl)-1H-pyrrole-2,5-dione
3-[6,7,8,9-tetrahydro-8-[(dimethylamino)methyl]pyrido[1,2-a]indol-10-yl]-4-(1-methyl-3-indolyl)-1H-pyrrole-2,5-dione
and pharmaceutically usable salts thereof.

**17.** A process according to claim 1, wherein
a) the compound or formula II reacted with ammonia at 100-150 °C, with hexamethyldisilazane and methanol at 40-110 °C or with hydroxylamine at 100 °C, in each case in an inert organic solvent,
b) the reduction with $LiAlH_4$ is carried out in an inert organic solvent at a temperature between 0 °C and the reflux temperature,
c) if desired, an alkoxycarbonyl group $R^8$ is converted into a H atom with an acid; a group alkyl-COO-$(CH_2)_q$- is converted with a base into a group HO-$(CH_2)_q$-; the latter group is converted by treatment with trifluoromethanesulphonic anhydride and then with ammonia, a mono-, di- or trialkylamine or a suitable heterocycle into the group $-(CH_2)_q-R^{10}$ in which $R^{10}$ is optionally mono-, di- or trialkylated amino or a 5- or 6-membered heterocycle; a group $-(CH_2)_q-OH$ is reacted with an alkanesulphonic anhydride; a resulting group alkyl-$SO_3$-$(CH_2)_q$- is converted with ammonia in dimethylformamide, with an alkali metal azide or with thiourea into a group $-(CH_2)_q-R^{10}$ in which $R^{10}$ is formamido, azido or $-SNHNH_2$; a group $-(CH_2)_q-N_3$ is catalytically hydrogenated to a group $-(CH_2)_q-NH_2$; a group alkyl-OCONH-$(CH_2)_q$- is converted with an acid into a group $-(CH_2)_q-NH_2$; the latter group is N-acylated or converted using 3,5-dimethyl-$N^2$-nitro-1-pyrazolyl-1-carboxamide or using 1,1-thiocarbonyldiimidazole into a group $-(CH_2)_q-NHC(NH)NNO_2$ or $-(CH_2)_q-NCS$; a group $-(CH_2)_p-CN$ is converted with hydrogen chloride and then ammonia into a group $-(CH_2)_p-C(NH)NH_2$; a compound in which Z is a N atom and $R^8$ is hydrogen is acylated to a compound in which $R^8$ is alkyl-CO-, alkyl-OCO- or aryl-OCO- or converted using an alkanesulphonyl chloride, using a trifluoroacetamidoalkanoyl chloride and ammonia, using 1,1-carbonyldiimidazole and ammonia or using 1,1-thiocarbonyldiimidazole and ammonia into a compound in which $R^8$ is alkyl-$SO_2$-, -CO-alkyl-$NH_2$, $-CONH_2$ or $-CSNH_2$.

**18.** A process for the manufacture of pharmaceutical preparations, especially antiinflammatory, immunological, oncological, bronchopulmonary or cardiovascular preparations or preparations for the treatment of asthma or AIDS, characterized by bringing a product according to claim 1 and an inert carrier material into a galenical administration form.

**19.** The use of a product according to claim 1 for the manufacture of a pharmaceutical preparation against inflammatory, immunological, oncological, bronchopulmonary or cardiovascular disorders or against asthma or AIDS.

**20.** Compounds of the general formula

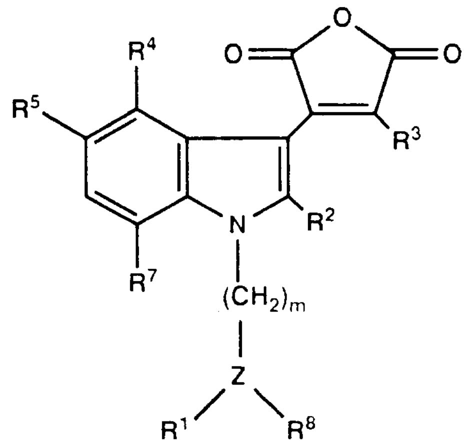

II

wherein $R^1$, $R^2$, $R^3$, $R^4$, $R^5$ $R^6$, $R^7$, $R^8$, Z and m have the significance given in claim 1.

**Revendications**

**Revendications pour les Etats contractants suivants : AT, BE, CH, DE, DK, FR, GB, IT, LI, LU, NL, SE**

**1.** Composés de formule générale

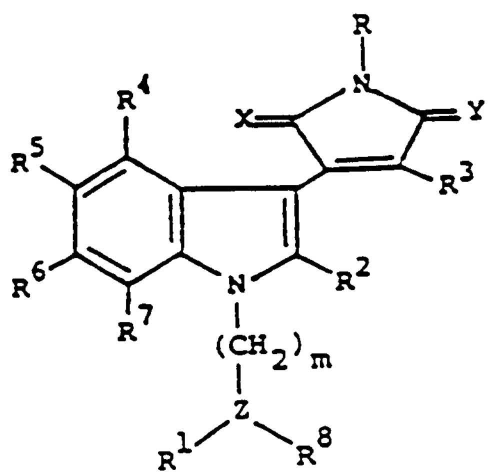

I

où R est l'hydrogène ou l'hydroxy, $R^1$ et $R^2$ représentent ensemble un groupe de formule $-(CH_2)_n-$ et $R^7$ est l'hydrogène ou $R^1$ et $R^7$ représentent ensemble un groupe de formule $-(CH_2)_n-$ et $R^2$ est l'hydrogène; $R^3$ est un aryle ou un hétéroaryle; $R^4$, $R^5$ et $R^6$ sont l'hydrogène, un halogène, un alkyle, l'hydroxy, un alcoxy, un haloalkyle, le nitro, l'amino, un acylamino, un alkylthio, un alkylsulfinyle ou un alkylsulfonyle; $R^8$ est un groupe de formule $-(CH_2)_p-R^9$ ou $-(CH_2)_q-R^{10}$; $R^9$ est l'hydrogène, un alkylcarbonyle, un aminoalkylcarbonyle, le cyano, un amidino, un alcoxycarbonyle, un aryloxycarbonyle, un alkylsulfonyle, un aminocarbonyle ou un aminothiocarbonyle; $R^{10}$ est l'hydroxy, un alcoxy, un halogène, un amino, un monoalkylamino, un dialkylamino, un trialkylamino, l'azido, un acylamino, un alkylsulfonylamino, un arylsulfonylamino, un alkylthio, un alcoxycarbonylamino, un aminoacylamino, un aminocarbonylamino, un isothiocyanato, un alkylcarbonyloxy, un alkylsulfonyloxy ou un arylsulfonyloxy, un groupe hétérocyclique saturé contenant de l'azote à 5 ou 6 chaînons, lié par l'atome de N ou un groupe de formule -U-C(V)-W; U est un atome de S ou NH; V représente les groupes NH, $NNO_2$, NCN ou $CHNO_2$; W est un amino, un mono- ou dialkylamino; l'un des symboles X et Y est un atome de O et l'autre symbole O ou (H,H); Z est le groupe CH ou un atome de N; m, p et q sont des nombres allant de 0 à 5 et n est un nombre allant de 1 à 5, sous réserve que m et q soient un nombre allant de 2 à 5, lorsque Z est un atome de N;

ainsi que les sels pharmaceutiquement utilisables des composés acides de formule I avec des bases et des composés basiques de formule I avec des acides.

**2.** Composés selon la revendication 1, où R est l'hydrogène, $R^9$ est l'hydrogène, un alkylcarbonyle, le cyano, un amidino, un alcoxycarbonyle, un alkylsulfonyle, un aminocarbonyle ou un aminothiocarbonyle et $R^{10}$ est l'hydroxy, un alcoxy, un halogène, un amino, un monoalkylamino, un dialkylamino, un trialkylamino, un azido, un acylamino, un alkylsulfonylamino, un arylsulfonylamino, un alkylthio, un aminocarbonylamino, un isothiocyanato, un alkylcarbonyloxy, un alkylsulfonyloxy ou un arylsulfonyloxy ou un groupe de formule -U-C(V)-W et $R^1$, $R^2$, $R^3$, $R^4$, $R^5$, $R^6$, $R^7$, $R^8$, U, V, W, X, Y, Z, m, n, p et q ont les significations données dans la revendication 1.

**3.** Composés selon la revendication 1 ou 2, où $R^1$ et $R^2$ représentent ensemble $-CH_2-$ et $R^7$ est l'hydrogène, m est égal à 1 ou 2 et Z est le groupe CH.

**4.** Composés selon la revendication 1 ou 2, où $R^1$ et $R^2$ représentent ensemble $-(CH_2)_2-$ et $R^7$ est l'hydrogène, m est égal à 1 et Z est le groupe CH.

**5.** Composés selon la revendication 1 ou 2, où $R^1$ et $R^2$ représentent ensemble $-CH_2-$ et $R^7$ est l'hydrogène, m est égal à 2 et Z est un atome de N.

**6.** Composés selon la revendication 1 ou 2, où $R^1$ et $R^7$ représentent ensemble $-CH_2-$ et $R^2$ est l'hydrogène, m est égal à 1 et Z est le groupe CH.

**7.** Composés selon la revendication 1 ou 2, où $R^1$ et $R^7$ représentent ensemble $-(CH_2)_2-$ et $R^2$ est l'hydrogène, m est égal à 0 et Z est le groupe CH.

**8.** Composés selon l'une des revendications 1 à où $R^3$ est le phényle, le naphtyle, le 3-benzothiényle, le 3-benzofurannyle ou le 3-indolyle, substitués, si désiré, par un ou plusieurs substituants provenant du groupe constitué par un halogène, un alkyle, l'hydroxy, un alcoxy, un haloalkyle, le nitro, l'amino, un acylamino, un alkylthio, un alkylsulfinyle et un alkylsulfonyle.

**9.** Composés selon la revendication 8, où $R^3$ est le 1-méthyl-3-indolyle.

**10.** Composés selon l'une des revendications 1 à 9, où $R^4$, $R^5$ et $R^6$ sont l'hydrogène.

**11.** Composés selon l'une des revendications 1 à 10, où $R^8$ est un groupe de formule $-(CH_2)_q-R^{10}$.

**12.** Composés selon la revendication 11, où q est égal à 1 ou 2.

**13.** Composés selon la revendication 11 ou 12, où $R^{10}$ est l'hydroxy, l'amino, un monoalkylamino, un dialkylamino, un trialkylamino, l'azido, un acylamino, un alkylcarbonyloxy ou un alkylsulfonyloxy ou un groupe de formule -U-C(V)-W.

**14.** Composés selon la revendication 13, où U est un atome de S, V est le groupe NH et W est un amino.

**15.** Composés selon l'une des revendications 1 à 14, où X et Y sont l'oxygène.

**16.** Composés selon la revendication 1 ou 2 provenant du groupe des composés suivants :

la 3-[8-(aminométhyl)-6,7,8,9-tétrahydropyrido [1,2-a]indol-10-yl]-4-(1-méthyl-3-indolyl)-1H-pyrrol-2,5-dione,

la 3-[7-(amidinothiométhyl)-6,7,8,9-tétrahydropyrido[1,2-a]-indol-10-yl]-4-(1-méthyl-3-indolyl)-1H-pyrrol-2,5-dione,

la 3-[6,7,8,9-tétrahydro-8-[(diméthylamino)méthyl]pyrido[1,2-a]indol-10-yl]-4-(1-méthyl-3-indolyl)-1H-pyrrol-2,5-dione,

et leurs sels pharmaceutiquement utilisables.

**17.** Composés de formule générale

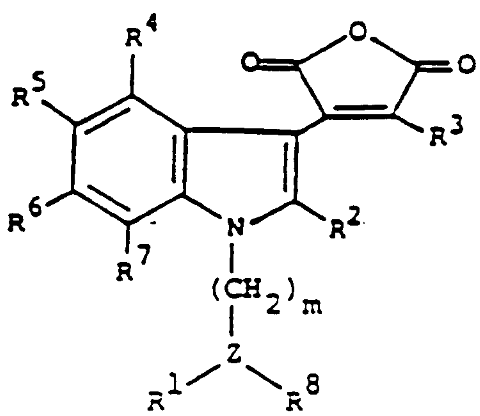

II

où $R^1$, $R^2$, $R^3$, $R^4$, $R^5$, $R^6$, $R^7$, $R^8$, Z et n ont les significations données dans la revendication 1.

**18.** Composés selon l'une des revendications 1 à 16 destinés à être utilisés comme substances thérapeutiquement actives, en particulier destinés à être utilisés comme substances ayant une activité antiinflammatoire, inmunologique, oncologique, broncho-pulmonaire et cardio-vasculaire ou comme substances actives dans le traitement de l'asthme ou du SIDA.

**19.** Procédé pour la préparation des composés selon l'une des revendications 1 à 16, caractérisé
a) en ce que, pour la préparation d'un composé de formule I dans laquelle X et Y sont l'oxygène, on fait réagir un composé de formule générale

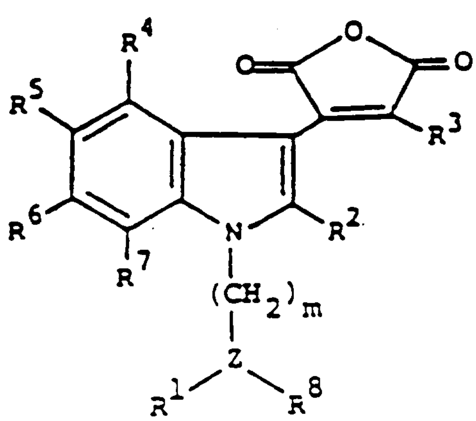

II

où $R^1$, $R^2$, $R^3$, $R^4$, $R^5$, $R^6$, $R^7$, $R^8$, Z et m ont les significations données dans la revendication 1, avec l'ammoniac sous pression ou avec l'hexaméthyldisilazane et le méthanol pour former un composé de formule I dans laquelle R est l'hydrogène ou avec l'hydroxylamine pour former un composé de formule I dans laquelle R est l'hydroxy ou
b) en ce que, pour la préparation d'un composé de formule I dans laquelle l'un des symboles X et Y est l'oxygène et l'autre symbole (H,H), on réduit un composé de formule I dans laquelle X et Y sont l'oxygène, avec l'hydrure de lithium et d'aluminium et
c) en ce que, si désiré, on modifie fonctionnellement un groupe réactif contenu dans un composé de formule I obtenu et
d) en ce que, si désiré, on transforme un composé acide de formule I en un sel pharmaceutiquement utilisable avec une base ou un composé basique de formule I en un sel pharmaceutiquement utilisable avec un acide.

**20.** Préparations pharmaceutiques, en particulier préparations antiinflammatoires, immunologiques, oncologiques, broncho-pulmonaires ou cardio-vasculaires ou préparations pour le traitement de l'asthme ou

du SIDA contenant un composé selon l'une des revendications 1 à 16 et un support inerte.

**21.** Utilisation d'un composé selon l'une des revendications 1 à 16 pour la préparation d'une composition pharmaceutique contre les maladies inflammatoires, immunologiques, oncologiques, broncho-pulmonaires ou cardio-vasculaires ou contre l'asthme ou le SIDA.

**Revendications pour les Etats contractants suivants : ES, GR**

**1.** Procédé pour la préparation des composés de formule générale

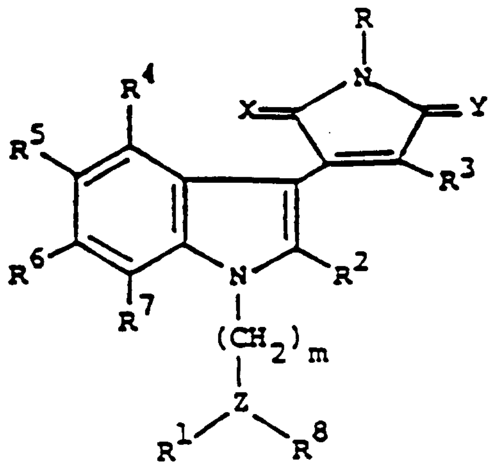

I

où R est l'hydrogène ou l'hydroxy, $R^1$ et $R^2$ représentent ensemble un groupe de formule $-(CH_2)_n-$ et $R^7$ est l'hydrogène ou $R^1$ et $R^7$ représentent ensemble un groupe de formule $-(CH_2)_n-$ et $R^2$ est l'hydrogène; $R^3$ est un aryle ou un hétéroaryle; $R^4$, $R^5$ et $R^6$ sont l'hydrogène, un halogène, un alkyle, l'hydroxy, un alcoxy, un haloalkyle, le nitro, l'amino, un acylamino, un alkylthio, un alkylsulfinyle ou un alkylsulfonyle; $R^8$ est un groupe de formule $-(CH_2)_p-R^9$ ou $-(CH_2)_q-R^{10}$ ; $R^9$ est l'hydrogène, un alkylcarbonyle, un aminoalkylcarbonyle, le cyano, un amidino, un alcoxycarbonyle, un aryloxycarbonyle, un alkylsulfonyle, un aminocarbonyle ou un aminothiocarbonyle; $R^{10}$ est l'hydroxy, un alcoxy, un halogène, un amino, un monoalkylamino, un dialkylamino, un trialkylamino, l'azido, un acylamino, un alkylsulfonylamino, un arylsulfonylamino, un alkylthio, un alcoxycarbonylamino, un aminoacylamino, un aminocarbonylamino, un isothiocyanato, un alkylcarbonyloxy, un alkylsulfonyloxy ou un arylsulfonyloxy, un groupe hétérocyclique saturé contenant de l'azote à 5 ou 6 chaînons, lié par l'atome de N ou un groupe de formule -U-C(V)-W; U est un atome de S ou NH; V représente les groupes NH, $NNO_2$, NCN ou $CHNO_2$; W est un amino, un mono- ou dialkylamino; l'un des symboles X et Y est un atome de O et l'autre symbole O ou (H,H); Z est le groupe CH ou un atome de N; m, p et q sont des nombres allant de 0 à 5 et n est un nombre allant de 1 à 5, sous réserve que m et q soient un nombre allant de 2 à 5, lorsque Z est un atome de N;
ainsi que des sels pharmaceutiquement utilisables des composés acides de formule I avec des bases et des composés basiques de formule I avec des acides, caractérisé

a) en ce que, pour la préparation d'un composé de formule I dans laquelle X et Y sont l'oxygène, on fait réagir un composé de formule générale

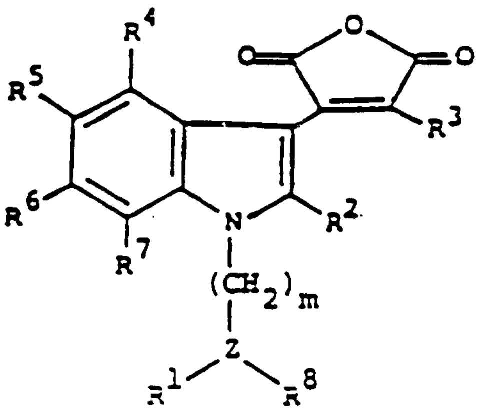

II

où $R^1$, $R^2$, $R^3$, $R^4$, $R^5$, $R^6$, $R^7$, $R^8$, Z et m ont les significations données dans la revendication 1, avec l'ammoniac sous pression ou avec l'hexaméthyldisilazane et le méthanol pour former un composé de formule I dans laquelle R est l'hydrogène ou avec l'hydroxylamine pour former un composé de formule I dans laquelle R est l'hydroxy ou

b) en ce que, pour la préparation d'un composé de formule I dans laquelle l'un des symboles X et Y est l'oxygène et l'autre symbole (H,H), on réduit un composé de formule I dans laquelle X et Y sont l'oxygène, avec l'hydrure de lithium et d'aluminium et

c) en ce que, si désiré, on modifie fonctionnellement un groupe réactif contenu dans un composé de formule I obtenu et

d) en ce que, si désiré, on transforme un composé acide de formule I en un sel pharmaceutiquement utilisable avec une base ou un composé basique de formule I en un sel pharmaceutiquement utilisable avec un acide.

**2.** Procédé selon la revendication 1, où R est l'hydrogène, $R^9$ est l'hydrogène, un alkylcarbonyle, le cyano, un amidino, un alcoxycarbonyle, un alkylsulfonyle, un aminocarbonyle ou un aminothiocarbonyle et $R^{10}$ est l'hydroxy, un alcoxy, un halogène, un amino, un monoalkylamino, un dialkylamino, un trialkylamino, un azido, un acylamino, un alkylsulfonylamino, un arylsulfonylamino, un alkylthio, un aminocarbonylamino, un isothiocyanato, un alkylcarbonyloxy, un alkylsulfonyloxy ou un arylsulfonyloxy ou un groupe de formule -U-C(V)-W et $R^1$, $R^2$, $R^3$, $R^4$, $R^5$, $R^6$, $R^7$, $R^8$, U, V, W, X, Y, Z, m, n, p et q ont les significations données dans la revendication 1.

**3.** Procédé selon la revendication 1 ou 2, où $R^1$ et $R^2$ représentent ensemble $-CH_2-$ et $R^7$ est l'hydrogène, m est égal à 1 ou 2 et Z est le groupe CH.

**4.** Procédé selon la revendication 1 ou 2, où $R^1$ et $R^2$ représentent ensemble $-(CH_2)_2-$ et $R^7$ est l'hydrogène, m est égal à 1 et Z est le groupe CH.

**5.** Procédé selon la revendication 1 ou 2, où $R^1$ et $R^2$ représentent ensemble $-CH_2-$ et $R^7$ est l'hydrogène, m est égal à 2 et Z est un atome de N.

**6.** Procédé selon la revendication 1 ou 2, où $R^1$ et $R^7$ représentent ensemble $-CH_2-$ et $R^2$ est l'hydrogène, m est égal à 1 et Z est le groupe CH.

**7.** Procédé selon la revendication 1 ou 2, où $R^1$ et $R^7$ représentent ensemble $-(CH_2)_2-$ et $R^2$ est l'hydrogène, m est égal à 0 et Z est le groupe CH.

**8.** Procédé selon l'une des revendications 1 à 6, où $R^3$ est le phényle, le naphtyle, le 3-benzothiényle, le 3-benzofurannyle ou le 3-indolyle, substitués, si désiré, par un ou plusieurs substituants provenant du groupe constitué par un halogène, un alkyle, l'hydroxy, un alcoxy, un haloalkyle, le nitro, l'amino, un acylamino, un alkylthio, un alkylsulfinyle et un alkylsulfonyle.

**9.** Procédé selon la revendication 8, où $R^3$ est le 1-méthyl-3-indolyle.

**10.** Procédé selon l'une des revendications 1 à 9, où $R^4$, $R^5$ et $R^6$ sont l'hydrogène.

**11.** Procédé selon l'une des revendications 1 à 10, où $R^8$ est un groupe de formule $-(CH_2)_q-R^{10}$.

**12.** Procédé selon la revendication 11, où q est égal à 1 ou 2.

**13.** Procédé selon la revendication 11 ou 12, où $R^{10}$ est l'hydroxy, l'amino, un monoalkylamino, un dialkylamino, un trialkylamino, l'azido, un acylamino, un alkylcarbonyloxy ou un alkylsulfonyloxy ou un groupe de formule -U-C(V)-W.

**14.** Procédé selon la revendication 13, où U est un atome de S, V est le groupe NH et W est un amino.

**15.** Procédé selon l'une des revendications 1 à 14, où X et Y sont l'oxygène.

**16.** Procédé selon la revendication 1 ou 2 dans lequel on prépare un composé provenant du groupe des composés suivants :
la 3-[8-(aminométhyl)-6,7,8,9-tétrahydropyrido[1,2-a]indol-10-yl]-4-(1-méthyl-3-indolyl)-1H-pyrrol-2,5-dione,
la 3-[7-(amidinothiométhyl)-6,7,8,9-tétrahydropyrido[1,2-a]-indol-10-yl]-4-(1-méthyl-3-indolyl)-1H-pyrrol-2,5-dione,
la 3-[6,7,8,9-tétrahydro-8-[(diméthylamino)méthyl]pyrido[1,2-a]indol-10-yl]-4-(1-méthyl-3-indolyl)-1H-pyrrol-2,5-dione,
et leurs sels pharmaceutiquement utilisables.

**17.** Procédé selon la revendication 1 dans lequel
a) on fait réagir le composé de formule II avec l'ammoniac à une température comprise entre 100 et 150 °C, avec l'hexaméthyldisilazane et le méthanol à une température comprise entre 40 et 110 °C ou avec l'hydroxylamine à 100 °C, dans un solvant organique inerte,
b) on effectue la réduction avec $LiAlH_4$ dans un solvant organique inerte à une température comprise entre 0 °C et la température de reflux,
c) si désiré, on transforme un groupe alcoxycarbonyle $R^8$ avec un acide en un atome de H; on transforme un groupe alkyl-COO-$(CH_2)_q$- avec une base en un groupe HO-$(CH_2)_q$-; on transforme ce dernier groupe par traitement avec l'anhydride trifluorométhanesulfonique, puis avec l'ammoniac, une mono-, di- ou trialkylamine ou un hétérocycle approprié en un groupe $-(CH_2)_q-R^{10}$ dans lequel $R^{10}$ est un amino éventuellement mono-, di- ou trialkylé ou un hétérocycle à 5 ou 6 chaînons; on fait réagir un groupe $-(CH_2)_q$-OH avec un anhydride alcanesulfonique; on transforme un groupe alkyl-$SO_3$-$(CH_2)_q$- obtenu avec l'ammoniac dans le diméthylformamide, avec un azide de métal alcalin ou avec la thiourée en un groupe $-(CH_2)_q-R^{10}$ dans lequel $R^{10}$ est le formamido, l'azido ou $-SNHNH_2$; on hydrogène catalytiquement un groupe $-(CH_2)_q-N_3$ pour former un groupe $-(CH_2)_q-NH_2$; on transforme un groupe alkyl-OCONH-$(CH_2)_q$- avec un acide en un groupe $-(CH_2)_q-NH_2$; on acyle en N ce dernier groupe ou on transforme celui-ci avec le 3,5-diméthyl-$N^2$-nitro-1-pyrrazolyl-1-carboxamide ou avec le 1,1-thiocarbonyldiimidazole en un groupe $-(CH_2)_q-NHC(NH)HNO_2$ ou $-(CH_2)_q$-NCS; on transforme un groupe $-(CH_2)_p$-CN avec l'acide chlorhydrique, puis avec l'ammoniac en un groupe $-(CH_2)_p-C(NH)NH_2$; on acyle un composé dans lequel Z est un atome de N et $R^8$ est l'hydrogène, pour former un composé dans lequel $R^8$ est un alkyl-CO-, alkyl-OCO- ou aryl-OCO- ou on le transforme avec un chlorure d'alcanesulfonyle, avec un chlorure de trifluoroacétamidoalcanoyle et l'ammoniac, avec le 1,1-carbonyldiimidazole et l'ammoniac ou avec le 1,1-thiocarbonyldiimidazole et l'ammoniac en un composé dans lequel $R^8$ est un alkyl-$SO_2$-, -CO-alkyl-$NH_2$, $-CONH_2$ ou $-CSNH_2$.

**18.** Procédé pour la préparation de compositions pharmaceutiques, en particulier de compositions antiinflammatoires, immunologiques, oncologiques, broncho-pulmonaires ou cardio-vasculaires ou de compositions pour le traitement de l'asthme ou du SIDA, caractérisé en ce que l'on met un produit selon la revendication 1 et un support inerte sous forme d'une présentation galénique.

**19.** Utilisation d'un produit selon la revendication 1 pour la préparation d'une composition pharmaceutique contre les maladies inflammatoires, immunologiques, oncologiques, broncho-pulmonaires ou cardio-vasculaires ou contre l'asthme ou le SIDA.

**20.** Composés de formule générale

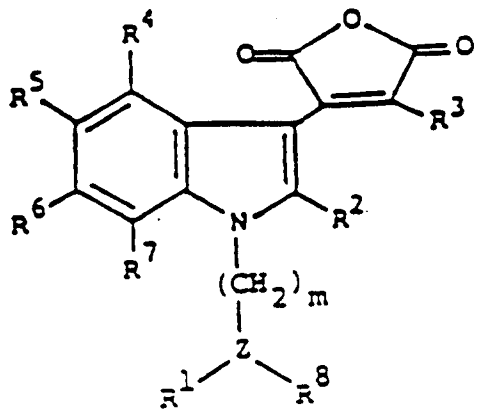

II

où $R^1$, $R^2$, $R^3$, $R^4$, $R^5$, $R^6$, $R^7$, $R^8$, Z et m ont les significations données dans la revendication 1.